# EUROPEAN PATENT APPLICATION

(11) **EP 4 631 502 A1**
(43) Date of publication of application: **15.10.2025**
(21) Application number: 24170035.0
(22) Date of filing: 12.04.2024
(51) Int. Cl.: A61K 31/352, A61K 31/185, A61K 31/375, A61K 31/4188, A61K 31/4415, A61K 31/51, A61K 31/525, A61K 31/593, A61K 31/7048, A61P 25/00

(54) **USE OF FLAVONES SUCH AS QUERCETIN TO INCREASE COGNITIVE FUNCTION AND TO REDUCE JITTERNESS**

(71) Applicant: Nicoventures Trading Limited, London WC2R 3LA (GB)
(72) Inventor: SASS, Anuradha Bansal, London, WC2R 3LA (GB)
(74) Representative: D Young & Co LLP

(57) **Abstract**

The present disclosure relates to uses of compounds or combinations thereof to increase cognitive performance, alertness and/or energy levels of a human or animal, as well as an oral product comprising said compounds or combinations thereof.

## Description

### FIELD

The present disclosure relates to uses of a compound of Formula (I), a derivative thereof, or a combination thereof; and an oral product comprising a compound of Formula (I), a derivative thereof, or a combination thereof. In particular, the uses and oral products are intended for human or animal use. The products are configured for oral ingested use and deliver substances such as flavours and/or active ingredients during use.

### BACKGROUND

Products that are easy to administer orally and which can provide a beneficial effect on particular mood states of a human or animal have become popular in recent years. For example, confectionary-type products (e.g. gummies or pastilles) containing vitamins or other mood-enhancing actives provide a convenient and pleasant mode of administration for such active ingredients. Other convenient modes of administration are food and beverages, for example energy drinks. Such products may include active ingredients that are delivered to the user in order to cause a biological response in the user that may enhance physical or mental performance of the user.

Beverages containing theanine and caffeine have been described in US 5,780,086 and US 6,268,009. More recently, EP1819241 describes beverages containing theanine and caffeine in a ratio of from 5:1 to 1:1.5. More generally, caffeinated beverages are widely consumed. In such beverages, caffeine may be naturally present such as in tea or coffee, or may be included as an added ingredient, for example in beverages such as colas and energy drinks. The presence of caffeine in such products may be considered desirable, for example to provide stimulant effects such as increased alertness, cognitive performance and/or energy levels. However, caffeine use may also be associated with other biological effects that may be considered less desirable, for example jitters, increased heart rate, 'crash' (i.e. tiredness and/or fatigue sometime after the initial stimulant effect), and/or diuresis.

It would be desirable to provide compounds that could provide one or more of the same or related advantageous benefits of caffeine, e.g. increased alertness, cognitive performance and/or energy levels. It would further be desirable if such compounds also provided additional advantageous physiological effects, including reduced levels of one or more of the same or related disadvantageous effects of caffeine, e.g. jitteriness. Oral products configured for oral use which may deliver such compounds to the consumer in an enjoyable and effective form, such as in the form of a liquid shot or chewable solid product, would also be desirable.

### BRIEF SUMMARY

In accordance with some embodiments described herein, there is provided a use of a compound of Formula (I), a derivative thereof, or a combination thereof, to increase cognitive performance, alertness and/or energy levels of a human or animal: 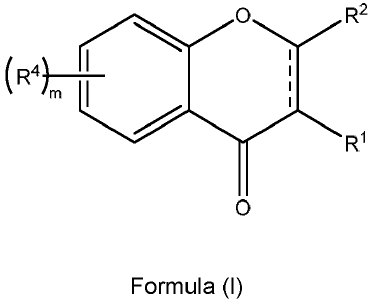 wherein:
(i) R¹ is H, OH, O-alkyl, O-alkenyl, SH, alkylthio, alkenylthio, NH₂, alkylamino, dialkylamino, alkenylamino, or dialkenylamino; and R² is an optionally substituted phenyl group; or
(ii) R¹ is an optionally substituted phenyl group; and R² is H, OH, O-alkyl, O-alkenyl, SH, alkylthio, alkenylthio, NH₂, alkylamino, dialkylamino, alkenylamino, or dialkenylamino;

wherein the optional substituents of the phenyl groups are independently selected from OH, O-alkyl, O-alkenyl, SH, alkylthio, alkenylthio, NH₂, alkylamino, dialkylamino, alkenylamino, and dialkenylamino;
each R⁴ is independently selected from OH, O-alkyl, O-alkenyl, SH, alkylthio, alkenylthio, NH₂, alkylamino, dialkylamino, alkenylamino, and dialkenylamino; and
m is an integer from 0 to 4.

In accordance with some embodiments described herein, there is provided an oral product comprising a combination of active ingredients, wherein the combination comprises:
(a) a compound of Formula (I), a derivative thereof, or a combination thereof: 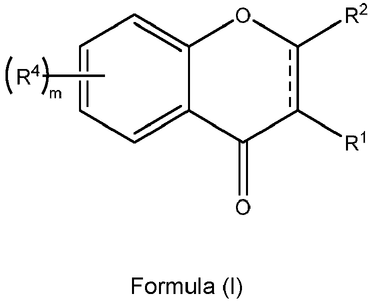 wherein:
   (i) R¹ is H, OH, O-alkyl, O-alkenyl, SH, alkylthio, alkenylthio, NH₂, alkylamino, dialkylamino, alkenylamino, or dialkenylamino; and R² is an optionally substituted phenyl group; or
   (ii) R¹ is an optionally substituted phenyl group; and R² is H, OH, O-alkyl, O-alkenyl, SH, alkylthio, alkenylthio, NH₂, alkylamino, dialkylamino, alkenylamino, or dialkenylamino;

   wherein the optional substituents of the phenyl groups are independently selected from OH, O-alkyl, O-alkenyl, SH, alkylthio, alkenylthio, NH₂, alkylamino, dialkylamino, alkenylamino, and dialkenylamino;
   each R⁴ is independently selected from OH, O-alkyl, O-alkenyl, SH, alkylthio, alkenylthio, NH₂, alkylamino, dialkylamino, alkenylamino, and dialkenylamino; and
   m is an integer from 0 to 4; and
(b) one or more B vitamins selected from B1, B2, B6 and B7; or a compound selected from maca root, ginseng, coenzyme Q10, caffeine, vitamin C, beta-alanine, theacrine, taurine, zinc or a salt thereof, L-theanine, theobromine, Echinacea, selenium, lemon balm, chamomile, L-tryptophan, piperine or a geometric isomer thereof, saffron, and combinations thereof,.

In accordance with some embodiments described herein, there is provided a process for preparing an oral product. The process comprising: (a) contacting at least one binding agent and at least one bulking agent selected from the group consisting of a sugar alcohol, or a sugar, or a combination of at least one sugar alcohol and at least one sugar, and optionally adding water, (b) heating the mixture of binding agent, sugar alcohol and/or sugar, and optionally water, (c) adding a combination of active ingredients, wherein the combination of active ingredients is as defined herein for the oral product, and (d) allowing the resulting mixture to set to provide an oral product in the form of a chew. Alternatively the process comprises: (a) combining active ingredients, wherein the combination of active ingredients is as defined herein for the oral product, (b) contacting the active ingredients with water, and (c) mixing the active ingredients with the water to prepare an oral product in the form of a liquid.

These and other features, aspects, and advantages of the disclosure will be apparent from a reading of the following detailed description. The invention includes any combination of two, three, four, or more of the above-noted embodiments as well as combinations of any two, three, four, or more features or elements set forth in this disclosure, regardless of whether such features or elements are expressly combined in a specific embodiment description herein. This disclosure is intended to be read holistically such that any separable features or elements of the disclosed invention, in any of its various aspects and embodiments, should be viewed as intended to be combinable unless the context clearly dictates otherwise.

For ease of reference, these and further aspects of the present invention are now discussed under appropriate section headings. However, the teachings under each section are not necessarily limited to each particular section.

### DETAILED DESCRIPTION

It is to be understood that this invention is not limited to the particular configurations, process steps, and materials disclosed herein as such configurations, process steps, and materials may vary somewhat. It is also to be understood that the terminology employed herein is used for the purpose of describing particular embodiments only and is not intended to be limiting since the scope of the present invention will be limited only by the appended claims and equivalents thereof.

As used in this specification and the claims, the singular forms "a," "an," and "the" include plural referents unless the context clearly dictates otherwise. Reference to "dry % by weight" or "dry weight basis" refers to weight on the basis of dry ingredients (i.e., all ingredients except water). Reference to "wet weight" refers to the weight of the product or composition including water. Unless otherwise indicated, reference to "% by weight" (or "% by weight") of a product or composition reflects the total wet weight of the product or composition (i.e., including water).

In this specification, unless otherwise stated, the term "about" modifying the quantity of an ingredient in the oral product of the invention or employed in the methods of the invention refers to variation in the numerical quantity that can occur, for example, through typical measuring and liquid handling procedures used for making concentrates or use solutions in the real world; through inadvertent error in these procedures; through differences in the manufacture, source, or purity of the ingredients employed to make the oral product, or to carry out the methods; and the like. The term "about" also encompasses amounts that differ due to different equilibrium conditions for a product or composition resulting from a particular initial mixture. Whether or not modified by the term "about", the claims include equivalents to the quantities.

### Use

Various aspects of the present disclosure described herein relate to the use of a compound of Formula (I), a derivative thereof, or a combination thereof: 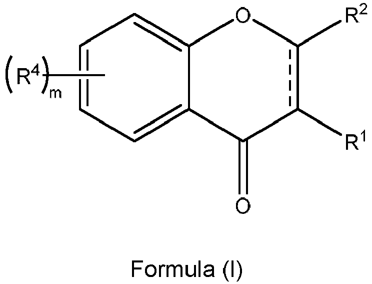 wherein:
(i) R¹ is H, OH, O-alkyl, O-alkenyl, SH, alkylthio, alkenylthio, NH₂, alkylamino, dialkylamino, alkenylamino, or dialkenylamino; and R² is an optionally substituted phenyl group; or
(ii) R¹ is an optionally substituted phenyl group; and R² is H, OH, O-alkyl, O-alkenyl, SH, alkylthio, alkenylthio, NH₂, alkylamino, dialkylamino, alkenylamino, or dialkenylamino;

wherein the optional substituents of the phenyl groups are independently selected from OH, O-alkyl, O-alkenyl, SH, alkylthio, alkenylthio, NH₂, alkylamino, dialkylamino, alkenylamino, and dialkenylamino;
each R⁴ is independently selected from OH, O-alkyl, O-alkenyl, SH, alkylthio, alkenylthio, NH₂, alkylamino, dialkylamino, alkenylamino, and dialkenylamino; and
m is an integer from 0 to 4.

It has been found by the present inventors that the compounds of Formula (I) may be used as a substitute or adjunct for caffeine. Caffeine (1,3,7-trimethylxanthine) is a central nervous system stimulant, a bitter tasting alkaloid that is naturally found in a variety of plant species, including tea leaves, kola nuts, cocoa, guarana, and coffee beans. Caffeine may be used to enhance alertness, attention, cognitive performance as well as physical performance. However, caffeine use may also be associated with undesirable effects such as jitters, increased heart rate, 'crash' (i.e. tiredness and/or fatigue sometime after the initial stimulant effect), and/or diuresis. Without wishing to be bound by theory, the physiological effects of caffeine are believed to be mediated via several mechanisms including: antagonism of adenosine receptors, inhibition of phosphodiesterases, release of calcium from intracellular stores, and antagonism of benzodiazepine receptors. In particular, antagonism of adenosine receptors appears to be highly important for effects relating to behaviour and cognitive function. Due to this antagonism, caffeine also indirectly affects the release of norepinephrine, dopamine, acetylcholine, serotonin, glutamate, gamma-aminobutyric acid (GABA), and possibly neuropeptides. There are two main classes of adenosine receptor: A1 and A2. Caffeine and its metabolite paraxanthine are non-selective antagonists for both, with *in vivo* concentrations associated with mild CNS stimulation generally being around 5-10 µM.

The present inventors have found that the compounds of Formula (I) of the present disclosure are adenosine receptor antagonists with at least comparable potency to caffeine, as described in more detail in the Examples of the present disclosure. Accordingly, the compounds of Formula (I) of the present disclosure may be used to induce similar CNS stimulatory effects to caffeine in a subject, for example when used in an oral product as described herein. This is unexpected because compounds of Formula (I) of the present disclosure have generally been considered as antioxidants and used as such. As described further in the below disclosure, said compounds of Formula (I) may have one or more advantages over caffeine in the uses of the aspects and embodiments described herein, e.g. a more favourable profile of physiological effects, such as providing desirable CNS stimulatory effects while providing reduced undesirable effects on other physiological processes.

Thus, in various aspects, the compound of Formula (I), a derivative thereof, or a combination thereof is used as a CNS stimulant in a human or animal. In various aspects, the compound of Formula (I), a derivative thereof, or a combination thereof is used to increase cognitive performance, alertness and/or energy levels of a human or animal. In various aspects, the compound of Formula (I), a derivative thereof, or a combination thereof is used to improve feelings of being in control for a human or animal. In various aspects, the compound of Formula (I), a derivative thereof, or a combination thereof is used to reduce sleepiness in a human or animal. In various aspects, the compound of Formula (I), a derivative thereof, or a combination thereof is used to increase mental and/or physical energy of a human or animal. In various aspects, the compound of Formula (I), a derivative thereof, or a combination thereof is used to improve attention and/or concentration of a human or animal.

In various aspects, the compound of Formula (I), a derivative thereof, or a combination thereof may be associated with the rapid onset of mental and/or physical energy. In various aspects, the compound of Formula (I), a derivative thereof, or a combination thereof may provide a short, medium or long-term mental and/or physical energy boost for the user. For example, the compound of Formula (I), a derivative thereof, or a combination thereof may be associated with increased mental energy (i.e. improved cognitive performance, increased energy (alertness, arousal) or reduced mental fatigue and increased motivation of the consumer to perform mental tasks) and increased physical energy (i.e. improved endurance performance and capacity). The compound of Formula (I), a derivative thereof, or a combination thereof may be associated with improved energy levels over a short or sustained period of time. The increase in energy levels may also increase the user's overall feelings of enjoyment and enthusiasm, with feelings of confidence, motivation, pleasure and excitement being increased.

In various aspects, the compound of Formula (I), a derivative thereof, or a combination thereof may be associated with improved mental focus, alertness, attention and/or concentration of the consumer as compared with previously known products. The compound of Formula (I), a derivative thereof, or a combination thereof may also help to increase alertness and concentration over a short or sustained period of time, and also improve reaction times and overall mood with psychological scores being improved after consumption. The compound of Formula (I), a derivative thereof, or a combination thereof may provide a short-term increase in mental performance and cognition for the consumer when consumed. Cognitive and mental performance may be defined as improvement of attention, alertness, learning, memory, mood enhancement as well as resistance to stress. Attention refers to the selective ability to concentrate on one task to the exclusion of others and a sustained ability to concentrate over a period of time.

As noted above and detailed in the Examples herein below, it has also been found by the present inventors that compounds according to Formula (I) as defined herein may be associated with further advantageous physiological effects other than those arising through adenosine receptor antagonism. Accordingly, the uses and oral products of the present disclosure may exhibit an improved spectrum of physiological effects compared to the use of caffeine alone. For example, compounds according to Formula (I) have been unexpectedly found by the present inventors to be implicated in suppressing muscle activity, e.g. contraction. Such an effect may counteract undesirable effects of stimulation, such as jitteriness, that may be experienced with caffeine consumption. Thus, in some embodiments, the use further comprises reduced jitteriness in the human or animal. As used herein, the term jitteriness refers to physical sensations such as effects on muscle contraction (e.g. involuntary contractions/spams, twitching), elevated heart rate/palpitations, and/or associated feelings such as nervous energy. Jitteriness in a subject can be measured by visual analogue scales. For example, the "Caffeine Research Visual Analogue Scales (Caff-VAS)" include a "jittery" visual analogue scale, and have been previously used in research into the effects of caffeine (see Rogers PJ, Richardson NJ, Elliman NA (1995) "Overnight caffeine abstinence and negative reinforcement of preference for caffeine-containing drinks." Psychopharmacology 120:457-462; and Rogers PJ, Martin J, Smith C, Heatherley SV, Smit HJ (2003) "Absence of reinforcing, mood and psychomotor performance effects of caffeine in habitual non-consumers of caffeine" Psychopharmacology 167:54-62).

In some embodiments, the reduced jitteriness in the human or animal is relative to the use of caffeine in said human or animal. For example, a lower degree of jitteriness (e.g. as measured by a visual analogue scale as detailed above) may be measured in subjects at one or more time-points after consumption of an oral product (such as those described herein) comprising a compound of Formula (I), a derivative thereof, or a combination thereof as defined herein, when compared to subjects that have consumed caffeine, e.g. in a equivalent oral product that contains caffeine instead of a compound of Formula (I), a derivative thereof, or a combination thereof according to the present disclosure. Put differently, the reduced jitteriness may be relative to the use of caffeine alone. In some embodiments, reduced jitteriness may be observed versus a placebo, i.e. an equivalent oral product that does not contain any active ingredients. In any of the foregoing embodiments, the reduced jitteriness may be experienced when comparing an equivalent amount (e.g. by weight) of the compound of Formula (I), derivative thereof, or combination thereof vs an amount of caffeine. In other embodiments, the reduced jitteriness may be experienced when comparing a lower amount (e.g. by weight) of the compound of Formula (I), derivative thereof, or combination thereof relative to caffeine. Such amounts may be as further described herein below for each of the compound of Formula (I), derivative thereof, or combination thereof, and caffeine. A person of skill in the art will further recognise that reduced jitteriness as defined herein may also contribute to other effects, for example feelings of relaxation, reduced anxiety, and/or reduced tension.

In some embodiments, the compound of Formula (I), a derivative thereof, or a combination thereof is used in an oral product according to the disclosure herein below.

In some embodiments, the compound of Formula (I), a derivative thereof, or a combination thereof may be used in combination with one or more B vitamins selected from B1, B2, B6, and B7.

In some embodiments, the compound of Formula (I), a derivative thereof, or a combination thereof is used in combination with one or more B vitamins selected from B1, B2, B6 and B7; or a compound selected from maca root, ginseng, coenzyme Q10, caffeine, vitamin C, beta-alanine, theacrine, taurine, zinc or a salt thereof, L-theanine, theobromine, Echinacea, selenium, lemon balm, chamomile, L-tryptophan, piperine or a geometric isomer thereof, saffron, or a combination thereof.

In some embodiments, the compound of Formula (I), a derivative thereof, or a combination thereof is used in combination with any of the additional active ingredients as described herein below.

In further aspects, the uses of the present disclosure may be associated with one or more effects including boosting immune response or supporting immunity as compared with previously known products, supporting resilience of a human or animal against infection, decreasing stress and anxiety in a human or animal, providing short-term mood enhancement, increasing sexual drive and/or desire for sexual activity in a human or animal, and increasing mental and/or physical activity of a human or animal.

Such effects may be associated with the use of the compound of Formula (I), derivative thereof, or combination thereof. In any of the foregoing aspects and embodiments, some or all of the effects may be associated with the use of the compound of Formula (I), derivative thereof, or combination thereof when comprised in combination with further actives, for example in an oral product according to the disclosure herein. In such a combination of actives, the active ingredients other than the compound of Formula (I), derivative thereof, or combination thereof may be any of the active ingredients described herein. In some embodiments, the effect or combination of effects obtained from a combination of active ingredients may not be obtainable when each of the active ingredients is used in isolation.

Such effects may be measured by consumer testing. For example, pleasure/arousal/dominance may be measured by the SAM (self-assessment manikin) technique, anger/confusion/depression/tension may be measured by POMS-SF (profile of mood states), galvanic skin response may be measured e.g. by Biopac monitors, contentedness may be measured by STAI-State (State Trait Anxiety Inventory-State), "de-stress"/tranquil may be measured by visual analogue mood scale (VAMS), calmness/nervousness/"in control" may be measured by visual analogue scale (VAS), vigour/friendliness/fatigue/anger/confusion/dejection/tension may be measured by POMS-SF, and general wellbeing/mood may be measured by Quality of Life Escale (QOLS) and/or STAI-State. Cognitive accuracy may be measured using a cognitive battery including RVIP (Rapid Visual Information Processing), mental arithmetic tests (e.g. serial -3 substraction), numeric working memory (NWM), and Corsi blocks. Alertness, physical energy, fatigue reduction, feeling of stimulation, and vigour may be measured using VAS. Mental fatigue may be measured by subjecting users to cognitively demanding tasks intended to cause fatigue and then assessment by POMS. Stimulation can be measured using VAS. Cognitive speed may be measured via immediate or delayed word recognition (e.g. using the COMPASS tool), and/or NWM tests. Intimacy, pleasure, sensation, arousal, "in the moment" and motivation may be measured using VAS. Sexual desire may be measured using the Sexual Desire Inventory (SDI) questionnaire (see Spector, Carey, Steinberg, J Sex Marital Ther (1996)).

It will be understood that all disclosure herein concerning the oral product and compound of Formula (I) applies to the disclosed uses. The disclosure is not repeated for conciseness.

### Compound of Formula (I)

The present disclosure relates to uses of a compound of Formula (I), a derivative thereof, or a combination thereof; and oral products comprising the same: 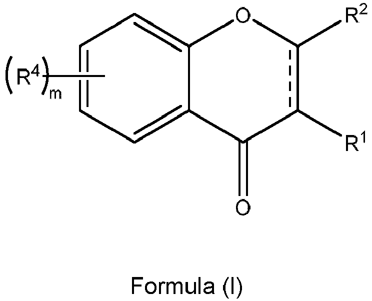 wherein:
(i) R¹ is H, OH, O-alkyl, O-alkenyl, SH, alkylthio, alkenylthio, NH₂, alkylamino, dialkylamino, alkenylamino, or dialkenylamino; and R² is an optionally substituted phenyl group; or
(ii) R¹ is an optionally substituted phenyl group; and R² is H, OH, O-alkyl, O-alkenyl, SH, alkylthio, alkenylthio, NH₂, alkylamino, dialkylamino, alkenylamino, or dialkenylamino;

wherein the optional substituents of the phenyl groups are independently selected from OH, O-alkyl, O-alkenyl, SH, alkylthio, alkenylthio, NH₂, alkylamino, dialkylamino, alkenylamino, and dialkenylamino;
each R⁴ is independently selected from OH, O-alkyl, O-alkenyl, SH, alkylthio, alkenylthio, NH₂, alkylamino, dialkylamino, and dialkenylamino; and
m is an integer from 0 to 4.

In any of the aspects described herein, the compound of Formula (I) may be further defined according to any of the further embodiments described herein below. It will be understood that said embodiments of the compound of Formula (I) may be combined with any of the embodiments of the oral product and uses thereof disclosed herein.

All aspects include, where appropriate, all enantiomers and tautomers of the compounds of formula (I). The person skilled in the art will recognise compounds that possess optical properties (one or more chiral carbon atoms) or tautomeric characteristics. The corresponding enantiomers and/or tautomers may be isolated/prepared by methods known in the art. Some of the compounds of Formula (I) may exist as stereoisomers and/or geometric isomers - e.g. they may possess one or more asymmetric and/or geometric centers and so may exist in two or more stereoisomeric and/or geometric forms. All aspects include, where appropriate, the use of all the individual stereoisomers and geometric isomers of those compounds, and mixtures thereof. The terms used in the claims encompass these forms.

The compound of Formula (I) or derivative thereof of the present disclosure may be used/present in different solid forms such as different crystalline forms or in the form of an amorphous solid. The present disclosure encompasses different crystalline forms as well as amorphous forms of said compound.

As used herein, the term "alkyl" refers to substituted or unsubstituted, saturated straight, branched, primary, secondary or tertiary hydrocarbons. The alkyl group may, for instance, be a C₁-C₁₂ group, which is an alkyl group having 1 to 12 carbon atoms. In some embodiments, alkyl groups will include C₁-C₁₀, C₁-C₈, C₁-C₆, C₁-C₄ or C₁-C₃ alkyl groups. Examples of C₁-C₁₀ alkyl groups include, but are not limited to, methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, heptyl, octyl, 2-ethylhexyl, nonyl and decyl and their isomers. Examples of C₁-C₆ alkyl groups include, but are not limited to, methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, and their isomers. C₁-C₄-alkyl means, for example, methyl, ethyl, propyl, 1-methylethyl, butyl, 1-methylpropyl, 2-methylpropyl or 1,1-dimethylethyl.

Cyclic alkyl groups, may be referred to as "cycloalkyl" and include those with 3 to 10 carbon atoms having single or multiple fused rings. Non-limiting examples of cycloalkyl groups include adamantyl, cyclopropyl, cyclobutyl, cyclopentyl, cyclohexyl, cycloheptyl, cyclooctyl and the like.

Carbocyclic groups are cyclic groups composed exclusively of carbon. The carbocyclic groups include both aromatic rings such as phenyl and non-aromatic rings such cyclohexyl and include those with 3 to 14 carbon atoms having single or multiple fused rings.

The term "alkenyl" refers to both straight and branched carbon chains, substituted or unsubstituted, which have at least one carbon-carbon double bond. In some embodiments, alkenyl groups may include C₂ -C₁₂ alkenyl groups. In other embodiments, alkenyl includes C₂-C₁₀, C₂-C₈, C₂-C₆ or C₂-C₄ alkenyl groups. In one embodiment of alkenyl, the number of double bonds is 1-3; in another embodiment of alkenyl, the number of double bonds is one. Other ranges of carbon-carbon double bonds and carbon numbers are also contemplated depending on the location of the alkenyl moiety on the molecule. "C₂-C₁₀-alkenyl" groups may include more than one double bond in the chain. Examples of C₂-C₆ alkenyl groups include, but are not limited to, ethenyl, 1-propenyl, 2-propenyl, 1-methyl-ethenyl, 1-butenyl, 2-butenyl, 3-butenyl, 1-methyl-1-propenyl, 2-methyl-1-propenyl, 1-methyl-2-propenyl, 2-methyl-2-propenyl; 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl.

The alkyl and cycloalkyl and carbocyclic groups described herein can be unsubstituted or substituted with one or more moieties selected from the group consisting of alkyl, halo, haloalkyl, hydroxyl, carboxyl, acyl, acyloxy, amino, alkyl- or dialkylamino, amido, arylamino, alkoxy, aryloxy, nitro, cyano, azido, thiol, imino, sulfonic acid, sulfate, sulfonyl, sulfanyl, sulfinyl, sulfamoyl, ester, phosphonyl, phosphinyl, phosphoryl, phosphine, thioester, thioether, acid halide, anhydride, oxime, hydrazine, carbamate, phosphonic acid, phosphate, phosphonate, or any other viable functional group that does not inhibit the biological activity of the compounds of the invention, either unprotected, or protected as necessary, as known to those skilled in the art, for example, as taught in Greene and Wuts, Protective Groups in Organic Synthesis, John Wiley and Sons, Third Edition, 1999, hereby incorporated by reference."Cycloalkenyl" refers to monovalent cyclic alkenyl groups of from 4 to 10 carbon atoms having single or multiple fused rings which fused rings may or may not be cycloalkenyl provided that the point of attachment is to a cycloalkenyl ring atom. Examples of cycloalkenyl groups include, by way of example, cyclobutenyl, cyclopentenyl, cyclohexenyl, cycloheptenyl, cyclooctenyl and the like. Alkenyl and cycloalkenyl groups may be unsubstituted or substituted with one or more substituents as described for alkyl above.

"Alkynyl" refers to both straight and branched carbon chains, unsubstituted or substituted, which have at least one carbon-carbon triple bond. In one embodiment of alkynyl, the number of triple bonds is 1-3; in another embodiment of alkynyl, the number of triple bonds is one. In some embodiments, alkynyl groups include from 2 to 12 carbon atoms. In other embodiments, alkynyl groups may include C₂-C₁₀, C₂-C₈, C₂-C₆ or C₂-C₄ alkynyl groups. Other ranges of carbon-carbon triple bonds and carbon numbers are also contemplated depending on the location of the alkenyl moiety on the molecule. For example, the term "C₂-C₁₀-alkynyl" as used herein refers to a straight-chain or branched unsaturated hydrocarbon group having 2 to 10 carbon atoms and containing at least one triple bond, such as ethynyl, prop-1-yn-1-yl, prop-2-yn-1-yl, n-but-1-yn-1-yl, n-but-1-yn-3-yl, n-but-1-yn-4-yl, n-but-2-yn-1-yl, n-pent-1-yn-1-yl, n-pent-1-yn-3-yl, n-pent-1-yn-4-yl, n-pent-1-yn-5-yl, n-pent-2-yn-1-yl, n-pent-2-yn-4-yl, n-pent-2-yn-5-yl, 3-methylbut-1-yn-3-yl, 3-methylbut-1-yn-4-yl, n-hex-1-yn-1-yl, n-hex-1-yn-3-yl, n-hex-1-yn-4-yl, n-hex-1-yn-5-yl, n-hex-1-yn-6-yl, n-hex-2-yn-1-yl, n-hex-2-yn-4-yl, n-hex-2-yn-5-yl, n-hex-2-yn-6-yl, n-hex-3-yn-1-yl, n-hex-3-yn-2-yl, 3-methylpent-1-yn-1-yl, 3-methylpent-1-yn-3-yl, 3-methylpent-1-yn-4-yl, 3-methylpent-1-yn-5-yl, 4-methylpent-1-yn-1-yl, 4-methylpent-2-yn-4-yl or 4-methylpent-2-yn-5-yl and the like.

The term "haloalkyl" refers to an alkyl group, as defined herein, which is substituted by one or more halogen atoms. For example, C₁-C₄-haloalkyl includes, but is not limited to, chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl and the like.

The term "haloalkenyl" refers to an alkenyl group, as defined herein, which is substituted by one or more halogen atoms.

The term "haloalkynyl" refers to an alkynyl group, as defined herein, which is substituted by one or more halogen atoms.

"Alkoxy" refers to alkyl-O- or O-alkyl (the terms being used interchangeably herein), wherein alkyl is as defined above. Similarly, the terms "alkenyloxy," "alkynyloxy," "haloalkoxy," "haloalkenyloxy," "haloalkynyloxy," "cycloalkoxy," "cycloalkenyloxy," "halocycloalkoxy," and "halocycloalkenyloxy" refer to the groups alkenyl-O-, alkynyl-O-, haloalkyl-O-, haloalkenyl-O-, haloalkynyl-O-, cycloalkyl-O-, cycloalkenyl-O-, halocycloalkyl-O-, and halocycloalkenyl-O-, respectively, wherein alkenyl, alkynyl, haloalkyl, haloalkenyl, haloalkynyl, cycloalkyl, cycloalkenyl, halocycloalkyl, and halocycloalkenyl are as defined above. Examples of C₁-C₆-alkoxy include, but are not limited to, methoxy, ethoxy, OCH₂-C₂H₅, OCH(CH₃)₂, n-butoxy, OCH(CH₃)-C₂H₅, OCH₂-CH(CH₃)₂, OC(CH₃)₃, n-pentoxy, 1-methylbutoxy, 2-methylbutoxy, 3-methylbutoxy, 1,1-dimethylpropoxy, 1,2-dimethylpropoxy, 2,2-dimethyl-propoxy, 1-ethylpropoxy, n-hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy, 1-ethyl-2-methylpropoxy and the like.

"Aryl" refers to an unsubstituted or substituted monovalent aromatic carbocyclic group of from 6 to 14 carbon atoms having a single ring or multiple fused rings. Aryl groups include, but are not limited to, phenyl, biphenyl, tolyl, xylyl, and naphthyl. In some embodiments aryl includes tetrahydronaphthyl, phenylcyclopropyl and indanyl. Aryl groups may be unsubstituted or substituted by one or more moieties selected from halogen, cyano, nitro, hydroxy, mercapto, amino, alkyl, alkenyl, alkynyl, cycloalkyl, cycloalkenyl, haloalkyl, haloalkenyl, haloalkynyl, halocycloalkyl, halocycloalkenyl, alkoxy, alkenyloxy, alkynyloxy, haloalkoxy, haloalkenyloxy, haloalkynyloxy, cycloalkoxy, cycloalkenyloxy, halocycloalkoxy, halocycloalkenyloxy, alkylthio, haloalkylthio, cycloalkylthio, halocycloalkylthio, alkylsulfinyl, alkenylsulfinyl, alkynyl-sulfinyl, haloalkylsulfinyl, haloalkenylsulfinyl, haloalkynylsulfinyl, alkylsulfonyl, alkenylsulfonyl, alkynylsulfonyl, haloalkyl-sulfonyl, haloalkenylsulfonyl, haloalkynylsulfonyl, -SF₅, alkylamino, alkenylamino, alkynylamino, di(alkyl)amino, di(alkenyl)-amino, di(alkynyl)amino, or trialkylsilyl.

The term "aralkyl" refers to an aryl group that is bonded to the parent compound through a diradical alkylene bridge, (-CH₂-)ₙ, where n is 1-12 and where "aryl" is as defined above.

"Heteroaryl" refers to a monovalent aromatic group of from 1 to 15 carbon atoms having one or more oxygen, nitrogen, and sulfur heteroatoms within the ring, preferably 1 to 4 heteroatoms. The nitrogen and sulfur heteroatoms may optionally be oxidized. Such heteroaryl groups can have a single ring (e.g., pyridyl or furyl) or multiple fused rings provided that the point of attachment is through a heteroaryl ring atom. Examples of heteroaryls include pyridyl, pyridazinyl, pyrimidinyl, pyrazinyl, triazinyl, pyrrolyl, indolyl, quinolinyl, isoquinolinyl, quinazolinyl, quinoxalinnyl, furanyl, thiophenyl, furyl, pyrrolyl, imidazolyl, oxazolyl, isoxazolyl, isothiazolyl, pyrazolyl benzofuranyl, benzothiophenyl, imidazopyridyl, imidazopyrimidyl, or pyrrolopyrimidyl. Heteroaryl rings may be unsubstituted or substituted by one or more moieties as described for aryl above.

"Heterocyclyl," "heterocyclic" or "heterocyclo" refers to fully saturated or unsaturated, cyclic groups, for example, 3 to 7 membered monocyclic, 7 to 11 membered bicyclic, or 10 to 15 membered tricyclic ring systems, which have one or more oxygen, sulfur or nitrogen heteroatoms in ring, preferably 1 to 4 heteroatoms. The nitrogen and sulfur heteroatoms may optionally be oxidized and the nitrogen heteroatoms may optionally be quaternized. The heterocyclic group may be attached at any heteroatom or carbon atom of the ring or ring system and may be unsubstituted or substituted by one or more moieties as described for aryl groups above.

Exemplary monocyclic heterocyclic groups include, but are not limited to, aziridinyl, azetidinyl, oxetanyl, pyrrolidinyl, pyrrolyl, pyrazolyl, oxetanyl, pyrazolinyl, imidazolyl, imidazolinyl, imidazolidinyl, oxazolyl, oxazolidinyl, isoxazolinyl, isoxazolyl, thiazolyl, thiadiazolyl, thiazolidinyl, isothiazolyl, isothiazolidinyl, furyl, tetrahydrofuryl, thienyl, oxadiazolyl, piperidinyl, piperazinyl, 2-oxopiperazinyl, 2-oxopiperidinyl, 2-oxopyrrolodinyl, 2-oxoazepinyl, azepinyl, 4-piperidonyl, pyridinyl, pyrazinyl, pyrimidinyl, pyridazinyl, tetrahydropyranyl, morpholinyl, thiamorpholinyl, thiamorpholinyl sulfoxide, thiamorpholinyl sulfone, 1,3-dioxolane and tetrahydro-1,1-dioxothienyl, triazolyl, triazinyl, and the like.

Exemplary bicyclic heterocyclic groups include, but are not limited to, indolyl, benzothiazolyl, benzoxazolyl, benzodioxolyl, benzothienyl, quinuclidinyl, quinolinyl, tetra-hydroisoquinolinyl, isoquinolinyl, benzimidazolyl, benzopyranyl, indolizinyl, benzofuryl, chromonyl, coumarinyl, benzopyranyl, cinnolinyl, quinoxalinyl, indazolyl, pyrrolopyridyl, furopyridinyl (such as furo[2,3-c]pyridinyl, furo[3,2-b]pyridinyl]or furo[2,3-b]pyridinyl), dihydroisoindolyl, dihydroquinazolinyl (such as 3,4-dihydro-4-oxo-quinazolinyl), tetrahydroquinolinyl and the like.

The term "alkylthio" refers to alkyl-S-, where "alkyl" is as defined above. In some embodiments, the alkyl component of the alkylthio group will include C₁-C₁₀, C₁-C₈, C₁-C₆, C₁-C₄ or C₁-C₃ alkyl groups. For example, C₁-C₆ alkylthio include, but are not limited to, methylthio, ethylthio, propylthio, 1-methylethylthio, butylthio, 1-methylpropylthio, 2-methylpropylthio, 1,1-dimethylethylthio, pentylthio, 1-methylbutylthio, 2-methylbutylthio, 3-methylbutylthio, 2,2-dimethylpropylthio, 1-ethylpropylthio, hexylthio, 1,1-dimethylpropylthio, 1,2-dimethylpropylthio, 1-methylpentylthio, 2-methylpentylthio, 3-methylpentylthio, 4-methylpentylthio, 1,1-dimethylbutylthio, 1,2-dimethylbutylthio, 1,3-dimethylbutylthio, 2,2-dimethylbutylthio, 2,3-dimethylbutylthio, 3,3-dimethylbutylthio, 1-ethylbutylthio, or 2-ethylbutylthio. C₁-C₄-alkylthio include, but are not limited to, methylthio, ethylthio, propylthio, 1-methylethylthio, butylthio, 1-methylpropylthio, 2-methylpropylthio or 1,1-dimethylethylthio.

Similarly, the terms "haloalkylthio," "cycloalkylthio," "halocycloalkylthio" refer to the groups -S-haloalkyl, -S-cycloalkyl, and -S-halocycloalkyl, respectively, where the terms "haloalkyl," "cycloalkyl," and "halocycloalkyl" are as defined above.

The term alkenylthio refers to alkenyl-S-, where "alkenyl" is as defined above. In some embodiments, the alkenyl component of the alkenylthio group will include C₂-C₁₀, C₂-C₈, C₂-C₆, C₂-C₄ or C₂-C₃ alkenyl groups. For example, C₂-C₆ alkenylthio include, but are not limited to ethenylthio, 1-propenylthio, 2-propenylthio, 1-methyl-ethenylthio, 1-butenylthio, 2-butenylthio, 3-butenylthio, 1-methyl-1-propenylthio, 2-methyl-1-propenylthio, 1-methyl-2-propenylthio, 2-methyl-2-propenylthio; 1-pentenylthio, 2-pentenylthio, 3-pentenylthio, 4-pentenylthio, 1-methyl-1-butenylthio, 2-methyl-1-butenylthio, 3-methyl-1-butenylthio, 1-methyl-2-butenylthio, 2-methyl-2-butenylthio, 3-methyl-2-butenylthio, 1-methyl-3-butenylthio, 2-methyl-3-butenylthio, 3-methyl-3-butenylthio, 1,1-dimethyl-2-propenylthio, 1,2-dimethyl-1-propenylthio, 1,2-dimethyl-2-propenylthio, 1-ethyl-1-propenylthio, 1-ethyl-2-propenylthio, 1-hexenylthio, 2-hexenylthio, 3-hexenylthio, 4-hexenylthio, 5-hexenylthio, 1-methyl-1-pentenylthio, 2-methyl-1-pentenylthio, 3-methyl-1-pentenylthio, 4-methyl-1-pentenylthio, 1-methyl-2-pentenylthio, 2-methyl-2-pentenylthio, 3-methyl-2-pentenylthio, 4-methyl-2-pentenylthio, 1-methyl-3-pentenylthio, 2-methyl-3-pentenylthio, 3-methyl-3-pentenylthio, 4-methyl-3-pentenylthio, 1-methyl-4-pentenylthio, 2-methyl-4-pentenylthio, 3-methyl-4-pentenylthio, 4-methyl-4-pentenylthio, 1,1-dimethyl-2-butenylthio, 1,1-dimethyl-3-butenylthio, 1,2-dimethyl-1-butenylthio, 1,2-dimethyl-2-butenylthio, 1,2-dimethyl-3-butenylthio, 1,3-dimethyl-1-butenylthio, 1,3-dimethyl-2-butenylthio, 1,3-dimethyl-3-butenylthio, 2,2-dimethyl-3-butenylthio, 2,3-dimethyl-1-butenylthio, 2,3-dimethyl-2-butenylthio, 2,3-dimethyl-3-butenylthio, 3,3-dimethyl-1-butenylthio, 3,3-dimethyl-2-butenylthio, 1-ethyl-1-butenylthio, 1-ethyl-2-butenylthio, 1-ethyl-3-butenylthio, 2-ethyl-1-butenylthio, 2-ethyl-2-butenylthio, 2-ethyl-3-butenylthio, 1,1,2-trimethyl-2-propenylthio, 1-ethyl-1-methyl-2-propenylthio, 1-ethyl-2-methyl-1-propenylthio or 1-ethyl-2-methyl-2-propenylthio.

The term "alkylsulfinyl" refers to the group alkyl-S(=O)-, where "alkyl" is as defined above. In some embodiments, the alkyl component in alkylsulfinyl groups will include C₁-C₁₂, C₁-C₁₀, C₁-C₈, C₁-C₆, C₁-C₄ or C₁-C₃ alkyl groups. Examples include, but are not limited to, - SO-CH₃, -SO-C₂H₅, n-propylsulfinyl, 1-methylethylsulfinyl, n-butylsulfinyl, 1-methylpropylsulfinyl, 2-methylpropylsulfinyl, 1,1-dimethylethylsulfinyl, n-pentylsulfinyl, 1-methylbutylsulfinyl, 2-methylbutylsulfinyl, 3-methylbutylsulfinyl, 1,1-dimethylpropylsulfinyl, 1,2-dimethylpropylsulfinyl, 2,2-dimethylpropylsulfinyl, 1-ethylpropylsulfinyl, n-hexylsulfinyl, 1-methylpentylsulfinyl, 2-methylpentylsulfinyl, 3-methylpentylsulfinyl, 4-methylpentylsulfinyl, 1,1-dimethylbutylsulfinyl, 1,2-dimethylbutylsulfinyl, 1,3-dimethylbutylsulfinyl, 2,2-dimethylbutylsulfinyl, 2,3-dimethylbutylsulfinyl, 3,3-dimethylbutylsulfinyl, 1-ethylbutylsulfinyl, 2-ethylbutylsulfinyl, 1,1,2-trimethylpropylsulfinyl, 1,2,2-trimethylpropylsulfinyl, 1-ethyl-1-methylpropylsulfinyl or 1-ethyl-2-methylpropylsulfinyl.

Similarly, the terms "alkenylsulfinyl," "alkynylsulfinyl," "haloalkylsulfinyl," "haloalkenylsulfinyl," and "haloalkynylsulfinyl" refer to the groups alkenyl-S(=O)-, alkynyl-S(=O)-, and haloalkyl-S(=O)-, haloalkenyl-S(=O)-, and haloalkynyl-S(=O)-, where the terms "alkenyl," "alkynyl," "haloalkyl," "haloalkenyl," and "haloalkynyl" are as defined above.

The term "alkylsulfonyl" refers to the group alkyl-S(=O)z-, where the term "alkyl" is as defined above. In some embodiments, the alkyl component in alkylsulfonyl groups will include C₁-C₁₂, C₁-C₁₀, C₁-C₈, C₁-C₆ or C₁-C₄ alkyl groups. Examples include, but are not limited to, - SO₂-CH₃, -SO₂-C₂H₅, n-propylsulfonyl, -SO₂-CH(CH₃)₂, n-butylsulfonyl, 1-methylpropylsulfonyl, 2-methylpropylsulfonyl, -SO₂-C(CH₃)₃, n-pentylsulfonyl, 1-methylbutylsulfonyl, 2-methylbutylsulfonyl, 3-methylbutylsulfonyl, 1,1-dimethylpropylsulfonyl, 1,2-dimethylpropylsulfonyl, 2,2-dimethylpropylsulfonyl, 1-ethylpropylsulfonyl, n-hexylsulfonyl, 1-methylpentylsulfonyl, 2-methylpentylsulfonyl, 3-methylpentylsulfonyl, 4-methylpentylsulfonyl, 1,1-dimethylbutylsulfonyl, 1,2-dimethylbutylsulfonyl, 1,3-dimethylbutylsulfonyl, 2,2-dimethylbutylsulfonyl, 2,3-dimethylbutylsulfonyl, 3,3-dimethylbutylsulfonyl, 1-ethylbutylsulfonyl, 2-ethylbutylsulfonyl, 1,1,2-trimethylpropylsulfonyl, 1,2,2-trimethylpropylsulfonyl, 1-ethyl-1-methylpropylsulfonyl or 1-ethyl-2-methylpropylsulfonyl and the like.

The terms "alkenylsulfonyl," "alkynylsulfonyl," "haloalkylsulfonyl," "haloalkenylsulfonyl," and "haloalkynylsulfonyl" refer to the groups alkenyl-S(=O)z-, alkynyl-S(=O)z-, and haloalkylS(=O)₂-, haloalkenyl-S(=O)₂-, and haloalkynyl-S(=O)₂-, where the terms "alkenyl," "alkynyl," "haloalkyl," "haloalkenyl," and "haloalkynyl" are as defined above.

The terms "alkylamino," "dialkylamino," "alkenylamino," "alkynylamino," "di(alkenyl)amino," and "di(alkynyl)amino" refer to the groups -NH(alkyl), -N(alkyl)₂, - NH(alkenyl), -NH(alkynyl), -N(alkenyl)₂ and -N(alkynyl)₂, where the terms "alkyl," "alkenyl," and "alkynyl" are as defined above. Also contemplated are the groups -N(alkyl)(alkenyl) wherein the terms "alkyl" and "alkenyl" are as defined above. In some embodiments, the alkyl component in alkylamino or dialkylamino groups will include C₁-C₁₂, C₁-C₁₀, C₁-C₈, C₁-C₆ or C₁-C₃ alkyl groups. In some embodiments, the alkenyl component in alkenylamino or dialkenylamino groups will include C₁-C₁₂, C₁-C₁₀, C₁-C₈, C₁-C₆ or C₁-C₃ alkenyl groups.

In various embodiments of the compound of Formula (I) wherein (i) R² is an optionally substituted phenyl group, R¹ is H, OH, O-alkyl, O-alkenyl, SH, alkylthio, alkenylthio, NH₂, alkylamino, dialkylamino, alkenylamino or dialkenylamino. In various embodiments, R¹ is H, OH, O-alkyl, O-alkenyl, alkylthio, alkenylthio, NH₂, alkylamino, dialkylamino, alkenylamino or dialkenylamino. In various embodiments, R¹ is H, OH, O-alkyl, O-alkenyl, alkylthio, alkenylthio, alkylamino, dialkylamino, alkenylamino or dialkenylamino. In various embodiments, R¹ is H, OH, O-alkyl, O-alkenyl, alkylamino, or dialkylamino. In various embodiments, R¹ is H, OH, O-alkyl, or O-alkenyl. In various embodiments, R¹ is H, OH or O-alkyl. In such embodiments, the alkyl groups may be preferably C₁₋₆ alkyl groups and the alkenyl groups may be preferably C₂₋₆ alkenyl groups. More preferably, the alkyl groups may be C₁₋₃ alkyl groups and the alkenyl groups may be preferably C₂₋₃ alkenyl groups.

In various embodiments of the compound of Formula (I) wherein (i) R² is an optionally substituted phenyl group, R¹ is H, OH, OMe, methylthio, methylamino, or dimethylamino. In various embodiments, each R¹ is H, OH, OMe, methylamino, or dimethylamino. In some embodiments, R¹ is H, OH and OMe.

In various embodiments of the compound of Formula (I) wherein (i) R¹ is an optionally substituted phenyl group, R² is H, OH, O-alkyl, O-alkenyl, SH, alkylthio, alkenylthio, NH₂, alkylamino, dialkylamino, alkenylamino or dialkenylamino. In various embodiments, R² is H, OH, O-alkyl, O-alkenyl, alkylthio, alkenylthio, NH₂, alkylamino, dialkylamino, alkenylamino or dialkenylamino. In various embodiments, R² is H, OH, O-alkyl, O-alkenyl, alkylthio, alkenylthio, alkylamino, dialkylamino, alkenylamino or dialkenylamino. In various embodiments, R² is H, OH, O-alkyl, O-alkenyl, alkylamino, or dialkylamino. In various embodiments, R² is H, OH, O-alkyl, or O-alkenyl. In various embodiments, R² is H, OH or O-alkyl. In such embodiments, the alkyl groups may be preferably C₁₋₆ alkyl groups and the alkenyl groups may be preferably C₂₋₆ alkenyl groups. More preferably, the alkyl groups may be C₁₋₃ alkyl groups and the alkenyl groups may be preferably C₂₋₃ alkenyl groups.

In various embodiments of the compound of Formula (I) wherein (i) R¹ is an optionally substituted phenyl group, R² is H, OH, OMe, methylthio, methylamino, or dimethylamino. In various embodiments, each R² is H, OH, OMe, methylamino, or dimethylamino. In some embodiments, R² is H, OH or OMe.

In various embodiments of the compound of Formula (I), m is an integer from 0 to 4. Thus, in various embodiments, m may be 0, 1, 2, 3, or 4. In various embodiments, m is an integer from 1 to 4, such as 1, 2, 3, or 4. In various embodiments, m is an integer from 0 to 3, such as 0, 1, 2, or 3. In various embodiments, m is an integer from 1 to 3, such as 1, 2, or 3. In other embodiments, m is an integer from 2 to 4, such as 2, 3, or 4. In some embodiments, m is 2 or 3. When m is 2, in various embodiments the R⁴ groups may be at the C-5 and C-7 positions of the fused ring. When m is 3, in various embodiments the R⁴ groups may be at the C-5, C-6, C-7 positions of the fused ring.

In various embodiments of the compound of Formula (I) wherein (i) R² is an optionally substituted phenyl group, m is 1 to 4 and R¹ is H, OH, O-C₁₋₆alkyl, O-C₂₋₆alkenyl, SH, C₁₋₆alkylthio, C₂₋₆alkenylthio, NH₂, C₁₋₆alkylamino, C₁₋₆dialkylamino, C₂₋₆alkenylamino or C₂₋₆dialkenylamino. In various embodiments, m is 1 to 4 and R¹ is H, OH, O-C₁₋₆alkyl, O-C₂₋₆alkenyl, C₁₋₆alkylthio, C₂₋₆alkenylthio, NH₂, C₁₋₆alkylamino, C₁₋₆dialkylamino, C₂₋₆alkenylamino or C₂₋₆dialkenylamino. In various embodiments, m is 1 to 4 and R¹ is H, OH, O-C₁₋₆alkyl, O-C₂₋₆alkenyl, C₁₋₆alkylthio, C₂₋₆alkenylthio, C₁₋₆alkylamino, C₁₋₆dialkylamino, C₂₋₆alkenylamino or C₂₋₆dialkenylamino. In various embodiments, m is 1 to 4 and R¹ is H, OH, O-C₁₋₆alkyl, O-C₂₋₆alkenyl, C₁₋₆alkylamino, or C₁₋₆dialkylamino. In various embodiments, m is 1 to 4 and R¹ is H, OH, O-C₁₋₆alkyl, or O-C₂₋₆alkenyl. In various embodiments, m is 1 to 4 and R¹ is H, OH or O-C₁₋₆alkyl. In various embodiments of the compound of Formula (I) wherein (i) R² is an optionally substituted phenyl group, m is 1 to 4 and R¹ is H, OH, OMe, methylthio, methylamino, or dimethylamino. In various embodiments, m is 1 to 4 and each R¹ is H, OH, OMe, methylamino, or dimethylamino. In some embodiments, m is 1 to 4 and R¹ is H, OH and OMe.

In various embodiments of the compound of Formula (I) wherein (i) R² is an optionally substituted phenyl group, m is 2 or 3 and R¹ is H, OH, O-C₁₋₆alkyl, O-C₂₋₆alkenyl, SH, C₁₋₆alkylthio, C₂₋₆alkenylthio, NH₂, C₁₋₆alkylamino, C₁₋₆dialkylamino, C₂₋₆alkenylamino or C₂₋₆dialkenylamino. In various embodiments, m is 2 or 3 and R¹ is H, OH, O-C₁₋₆alkyl, O-C₂₋₆alkenyl, C₁₋₆alkylthio, C₂₋₆alkenylthio, NH₂, C₁₋₆alkylamino, C₁₋₆dialkylamino, C₂₋₆alkenylamino or C₂₋₆dialkenylamino. In various embodiments, m is 2 or 3 and R¹ is H, OH, O-C₁₋₆alkyl, O-C₂₋₆alkenyl, C₁₋₆alkylthio, C₂₋₆alkenylthio, C₁₋₆alkylamino, C₁₋₆dialkylamino, C₂₋₆alkenylamino or C₂₋₆dialkenylamino. In various embodiments, m is 2 or 3 and R¹ is H, OH, O-C₁₋₆alkyl, O-C_{2- 6}alkenyl, C₁₋₆alkylamino, or C₁₋₆dialkylamino. In various embodiments, m is 2 or 3 and R¹ is H, OH, O-C₁₋₆alkyl, or O-C₂₋₆alkenyl. In various embodiments, m is 2 or 3 and R¹ is H, OH or O-C₁₋₆alkyl. In various embodiments of the compound of Formula (I) wherein (i) R² is an optionally substituted phenyl group, m is 2 or 3 and R¹ is H, OH, OMe, methylthio, methylamino, or dimethylamino. In various embodiments, m is 2 or 3 and each R¹ is H, OH, OMe, methylamino, or dimethylamino. In some embodiments, m is 2 or 3 and R¹ is H, OH and OMe. In any of the foregoing embodiments, when m is 2 the R⁴ groups may be at the C-5 and C-7 positions of the fused ring. When m is 3, in various embodiments the R⁴ groups may be at the C-5, C-6, C-7 positions of the fused ring.

In various embodiments of the compound of Formula (I) wherein (i) R² is an optionally substituted phenyl group, m is 2 or 3 and R¹ is H, OH, O-C₁₋₃alkyl, O-C₂₋₃alkenyl, SH, C₁₋₃alkylthio, C₂₋₃alkenylthio, NH₂, C₁₋₃alkylamino, C₁₋₃dialkylamino, C₂₋₃alkenylamino or C₂₋₃dialkenylamino. In various embodiments, m is 2 or 3 and R¹ is H, OH, O-C₁₋₃alkyl, O-C₂₋₃alkenyl, C₁₋₃alkylthio, C₂₋₃alkenylthio, NH₂, C₁₋₃alkylamino, C₁₋₃dialkylamino, C₂₋₃alkenylamino or C₂₋₃dialkenylamino. In various embodiments, m is 2 or 3 and R¹ is H, OH, O-C₁₋₃alkyl, O-C₂₋₃alkenyl, C₁₋₃alkylthio, C₂₋₃alkenylthio, C₁₋₃alkylamino, C₁₋₃dialkylamino, C₂₋₃alkenylamino or C₂₋₃dialkenylamino. In various embodiments, m is 2 or 3 and R¹ is H, OH, O-C₁₋₃alkyl, O-C₂₋₃alkenyl, C₁₋₃alkylamino, or C₁₋₃dialkylamino. In various embodiments, m is 2 or 3 and R¹ is H, OH, O-C₁₋₃alkyl, or O-C₂₋₃alkenyl. In various embodiments, m is 2 or 3 and R¹ is H, OH or O-C₁₋₃alkyl. In various embodiments of the compound of Formula (I) wherein (i) R² is an optionally substituted phenyl group, m is 2 or 3 and R¹ is H, OH, OMe, methylthio, methylamino, or dimethylamino. In various embodiments, m is 2 or 3 and each R¹ is H, OH, OMe, methylamino, or dimethylamino. In some embodiments, m is 2 or 3 and R¹ is H, OH and OMe. In any of the foregoing embodiments, when m is 2 the R⁴ groups may be at the C-5 and C-7 positions of the fused ring. When m is 3, in various embodiments the R⁴ groups may be at the C-5, C-6, C-7 positions of the fused ring.

In various embodiments of the compound of Formula (I) wherein (i) R¹ is an optionally substituted phenyl group, m is 1 to 4 and R² is H, OH, O-C₁₋₆alkyl, O-C₂₋₆alkenyl, SH, C₁₋₆alkylthio, C₂₋₆alkenylthio, NH₂, C₁₋₆alkylamino, C₁₋₆dialkylamino, C₂₋₆alkenylamino or C₂₋₆dialkenylamino. In various embodiments, m is 1 to 4 and R² is H, OH, O-C₁₋₆alkyl, O-C₂₋₆alkenyl, C₁₋₆alkylthio, C₂₋₆alkenylthio, NH₂, C₁₋₆alkylamino, C₁₋₆dialkylamino, C₂₋₆alkenylamino or C₂₋₆dialkenylamino. In various embodiments, m is 1 to 4 and R² is H, OH, O-C₁₋₆alkyl, O-C₂₋₆alkenyl, C₁₋₆alkylthio, C₂₋₆alkenylthio, C₁₋₆alkylamino, C₁₋₆dialkylamino, C₂₋₆alkenylamino or C₂₋₆dialkenylamino. In various embodiments, m is 1 to 4 and R² is H, OH, O-C₁₋₆alkyl, O-C₂₋₆alkenyl, C₁₋₆alkylamino, or C₁₋₆dialkylamino. In various embodiments, m is 1 to 4 and R² is H, OH, O-C₁₋₆alkyl, or O-C₂₋₆alkenyl. In various embodiments, m is 1 to 4 and R² is H, OH or O-C₁₋₆alkyl. In various embodiments of the compound of Formula (I) wherein (i) R¹ is an optionally substituted phenyl group, m is 1 to 4 and R² is H, OH, OMe, methylthio, methylamino, or dimethylamino. In various embodiments, each R² is H, OH, OMe, methylamino, or dimethylamino. In some embodiments, m is 1 to 4 and R² is H, OH or OMe. In any of the foregoing embodiments, when m is 2 the R⁴ groups may be at the C-5 and C-7 positions of the fused ring. When m is 3, in various embodiments the R⁴ groups may be at the C-5, C-6, C-7 positions of the fused ring.

In various embodiments of the compound of Formula (I) wherein (i) R¹ is an optionally substituted phenyl group, m is 2 or 3 and R² is H, OH, O-C₁₋₆alkyl, O-C₂₋₆alkenyl, SH, C₁₋₆alkylthio, C₂₋₆alkenylthio, NH₂, C₁₋₆alkylamino, C₁₋₆dialkylamino, C₂₋₆alkenylamino or C₂₋₆dialkenylamino. In various embodiments, m is 2 or 3 and R² is H, OH, O-C₁₋₆alkyl, O-C₂₋₆alkenyl, C₁₋₆alkylthio, C₂₋₆alkenylthio, NH₂, C₁₋₆alkylamino, C₁₋₆dialkylamino, C₂₋₆alkenylamino or C₂₋₆dialkenylamino. In various embodiments, m is 2 or 3 and R² is H, OH, O-C₁₋₆alkyl, O-C₂₋₆alkenyl, C₁₋₆alkylthio, C₂₋₆alkenylthio, C₁₋₆alkylamino, C₁₋₆dialkylamino, C₂₋₆alkenylamino or C₂₋₆dialkenylamino. In various embodiments, m is 2 or 3 and R² is H, OH, O-C₁₋₆alkyl, O-C₂₋₆alkenyl, C₁₋₆alkylamino, or C₁₋₆dialkylamino. In various embodiments, m is 2 or 3 and R² is H, OH, O-C₁₋₆alkyl, or O-C₂₋₆alkenyl. In various embodiments, m is 2 or 3 and R² is H, OH or O-C₁₋₆alkyl. In various embodiments of the compound of Formula (I) wherein (i) R¹ is an optionally substituted phenyl group, m is 2 or 3 and R² is H, OH, OMe, methylthio, methylamino, or dimethylamino. In various embodiments, each R² is H, OH, OMe, methylamino, or dimethylamino. In some embodiments, m is 2 or 3 and R² is H, OH or OMe. In any of the foregoing embodiments, when m is 2 the R⁴ groups may be at the C-5 and C-7 positions of the fused ring. When m is 3, in various embodiments the R⁴ groups may be at the C-5, C-6, C-7 positions of the fused ring.

In various embodiments of the compound of Formula (I) wherein (i) R¹ is an optionally substituted phenyl group, m is 2 or 3 and R² is H, OH, O-C₁₋₃alkyl, O-C₂₋₃alkenyl, SH, C₁₋₃alkylthio, C₂₋₃alkenylthio, NH₂, C₁₋₃alkylamino, C₁₋₃dialkylamino, C₂₋₃alkenylamino or C₂₋₃dialkenylamino. In various embodiments, m is 2 or 3 and R² is H, OH, O-C₁₋₃alkyl, O-C₂₋₃alkenyl, C₁₋₃alkylthio, C₂₋₃alkenylthio, NH₂, C₁₋₃alkylamino, C₁₋₃dialkylamino, C₂₋₃alkenylamino or C₂₋₃dialkenylamino. In various embodiments, m is 2 or 3 and R² is H, OH, O-C₁₋₃alkyl, O-C₂₋₃alkenyl, C₁₋₃alkylthio, C₂₋₃alkenylthio, C₁₋₃alkylamino, C₁₋₃dialkylamino, C₂₋₃alkenylamino or C₂₋₃dialkenylamino. In various embodiments, m is 2 or 3 and R² is H, OH, O-C₁₋₃alkyl, O-C₂₋₃alkenyl, C₁₋₃alkylamino, or C₁₋₃dialkylamino. In various embodiments, m is 2 or 3 and R² is H, OH, O-C₁₋₃alkyl, or O-C₂₋₃alkenyl. In various embodiments, m is 2 or 3 and R² is H, OH or O-C₁₋₃alkyl. In various embodiments of the compound of Formula (I) wherein (i) R¹ is an optionally substituted phenyl group, m is 2 or 3 and R² is H, OH, OMe, methylthio, methylamino, or dimethylamino. In various embodiments, each R² is H, OH, OMe, methylamino, or dimethylamino. In some embodiments, m is 2 or 3 and R² is H, OH or OMe. In any of the foregoing embodiments, when m is 2 the R⁴ groups may be at the C-5 and C-7 positions of the fused ring. When m is 3, in various embodiments the R⁴ groups may be at the C-5, C-6, C-7 positions of the fused ring.

In various embodiments, each R⁴ is independently selected from OH, O-alkyl, O-alkenyl, SH, alkylthio, alkenylthio, NH₂, alkylamino, dialkylamino, alkenylamino and dialkenylamino. In various embodiments, each R⁴ is independently selected from OH, O-alkyl, O-alkenyl, alkylthio, alkenylthio, NH₂, alkylamino, dialkylamino, alkenylamino and dialkenylamino. In various embodiments, each R⁴ is independently selected from OH, O-alkyl, O-alkenyl, alkylthio, alkenylthio, alkylamino, dialkylamino, alkenylamino and dialkenylamino. In various embodiments, each R⁴ is independently selected from OH, O-alkyl, O-alkenyl, alkylamino, and dialkylamino. In various embodiments, each R⁴ is independently selected from OH, O-alkyl, and O-alkenyl. In various embodiments, each R⁴ is independently selected from OH and O-alkyl. In any of the foregoing embodiments, m may be an integer from 0 to 4, an integer from 1 to 4, an integer from 0 to 3, or an integer from 1 to 3. In any of the foregoing embodiments, m may be 2 or 3. When m is 2, in various embodiments the R⁴ groups may be at the C-5 and C-7 positions of the fused ring. When m is 3, in various embodiments the R⁴ groups may be at the C-5, C-6, C-7 positions of the fused ring.

In various embodiments m is 1 to 4 and each R⁴ is independently selected from OH, O-alkyl, O-alkenyl, alkylthio, alkenylthio, NH₂, alkylamino, dialkylamino, alkenylamino and dialkenylamino. In various embodiments, m is 1 to 4 and each R⁴ is independently selected from OH, O-alkyl, O-alkenyl, alkylthio, alkenylthio, alkylamino, dialkylamino, alkenylamino and dialkenylamino. In various embodiments, m is 1 to 4 and each R⁴ is independently selected from OH, O-alkyl, O-alkenyl, alkylamino, and dialkylamino. In various embodiments, m is 1 to 4 and each R⁴ is independently selected from OH, O-alkyl, and O-alkenyl. In various embodiments, m is 1 to 4 and each R⁴ is independently selected from OH and O-alkyl.

In various embodiments m is 2 or 3 and each R⁴ is independently selected from OH, O-alkyl, O-alkenyl, alkylthio, alkenylthio, NH₂, alkylamino, dialkylamino, alkenylamino and dialkenylamino. In various embodiments, m is 2 or 3 and each R⁴ is independently selected from OH, O-alkyl, O-alkenyl, alkylthio, alkenylthio, alkylamino, dialkylamino, alkenylamino and dialkenylamino. In various embodiments, m is 2 or 3 and each R⁴ is independently selected from OH, O-alkyl, O-alkenyl, alkylamino, and dialkylamino. In various embodiments, m is 2 or 3 and each R⁴ is independently selected from OH, O-alkyl, and O-alkenyl. In various embodiments, m is 2 or 3 and each R⁴ is independently selected from OH and O-alkyl.

In any of the foregoing embodiments, the alkyl groups may be preferably C₁₋₆ alkyl groups and the alkenyl groups may be preferably C₂₋₆ alkenyl groups. More preferably, the alkyl groups may be C₁₋₃ alkyl groups and the alkenyl groups may be preferably C₂₋₃ alkenyl groups. For instance, in various embodiments m is 2 or 3 and each R⁴ is independently selected from OH, O-C₁₋₆alkyl, O-C₂₋₆alkenyl, C₁₋₆alkylthio, C₂₋₆alkenylthio, NH₂, C₁₋₆alkylamino, C₁₋₆dialkylamino, C₂₋₆alkenylamino and C₂₋₆dialkenylamino. In various embodiments, m is 2 or 3 and each R⁴ is independently selected from OH, O-C₁₋₆alkyl, O-C₂₋₆alkenyl, C₁₋₆alkylthio, C₂₋₆alkenylthio, C₁₋₆alkylamino, C₁₋₆dialkylamino, C₂₋₆alkenylamino and C₂₋₆dialkenylamino. In various embodiments, m is 2 or 3 and each R⁴ is independently selected from OH, O-C₁₋₆alkyl, O-C₂₋₆alkenyl, C₁₋₆alkylamino, and C₁₋₆dialkylamino. In various embodiments, m is 2 or 3 and each R⁴ is independently selected from OH, O-C₁₋₆alkyl, and O-C₂₋₆alkenyl. In various embodiments, m is 2 or 3 and each R⁴ is independently selected from OH and O-C₁₋₆alkyl. In any of the foregoing embodiments, when m is 2, the R⁴ groups may be at the C-5 and C-7 positions of the fused ring. When m is 3, in various embodiments the R⁴ groups may be at the C-5, C-6, C-7 positions of the fused ring.

In various embodiments, each R⁴ is independently selected from OH, O-C₁₋₃alkyl, O-C₂₋₃alkenyl, SH, C₁₋₃alkylthio, C₂₋₃alkenylthio, NH₂, C₁₋₃alkylamino, C₁₋₃dialkylamino, C₂₋₃alkenylamino and C₂₋₃dialkenylamino. In various embodiments, each R⁴ is independently selected from OH, O-C₁₋₃alkyl, O-C₂₋₃alkenyl, C₁₋₃alkylthio, C₂₋₃alkenylthio, NH₂, C₁₋₃alkylamino, C₁₋₃dialkylamino, C₂₋₃alkenylamino and C₂₋₃dialkenylamino. In various embodiments, each R⁴ is independently selected from OH, O-C₁₋₃alkyl, O-C₂₋₃alkenyl, C₁₋₃alkylthio, C₂₋₃alkenylthio, C₁₋₃alkylamino, C₁₋₃dialkylamino, C₂₋₃alkenylamino and C₂₋₃dialkenylamino. In various embodiments, each R⁴ is independently selected from OH, O-C₁₋₃alkyl, O-C₂₋₃alkenyl, C₁₋₃alkylamino, and C₁₋₃dialkylamino. In various embodiments, each R⁴ is independently selected from OH, O-C₁₋₃alkyl, and O-C₂₋₃alkenyl. In various embodiments, each R⁴ is independently selected from OH and O-C₁₋₃alkyl. In any of the foregoing embodiments, m may be an integer from 0 to 4, an integer from 1 to 4, an integer from 0 to 3, or an integer from 1 to 3. In any of the foregoing embodiments, m may be 2 or 3.

In various embodiments m is 2 or 3 and each R⁴ is independently selected from OH, O-C₁₋₃alkyl, O-C₂₋₃alkenyl, C₁₋₃alkylthio, C₂₋₃alkenylthio, NH₂, C₁₋₃alkylamino, C₁₋₃dialkylamino, C₂₋₃alkenylamino and C₂₋₃dialkenylamino. In various embodiments, m is 2 or 3 and each R⁴ is independently selected from OH, O-C₁₋₃alkyl, O-C₂₋₃alkenyl, C₁₋₃alkylthio, C₂₋₃alkenylthio, C₁₋₃alkylamino, C₁₋₃dialkylamino, C₂₋₃alkenylamino and C₂₋₃dialkenylamino. In various embodiments, m is 2 or 3 and each R⁴ is independently selected from OH, O-C₁₋₃alkyl, O-C₂₋₃alkenyl, C₁₋₃alkylamino, and C₁₋₃dialkylamino. In various embodiments, m is 2 or 3 and each R⁴ is independently selected from OH, O-C₁₋₃alkyl, and O-C₂₋₃alkenyl. In various embodiments, m is 2 or 3 and each R⁴ is independently selected from OH and O-C₁₋₃alkyl. In any of the foregoing embodiments, when m is 2, the R⁴ groups may be at the C-5 and C-7 positions of the fused ring. When m is 3, in various embodiments the R⁴ groups may be at the C-5, C-6, C-7 positions of the fused ring.

In various embodiments, each R⁴ is independently selected from OH, OMe, methylthio, methylamino, and dimethylamino. In various embodiments, each R⁴ is independently selected from OH, OMe, methylamino, and dimethylamino. In some embodiments, each R⁴ is independently selected from OH and OMe. In various embodiments, m may be 2 or 3 and each R⁴ is independently selected from OH, OMe, methylamino, and dimethylamino. In some embodiments, m is 2 or 3 and each R⁴ is independently selected from OH and OMe. In such embodiments, when m is 2 the R⁴ groups may be at the C-5 and C-7 positions of the fused ring. When m is 3, in various embodiments the R⁴ groups may be at the C-5, C-6, C-7 positions of the fused ring.

Any of the embodiments of R⁴ disclosed herein above may be combined with the embodiments of m, R¹ and/or R² disclosed herein.

In various embodiments of the compound of Formula (I) wherein (i) R² is an optionally substituted phenyl group, R¹ is H, OH, O-C₁₋₆alkyl, O-C₂₋₆alkenyl, SH, C₁₋₆alkylthio, C₂₋₆alkenylthio, NH₂, C₁₋₆alkylamino, C₁₋₆dialkylamino, C₂₋₆alkenylamino, or C₂₋₆dialkenylamino, and each R⁴ is independently selected from OH, O-C₁₋₆alkyl, O-C₂₋₆alkenyl, SH, C₁₋₆alkylthio, C₂₋₆alkenylthio, NH₂, C₁₋₆alkylamino, C₁₋₆dialkylamino, C₂₋₆alkenylamino, or C₂₋₆dialkenylamino. In various embodiments, wherein (i) R² is an optionally substituted phenyl group, R¹ is H, OH, O-C₁₋₃alkyl, O-C₂₋₃alkenyl, SH, C₁₋₃alkylthio, C₂₋₃alkenylthio, NH₂, C₁₋₃alkylamino, C₁₋₃dialkylamino, C₂₋₃alkenylamino, or C₂₋₃dialkenylamino, and each R⁴ is independently selected from OH, O-C₁₋₃alkyl, O-C₂₋₃alkenyl, SH, C₁₋₃alkylthio, C₂₋₃alkenylthio, NH₂, C₁₋₃alkylamino, C₁₋₃dialkylamino, C₂₋₃alkenylamino, or C₂₋₃dialkenylamino.

In various embodiments of the compound of Formula (I) wherein (i) R² is an optionally substituted phenyl group, R¹ is H, OH or O-C₁₋₆alkyl, and each R⁴ is independently selected from OH and O-C₁₋₆alkyl. In various embodiments of the compound of Formula (I) wherein (i) R² is an optionally substituted phenyl group, R¹ is H, OH or O-C₁₋₃alkyl; and each R⁴ is independently selected from OH and O-C₁₋₃alkyl. In various embodiments wherein (i) R² is an optionally substituted phenyl group, R¹ is H, OH or OMe; and each R⁴ is independently selected from OH and OMe. In any of the foregoing embodiments, m may be 2 or 3. In such embodiments, when m is 2 the R⁴ groups may be at the C-5 and C-7 positions of the fused ring. When m is 3, in various embodiments the R⁴ groups may be at the C-5, C-6, C-7 positions of the fused ring.

In various embodiments of the compound of Formula (I) wherein (i) R¹ is an optionally substituted phenyl group, R² is H, OH, O-C₁₋₆alkyl, O-C₂₋₆alkenyl, SH, C₁₋₆alkylthio, C₂₋₆alkenylthio, NH₂, C₁₋₆alkylamino, C₁₋₆dialkylamino, C₂₋₆alkenylamino, or C₂₋₆dialkenylamino, and each R⁴ is independently selected from OH, O-C₁₋₆alkyl, O-C₂₋₆alkenyl, SH, C₁₋₆alkylthio, C₂₋₆alkenylthio, NH₂, C₁₋₆alkylamino, C₁₋₆dialkylamino, C₂₋₆alkenylamino, or C₂₋₆dialkenylamino. In various embodiments wherein (i) R¹ is an optionally substituted phenyl group R² is H, OH, O-C₁₋₃alkyl, O-C₂₋₃alkenyl, SH, C₁₋₃alkylthio, C₂₋₃alkenylthio, NH₂, C₁₋₃alkylamino, C₁₋₃dialkylamino, C₂₋₃alkenylamino, or C₂₋₃dialkenylamino, and each R⁴ is independently selected from OH, O-C₁₋₃alkyl, O-C₂₋₃alkenyl, SH, C₁₋₃alkylthio, C₂₋₃alkenylthio, NH₂, C₁₋₃alkylamino, C₁₋₃dialkylamino, C₂₋₃alkenylamino, or C₂₋₃dialkenylamino. In various embodiments wherein (i) R¹ is an optionally substituted phenyl group, R² is H, OH or O-C₁₋₆alkyl; and each R⁴ is independently selected from OH and O-C₁₋₆alkyl. In various embodiments, R² is H, OH or O-C₁₋₃alkyl; and each R⁴ is independently selected from OH and O-C₁₋₃alkyl. In various embodiments wherein (i) R¹ is an optionally substituted phenyl group, R² is H, OH or OMe; and each R⁴ is independently selected from OH and OMe. In any of the foregoing embodiments, m may be 2 or 3. In such embodiments, when m is 2 the R⁴ groups may be at the C-5 and C-7 positions of the fused ring. When m is 3, in various embodiments the R⁴ groups may be at the C-5, C-6, C-7 positions of the fused ring.

In various embodiments of the compound of Formula (I), the optional substituents of the phenyl groups are independently selected from OH, O-alkyl, O-alkenyl, SH, alkylthio, alkenylthio, NH₂, alkylamino, dialkylamino, alkenylamino, and dialkenylamino. In various embodiments, the optional substituents of the phenyl groups are independently selected from OH, O-alkyl, O-alkenyl, SH, alkylthio, alkenylthio, NH₂, alkylamino, dialkylamino, alkenylamino and dialkenylamino. In various embodiments, the optional substituents of the phenyl groups are independently selected from OH, O-alkyl, O-alkenyl, alkylthio, alkenylthio, NH₂, alkylamino, dialkylamino, alkenylamino and dialkenylamino. In various embodiments, the optional substituents of the phenyl groups are independently selected from OH, O-alkyl, O-alkenyl, alkylthio, alkenylthio, alkylamino, dialkylamino, alkenylamino and dialkenylamino. In various embodiments, the optional substituents of the phenyl groups are independently selected from OH, O-alkyl, O-alkenyl, alkylamino, and dialkylamino. In various embodiments, the optional substituents of the phenyl groups are independently selected from OH, O-alkyl, and O-alkenyl. In various embodiments, the optional substituents of the phenyl groups are independently selected from OH and O-alkyl.

In any of such embodiments, the alkyl groups may be preferably C₁₋₆ alkyl groups and the alkenyl groups may be preferably C₂₋₆ alkenyl groups. More preferably, the alkyl groups may be C₁₋₃ alkyl groups and the alkenyl groups may be preferably C₂₋₃ alkenyl groups.

In various embodiments, the optional substituents of the phenyl groups are independently selected from OH, OMe, methylthio, methylamino, and dimethylamino. In various embodiments, the optional substituents of the phenyl groups are independently selected from OH, OMe, methylamino, and dimethylamino. In some embodiments, the optional substituents of the phenyl groups are independently selected from OH and OMe.

In various embodiments, the phenyl groups are substituted by 1 to 5, 1 to 4, 1 to 3, or one or two of the groups disclosed herein. For example, in various embodiments the phenyl groups are substituted by 1 to 5, 1 to 4, 1 to 3, or one or two groups independently selected from OH, O-alkyl, O-alkenyl, SH, alkylthio, alkenylthio, NH₂, alkylamino, dialkylamino, alkenylamino, and dialkenylamino. In various embodiments, the phenyl groups are substituted by 1 to 5, 1 to 4, 1 to 3, or one or two groups independently selected from OH, O-alkyl, O-alkenyl, SH, alkylthio, alkenylthio, NH₂, alkylamino, dialkylamino, alkenylamino and dialkenylamino. In various embodiments, the phenyl groups are substituted by 1 to 5, 1 to 4, 1 to 3, or one or two groups independently selected from OH, O-alkyl, O-alkenyl, alkylthio, alkenylthio, NH₂, alkylamino, dialkylamino, alkenylamino and dialkenylamino. In various embodiments, the phenyl groups are substituted by 1 to 5, 1 to 4, 1 to 3, or one or two groups independently selected from OH, O-alkyl, O-alkenyl, alkylthio, alkenylthio, alkylamino, dialkylamino, alkenylamino and dialkenylamino. In various embodiments, the phenyl groups are substituted by 1 to 5, 1 to 4, 1 to 3, or one or two groups independently selected from OH, O-alkyl, O-alkenyl, alkylamino, and dialkylamino. In various embodiments, the phenyl groups are substituted by 1 to 5, 1 to 4, 1 to 3, or one or two groups independently selected from OH, O-alkyl, and O-alkenyl. In various embodiments, the phenyl groups are substituted by 1 to 5, 1 to 4, 1 to 3, or one or two groups independently selected from OH and O-alkyl. In some embodiments, the phenyl groups are substituted by one or two groups independently selected from OH and O-alkyl. In some embodiments, the phenyl groups are substituted by one group independently selected from OH and O-alkyl.

In any of such embodiments, the alkyl groups may be preferably C₁₋₆ alkyl groups and the alkenyl groups may be preferably C₂₋₆ alkenyl groups. More preferably, the alkyl groups may be C₁₋₃ alkyl groups and the alkenyl groups may be preferably C₂₋₃ alkenyl groups.

In some embodiments, the phenyl groups are substituted by 1 to 5, 1 to 4, or 1 to 3 or one or two groups independently selected from OH, OMe, methylthio, methylamino, and dimethylamino. In various embodiments, the phenyl groups are substituted by 1 to 5, 1 to 4, or 1 to 3 or one or two groups independently selected from OH, OMe, methylamino, and dimethylamino. In some embodiments, the phenyl groups are substituted by 1 to 5, 1 to 4, or 1 to 3 or one or two groups independently selected from OH and OMe.

In preferred embodiments the phenyl groups are substituted by one or two groups as defined in the above embodiments.

In any of the foregoing embodiments wherein the phenyl groups are substituted by two groups as defined herein above, said groups may be at the C-3' and C-4' positions of the phenyl ring. For example, in various embodiments the phenyl groups are substituted at the C-3' and C-4' positions of the phenyl ring by two groups independently selected from OH, O-C_{1- 6}alkyl, O-C₂₋₆alkenyl, SH, C₁₋₆alkylthio, C₂₋₆alkenylthio, NH₂, C₁₋₆alkylamino, C₁₋₆dialkylamino, C₂₋₆alkenylamino and C₂₋₆dialkenylamino. In various embodiments, the phenyl groups are substituted at the C-3' and C-4' positions of the phenyl ring by two groups independently selected from OH, O-C₁₋₆alkyl, O-C₂₋₆alkenyl, C₁₋₆alkylthio, C₂₋₆alkenylthio, NH₂, C₁₋₆alkylamino, C₁₋₆dialkylamino, C₂₋₆alkenylamino and C₂₋₆dialkenylamino. In various embodiments, the phenyl groups are substituted at the C-3' and C-4' positions of the phenyl ring by two groups independently selected from OH, O-C₁₋₆alkyl, O-C₂₋₆alkenyl, C₁₋₆alkylthio, C₂₋₆alkenylthio, C₁₋₆alkylamino, C₁₋₆dialkylamino, C₂₋₆alkenylamino and C₂₋₆dialkenylamino. In various embodiments, the phenyl groups are substituted at the C-3' and C-4' positions of the phenyl ring by two groups independently selected from OH, O-C₁₋₆alkyl, O-C₂₋₆alkenyl, C₁₋₆alkylamino, and C₁₋₆dialkylamino. In various embodiments, the phenyl groups are substituted at the C-3' and C-4' positions of the phenyl ring by two groups independently selected from OH, O-C₁₋₆alkyl, and O-Cz-salkenyl. In various embodiments, the phenyl groups are substituted at the C-3' and C-4' positions of the phenyl ring by two groups independently selected from OH and O-C₁₋₆alkyl. In further embodiments, the phenyl groups are substituted at the C-3' and C-4' positions of the phenyl ring by two groups independently selected from OH, O-C₁₋₃alkyl, O-C₂₋₃alkenyl, SH, C₁₋₃alkylthio, C₂₋₃alkenylthio, NH₂, C₁₋₃alkylamino, C₁₋₃dialkylamino, C₂₋₃alkenylamino and C₂₋₃dialkenylamino. In various embodiments, the phenyl groups are substituted at the C-3' and C-4' positions of the phenyl ring by two groups independently selected from OH, O-C₁₋₃alkyl, O-C₂₋₃alkenyl, C₁₋₃alkylthio, C₂₋₃alkenylthio, NH₂, C₁₋₃alkylamino, C₁₋₃dialkylamino, C₂₋₃alkenylamino and C₂₋₃dialkenylamino. In various embodiments, the phenyl groups are substituted at the C-3' and C-4' positions of the phenyl ring by two groups independently selected from OH, O-C₁₋₃alkyl, O-C₂₋₃alkenyl, C₁₋₃alkylthio, C₂₋₃alkenylthio, C₁₋₃alkylamino, C₁₋₃dialkylamino, C₂₋₃alkenylamino and C₂₋₃dialkenylamino. In various embodiments, the phenyl groups are substituted at the C-3' and C-4' positions of the phenyl ring by two groups independently selected from OH, O-C₁₋₃alkyl, O-C₂₋₃alkenyl, C₁₋₃alkylamino, and C₁₋₃dialkylamino. In various embodiments, the phenyl groups are substituted at the C-3' and C-4' positions of the phenyl ring by two groups independently selected from OH, O-C₁₋₃alkyl, and O-C₂₋₃alkenyl. In various embodiments, the phenyl groups are substituted at the C-3' and C-4' positions of the phenyl ring by two groups independently selected from OH and O-C₁₋₃alkyl. In some embodiments, the phenyl groups are substituted at the C-3' and C-4' positions of the phenyl ring by two groups independently selected from OH and OMe.

In any of the foregoing embodiments wherein the phenyl groups are substituted by one group as defined herein above, said group may in further embodiments be at the C-4' position of the phenyl ring. For example, in various embodiments the phenyl groups are substituted at the C-4' position of the phenyl ring by one group selected from OH, O-C₁₋₆alkyl, O-C₂₋₆alkenyl, SH, C₁₋₆alkylthio, C₂₋₆alkenylthio, NH₂, C₁₋₆alkylamino, C₁₋₆dialkylamino, C₂₋₆alkenylamino and C₂₋₆dialkenylamino. In various embodiments, the phenyl groups are substituted at the C-4' position of the phenyl ring by one group selected from OH, O-C₁₋₆alkyl, O-C₂₋₆alkenyl, C₁₋₆alkylthio, C₂₋₆alkenylthio, NH₂, C₁₋₆alkylamino, C₁₋₆dialkylamino, C₂₋₆alkenylamino and C₂₋₆dialkenylamino. In various embodiments, the phenyl groups are substituted at the C-4' position of the phenyl ring by one group selected from OH, O-C₁₋₆alkyl, O-C₂₋₆alkenyl, C₁₋₆alkylthio, C₂₋₆alkenylthio, C₁₋₆alkylamino, C₁₋₆dialkylamino, C₂₋₆alkenylamino and C₂₋₆dialkenylamino. In various embodiments, the phenyl groups are substituted at the C-4' position of the phenyl ring by one group selected from OH, O-C₁₋₆alkyl, O-C₂₋₆alkenyl, C₁₋₆alkylamino, and C₁₋₆dialkylamino. In various embodiments, the phenyl groups are substituted at the C-4' position of the phenyl ring by one group selected from OH, O-C₁₋₆alkyl, and O-C₂₋₆alkenyl. In various embodiments, the phenyl groups are substituted at the C-4' position of the phenyl ring by one group selected from OH and O-C₁₋₆alkyl. In further embodiments, the phenyl groups are substituted at the C-4' position of the phenyl ring by one group selected from OH, O-C₁₋₃alkyl, O-C₂₋₃alkenyl, SH, C₁₋₃alkylthio, C₂₋₃alkenylthio, NH₂, C₁₋₃alkylamino, C₁₋₃dialkylamino, C₂₋₃alkenylamino and C₂₋₃dialkenylamino. In various embodiments, the phenyl groups are substituted at the C-4' position of the phenyl ring by one group selected from OH, O-C₁₋₃alkyl, O-C₂₋₃alkenyl, C₁₋₃alkylthio, C₂₋₃alkenylthio, NH₂, C₁₋₃alkylamino, C₁₋₃dialkylamino, C₂₋₃alkenylamino and C₂₋₃dialkenylamino. In various embodiments, the phenyl groups are substituted at the C-4' position of the phenyl ring by one group selected from OH, O-C₁₋₃alkyl, O-C₂₋₃alkenyl, C₁₋₃alkylthio, C₂₋₃alkenylthio, C₁₋₃alkylamino, C₁₋₃dialkylamino, C₂₋₃alkenylamino and C₂₋₃dialkenylamino. In various embodiments, the phenyl groups are substituted at the C-4' position of the phenyl ring by one group selected from OH, O-C₁₋₃alkyl, O-C₂₋₃alkenyl, C₁₋₃alkylamino, and C₁₋₃dialkylamino. In various embodiments, the phenyl groups are substituted at the C-4' position of the phenyl ring by one group selected from OH, O-C₁₋₃alkyl, and O-C₂₋₃alkenyl. In various embodiments, the phenyl groups are substituted at the C-4' position of the phenyl ring by one group selected from OH and O-C₁₋₃alkyl. In some embodiments, the phenyl groups are substituted at the C-4' position of the phenyl ring by one group selected from OH and OMe.

Any of the embodiments of the optionally substituted phenyl group disclosed herein above may be combined with the embodiments of m, R¹, R² and/or R⁴ disclosed herein.

In various embodiments of the compound of Formula (I) wherein (i) R² is an optionally substituted phenyl group, R¹ is H, OH, O-C₁₋₆alkyl, O-C₂₋₆alkenyl, SH, C₁₋₆alkylthio, C₂₋₆alkenylthio, NH₂, C₁₋₆alkylamino, C₁₋₆dialkylamino, C₂₋₆alkenylamino, or C₂₋₆dialkenylamino; each R⁴ is independently selected from OH, O-C₁₋₆alkyl, O-C₂₋₆alkenyl, SH, C₁₋₆alkylthio, C₂₋₆alkenylthio, NH₂, C₁₋₆alkylamino, C₁₋₆dialkylamino, C₂₋₆alkenylamino, or C₂₋₆dialkenylamino, and the optional substituents of the phenyl groups are independently selected from OH, O-C₁₋₆alkyl, O-C₂₋₆alkenyl, SH, C₁₋₆alkylthio, C₂₋₆alkenylthio, NH₂, C₁₋₆alkylamino, C₁₋₆dialkylamino, C₂₋₆alkenylamino, or C₂₋₆dialkenylamino. In various embodiments of the compound of Formula (I) wherein (i) R² is an optionally substituted phenyl group, R¹ is H, OH, O-C₁₋₃alkyl, O-C₂₋₃alkenyl, SH, C₁₋₃alkylthio, C₂₋₃alkenylthio, NH₂, C₁₋₃alkylamino, C₁₋₃dialkylamino, C₂₋₃alkenylamino, or C₂₋₃dialkenylamino; each R⁴ is independently selected from OH, O-C₁₋₃alkyl, O-C₂₋₃alkenyl, SH, C₁₋₃alkylthio, C₂₋₃alkenylthio, NH₂, C₁₋₃alkylamino, C₁₋₃dialkylamino, C₂₋₃alkenylamino, or C₂₋₃dialkenylamino, and the optional substituents of the phenyl groups are independently selected from OH, O-C₁₋₃alkyl, O-C₂₋₃alkenyl, SH, C₁₋₃alkylthio, C₂₋₃alkenylthio, NH₂, C₁₋₃alkylamino, C₁₋₃dialkylamino, C₂₋₃alkenylamino, or C₂₋₃dialkenylamino. In various embodiments of the compound of Formula (I) wherein (i) R² is an optionally substituted phenyl group, R¹ is H, OH or O-C₁₋₆alkyl; each R⁴ is independently selected from OH and O-C₁₋₆alkyl, and the optional substituents of the phenyl groups are independently selected from OH and O-C₁₋₆alkyl. In various embodiments of the compound of Formula (I) wherein (i) R² is an optionally substituted phenyl group, R¹ is H, OH or O-C₁₋₃alkyl; each R⁴ is independently selected from OH and O-C₁₋₃alkyl, and the optional substituents of the phenyl groups are independently selected from OH and O-C₁₋₃alkyl. In various embodiments wherein (i) R² is an optionally substituted phenyl group, R¹ is H, OH or OMe; each R⁴ is independently selected from OH and OMe, and the optional substituents of the phenyl groups are independently selected from OH and OMe. In any of the foregoing embodiments, m may be 2 or 3. In such embodiments, when m is 2 the R⁴ groups may be at the C-5 and C-7 positions of the fused ring. When m is 3, in various embodiments the R⁴ groups are at the C-5, C-6, C-7 positions of the fused ring. In any of the foregoing embodiments, the phenyl groups may be substituted by 1 to 5, 1 to 4, 1 to 3, or one or two groups according to the embodiments disclosed herein. Where the phenyl groups are substituted by one group, in various embodiments the phenyl groups are substituted at the C-4' position of the phenyl ring. Where the phenyl groups are substituted by two groups, in various embodiments the phenyl groups are substituted at the C-3' and C-4' positions of the phenyl ring.

In various embodiments of the compound of Formula (I) wherein (i) R¹ is an optionally substituted phenyl group, R² is H, OH, O-C₁₋₆alkyl, O-C₂₋₆alkenyl, SH, C₁₋₆alkylthio, C₂₋₆alkenylthio, NH₂, C₁₋₆alkylamino, C₁₋₆dialkylamino, C₂₋₆alkenylamino, or C₂₋₆dialkenylamino; each R⁴ is independently selected from OH, O-C₁₋₆alkyl, O-C₂₋₆alkenyl, SH, C₁₋₆alkylthio, C₂₋₆alkenylthio, NH₂, C₁₋₆alkylamino, C₁₋₆dialkylamino, C₂₋₆alkenylamino, or C₂₋₆dialkenylamino, and the optional substituents of the phenyl groups are independently selected from OH, O-C₁₋₆alkyl, O-C₂₋₆alkenyl, SH, C₁₋₆alkylthio, C₂₋₆alkenylthio, NH₂, C₁₋₆alkylamino, C₁₋₆dialkylamino, C₂₋₆alkenylamino, or C₂₋₆dialkenylamino. In various embodiments of the compound of Formula (I) wherein (i) R¹ is an optionally substituted phenyl group, R² is H, OH, O-C₁₋₃alkyl, O-C₂₋₃alkenyl, SH, C₁₋₃alkylthio, C₂₋₃alkenylthio, NH₂, C₁₋₃alkylamino, C₁₋₃dialkylamino, C₂₋₃alkenylamino, or C₂₋₃dialkenylamino; each R⁴ is independently selected from OH, O-C₁₋₃alkyl, O-C₂₋₃alkenyl, SH, C₁₋₃alkylthio, C₂₋₃alkenylthio, NH₂, C₁₋₃alkylamino, C₁₋₃dialkylamino, C₂₋₃alkenylamino, or C₂₋₃dialkenylamino, and the optional substituents of the phenyl groups are independently selected from OH, O-C₁₋₃alkyl, O-C₂₋₃alkenyl, SH, C₁₋₃alkylthio, C₂₋₃alkenylthio, NH₂, C₁₋₃alkylamino, C₁₋₃dialkylamino, C₂₋₃alkenylamino, or C₂₋₃dialkenylamino. In various embodiments of the compound of Formula (I) wherein (i) R¹ is an optionally substituted phenyl group; R² is H, OH or O-C₁₋₆alkyl; each R⁴ is independently selected from OH and O-C₁₋₆alkyl, and the optional substituents of the phenyl groups are independently selected from OH and O-C₁₋₆alkyl. In various embodiments, R² is H, OH or O-C₁₋₃alkyl; each R⁴ is independently selected from OH and O-C₁₋₃alkyl, and the optional substituents of the phenyl groups are independently selected from OH and O-C₁₋₃alkyl. In various embodiments wherein (i) R¹ is an optionally substituted phenyl group, R² is H, OH or OMe; each R⁴ is independently selected from OH and OMe, and the optional substituents of the phenyl groups are independently selected from OH and OMe. In any of the foregoing embodiments, m may be 2 or 3. In such embodiments, when m is 2 the R⁴ groups may be at the C-5 and C-7 positions of the fused ring. When m is 3, in various embodiments the R⁴ groups are at the C-5, C-6, C-7 positions of the fused ring. Where the phenyl groups are substituted by one group, in various embodiments the phenyl groups are substituted at the C-4' position of the phenyl ring. Where the phenyl groups are substituted by two groups, in various embodiments the phenyl groups are substituted at the C-3' and C-4' positions of the phenyl ring.

In various embodiments of the compound of Formula (I), a double bond is not present between positions C-2 and C-3 of the fused ring. Such embodiments may be combined with any of the embodiments of m, R¹, R², R³ and the optional substituents of the phenyl groups disclosed herein.

In various embodiments a double bond is not present between positions C-2 and C-3 of the fused ring, R¹ is H, OH, O-C₁₋₃alkyl, O-C₂₋₃alkenyl, SH, C₁₋₃alkylthio, C₂₋₃alkenylthio, NH₂, C₁₋₃alkylamino, C₁₋₃dialkylamino, C₂₋₃alkenylamino, or C₂₋₃dialkenylamino; R² is an optionally substituted phenyl group wherein the optional substituents of the phenyl group are independently selected from OH, O-C₁₋₃alkyl, O-C₂₋₃alkenyl, SH, C₁₋₃alkylthio, C₂₋₃alkenylthio, NH₂, C₁₋₃alkylamino, C₁₋₃dialkylamino, C₂₋₃alkenylamino, and C₂₋₃dialkenylamino; and each R⁴ is independently selected from OH, O-C₁₋₃alkyl, O-C₂₋₃alkenyl, SH, C₁₋₃alkylthio, C₂₋₃alkenylthio, NH₂, C₁₋₃alkylamino, C₁₋₃dialkylamino, C₂₋₃alkenylamino, and C₂₋₃dialkenylamino. In various embodiments a double bond is not present between positions C-2 and C-3 of the fused ring, R¹ is H, OH or O-C₁₋₃alkyl; R² is an optionally substituted phenyl group wherein the optional substituents of the phenyl group are independently selected from OH and O-C₁₋₃alkyl; and each R⁴ is independently selected from OH and O-C₁₋₃alkyl. In any of the foregoing embodiments, the C₁₋₃alkyl may be methyl. In any of the foregoing embodiments, m may be 2 or 3. In such embodiments, when m is 2 the R⁴ groups may be at the C-5 and C-7 positions of the fused ring. When m is 3, in various embodiments the R⁴ groups are at the C-5, C-6, C-7 positions of the fused ring. Where the phenyl groups are substituted by one group, in various embodiments the phenyl groups are substituted at the C-4' position of the phenyl ring. Where the phenyl groups are substituted by two groups, in various embodiments the phenyl groups are substituted at the C-3' and C-4' positions of the phenyl ring.

In various embodiments a double bond is not present between positions C-2 and C-3 of the fused ring, R¹ is an optionally substituted phenyl group, wherein the optional substituents of the phenyl group are independently selected from OH, O-C₁₋₃alkyl, O-C₂₋₃alkenyl, SH, C₁₋₃alkylthio, C₂₋₃alkenylthio, NH₂, C₁₋₃alkylamino, C₁₋₃dialkylamino, C₂₋₃alkenylamino, and C₂₋₃dialkenylamino; R² is H, OH, O-C₁₋₃alkyl, SH, C₁₋₃alkylthio, C₂₋₃alkenylthio, NH₂, C₁₋₃alkylamino, C₁₋₃dialkylamino, C₂₋₃alkenylamino, or C₂₋₃dialkenylamino, and each R⁴ is independently selected from OH, O-C₁₋₃alkyl, O-C₂₋₃alkenyl, SH, C₁₋₃alkylthio, C₂₋₃alkenylthio, NH₂, C₁₋₃alkylamino, C₁₋₃dialkylamino, C₂₋₃alkenylamino, and C₂₋₃dialkenylamino. In various embodiments a double bond is not present between positions C-2 and C-3 of the fused ring, R¹ is an optionally substituted phenyl group, wherein the optional substituents of the phenyl group are independently selected from OH, and O-C₁₋₃alkyl; R² is H, OH, or O-C₁₋₃alkyl, and each R⁴ is independently selected from OH and O-C₁₋₃alkyl. In any of the foregoing embodiments, the C₁₋₃alkyl may be methyl. In any of the foregoing embodiments, m may be 2 or 3. In such embodiments, when m is 2 the R⁴ groups may be at the C-5 and C-7 positions of the fused ring. When m is 3, in various embodiments the R⁴ groups are at the C-5, C-6, C-7 positions of the fused ring. Where the phenyl groups are substituted by one group, in various embodiments the phenyl groups are substituted at the C-4' position of the phenyl ring. Where the phenyl groups are substituted by two groups, in various embodiments the phenyl groups are substituted at the C-3' and C-4' positions of the phenyl ring.

In other embodiments, a double bond is present between positions C-2 and C-3 of the fused ring. Such embodiments may be combined with any of the embodiments of m, R¹, R², R³ and the optional substituents of the phenyl groups disclosed herein.

In various embodiments a double bond is present between positions C-2 and C-3 of the fused ring, R¹ is H, OH, O-C₁₋₃alkyl, O-C₂₋₃alkenyl, SH, C₁₋₃alkylthio, C₂₋₃alkenylthio, NH₂, C₁₋₃alkylamino, C₁₋₃dialkylamino, C₂₋₃alkenylamino, or C₂₋₃dialkenylamino; R² is an optionally substituted phenyl group wherein the optional substituents of the phenyl group are independently selected from OH, O-C₁₋₃alkyl, O-C₂₋₃alkenyl, SH, C₁₋₃alkylthio, C₂₋₃alkenylthio, NH₂, C₁₋₃alkylamino, C₁₋₃dialkylamino, C₂₋₃alkenylamino, and C₂₋₃dialkenylamino; and each R⁴ is independently selected from OH, O-C₁₋₃alkyl, O-C₂₋₃alkenyl, SH, C₁₋₃alkylthio, C₂₋₃alkenylthio, NH₂, C₁₋₃alkylamino, C₁₋₃dialkylamino, C₂₋₃alkenylamino, and C₂₋₃dialkenylamino. In various embodiments a double bond is present between positions C-2 and C-3 of the fused ring, R¹ is H, OH, or O-C₁₋₃alkyl; R² is an optionally substituted phenyl group wherein the optional substituents of the phenyl group are independently selected from OH and O-C₁₋₃alkyl; and each R⁴ is independently selected from OH and O-C₁₋₃alkyl. In any of the foregoing embodiments, the C₁₋₃alkyl may be methyl. In any of the foregoing embodiments, m may be 2 or 3. In such embodiments, when m is 2 the R⁴ groups may be at the C-5 and C-7 positions of the fused ring. When m is 3, in various embodiments the R⁴ groups are at the C-5, C-6, C-7 positions of the fused ring. Where the phenyl groups are substituted by one group, in various embodiments the phenyl groups are substituted at the C-4' position of the phenyl ring. Where the phenyl groups are substituted by two groups, in various embodiments the phenyl groups are substituted at the C-3' and C-4' positions of the phenyl ring.

In various embodiments a double bond is present between positions C-2 and C-3 of the fused ring, R¹ is an optionally substituted phenyl group, wherein the optional substituents of the phenyl group are independently selected from OH, O-C₁₋₃alkyl, O-C₂₋₃alkenyl, SH, C₁₋₃alkylthio, C₂₋₃alkenylthio, NH₂, C₁₋₃alkylamino, C₁₋₃dialkylamino, C₂₋₃alkenylamino, and C₂₋₃dialkenylamino; R² is H, OH, O-C₁₋₃alkyl, SH, C₁₋₃alkylthio, C₂₋₃alkenylthio, NH₂, C₁₋₃alkylamino, C₁₋₃dialkylamino, C₂₋₃alkenylamino, or C₂₋₃dialkenylamino, and each R⁴ is independently selected from OH, O-C₁₋₃alkyl, O-C₂₋₃alkenyl, SH, C₁₋₃alkylthio, C₂₋₃alkenylthio, NH₂, C₁₋₃alkylamino, C₁₋₃dialkylamino, C₂₋₃alkenylamino, and C₂₋₃dialkenylamino. In various embodiments a double bond is present between positions C-2 and C-3 of the fused ring, R¹ is an optionally substituted phenyl group, wherein the optional substituents of the phenyl group are independently selected from OH and O-C₁₋₃alkyl; R² is H, OH or O-C₁₋₃alkyl, and each R⁴ is independently selected from OH and O-C₁₋₃alkyl. In any of the foregoing embodiments, the C₁₋₃alkyl may be methyl. In any of the foregoing embodiments, m may be 2 or 3. In such embodiments, when m is 2 the R⁴ groups may be at the C-5 and C-7 positions of the fused ring. When m is 3, in various embodiments the R⁴ groups are at the C-5, C-6, C-7 positions of the fused ring. Where the phenyl groups are substituted by one group, in various embodiments the phenyl groups are substituted at the C-4' position of the phenyl ring. Where the phenyl groups are substituted by two groups, in various embodiments the phenyl groups are substituted at the C-3' and C-4' positions of the phenyl ring.

In various embodiments, R¹ is H, OH, O-alkyl, O-alkenyl, SH, alkylthio, alkenylthio, NH₂, alkylamino, dialkylamino, alkenylamino, or dialkenylamino; and R² is an optionally substituted phenyl group. In such embodiments, the compound of Formula (I) may be a compound of Formula (Ia): 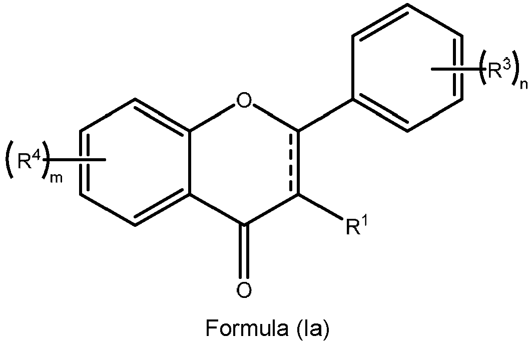 wherein each of R¹, R³, R⁴ and m may be as defined according to any of the embodiments disclosed herein and n is an integer from 0 to 5.

In various embodiments, m is an integer from 0 to 4. In any of the foregoing embodiments, m may be 0, 1, 2, 3, or 4. In various embodiments, m is an integer from 1 to 4, such as 1, 2, 3, or 4. In various embodiments, m is an integer from 0 to 3, such as 0, 1, 2, or 3. In various embodiments, m is an integer from 1 to 3, such as 1, 2, or 3. In other embodiments, m is an integer from 2 to 4, such as 2, 3, or 4. In some embodiments, m is 2 or 3. When m is 2, in various embodiments the R⁴ groups may be at the C-5 and C-7 positions of the fused ring. When m is 3, in various embodiments the R⁴ groups may be at the C-5, C-6, C-7 positions of the fused ring.

In various embodiments of the compound of Formula (Ia), n is an integer from 0 to 5. Thus, n may be 0, 1, 2, 3, 4, or 5. In various embodiments, n is an integer from 1 to 5, such as 1, 2, 3, 4, or 5. In various embodiments, n is an integer from 0 to 4, such as 0, 1, 2, 3, or 4. In various embodiments, n is an integer from 1 to 4, such as 1, 2, 3, or 4. In various embodiments, n is an integer from 0 to 3, such as 0, 1, 2 or 3. In various embodiments, n is an integer from 1 to 3, such as 1, 2, or 3., In various embodiments, n is an integer from 0 to 2, such as 0, 1, or 2. In various embodiments, n is an 1 or 2. In some embodiments, n is 2. In such embodiments, the R³ groups may be at the C-3' and C-4' positions of the phenyl group.

In various embodiments of the compound of Formula (Ia), m is 2 and n is 1. In various embodiments, m is 3 and n is 1. In various embodiments, m is 2 and n is 2. In various embodiments, m is 3 and n is 2.

In various embodiments of the compound of Formula (Ia), m is 2, the R⁴ groups are at the C-5 and C-7 positions of the fused ring, n is 1, and the R³ group is at the C-4' position of the phenyl group. In various embodiments of the compound of Formula (Ia), m is 2, the R⁴ groups are at the C-5 and C-7 positions of the fused ring, n is 2, and the R³ groups are at the C-3' and C-4' positions of the phenyl group.

In various embodiments of the compound of Formula (Ia), m is 3, the R⁴ groups are at the C-5, C-6, C-7 positions of the fused ring, n is 1, and the R³ group is at the C-4' position of the phenyl group. In various embodiments of the compound of Formula (Ib), m is 3, the R⁴ groups are at the C-5, C-6, C-7 positions of the fused ring, n is 2, and the R³ groups are at the C-3' and C-4' positions of the phenyl group.

In some embodiments, n is 1. In such embodiments, the R³ group may in further embodiments be at the C-4' position of the phenyl group. Thus, in some embodiments, the compound of Formula (Ia) may be a compound of Formula (laa):

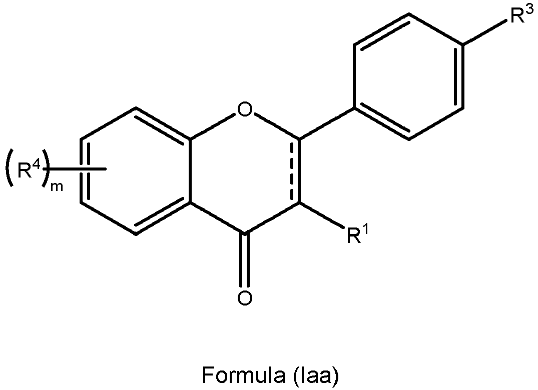

In various embodiments of the compound of Formula (laa), each of R¹, R³, R⁴ and m may be as defined in any of the embodiments disclosed herein.

In various embodiments of the compound of Formula (Ia), a double bond is present between positions C-2 and C-3 of the fused ring. Thus, in various embodiments, the compound of Formula (Ia) is a compound of Formula (lab): 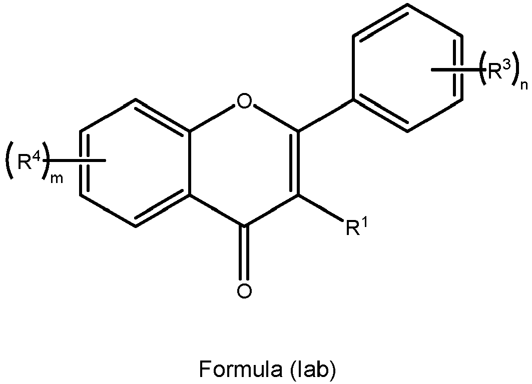 wherein each of R¹, R³, R⁴, m, and n may be as defined in any of the embodiments disclosed herein.

In various embodiments of the compound of Formula (Ia), a double bond is present between positions C-2 and C-3 of the fused ring, n is 1, and the R³ group is at the C-4' position of the phenyl group. Thus, in various embodiments, the compound of Formula (Ia) is a compound of Formula (lac): 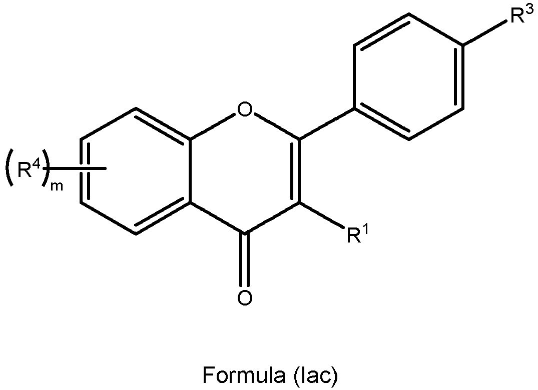 wherein each of R¹, R³, R⁴, and m may be as defined according to any of the embodiments disclosed herein.

In various embodiments of the compound of Formula (Ia), m is 2 and the R⁴ groups are at the C-5 and C-7 positions of the fused ring. Thus, in various embodiments, the compound of Formula (Ia) is a compound of Formula (lad): 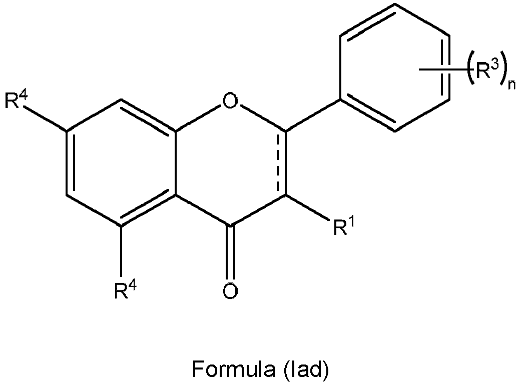 wherein each of R¹, R³, R⁴, and n may be as defined in any of the embodiments disclosed herein.

In various embodiments of the compound of Formula (Ia), a double bond is present between positions C-2 and C-3 of the fused ring, m is 2, and the R⁴ groups are at the C-5 and C-7 positions of the fused ring. Thus, in various embodiments, the compound of Formula (Ia) is a compound of Formula (lae): 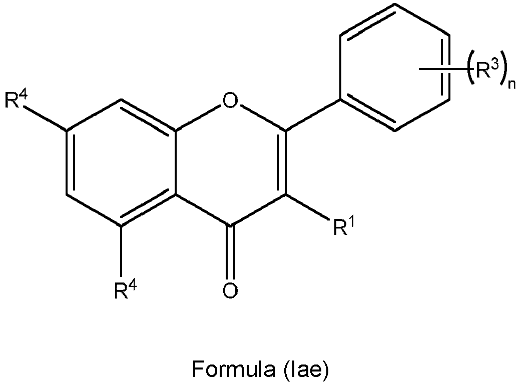 wherein each of R¹, R³, R⁴, and n may be as defined according to any of the embodiments disclosed herein.

In various embodiments of the compound of Formula (Ia), m is 2, the R⁴ groups are at the C-5 and C-7 positions of the fused ring, n is 1 and the R³ group is at the C-4' position of the phenyl group. Thus, in various embodiments, the compound of Formula (Ia) is a compound of Formula (laf): 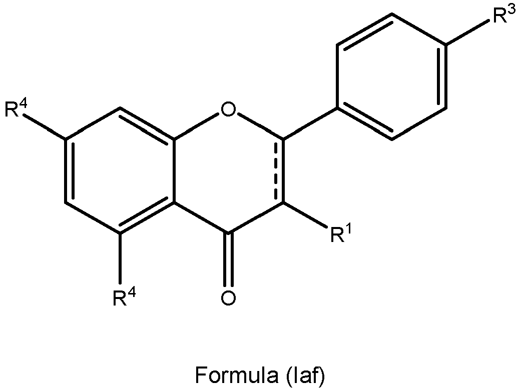 wherein each of R¹, R³, and R⁴ may be as defined according to any of the embodiments disclosed herein.

In various embodiments of the compound of Formula (Ia), a double bond is present between positions C-2 and C-3 of the fused ring, m is 2, the R⁴ groups are at the C-5 and C-7 positions of the fused ring, n is 1 and the R³ group is at the C-4' position of the phenyl group. Thus, in various embodiments, the compound of Formula (Ia) is a compound of Formula (lag): 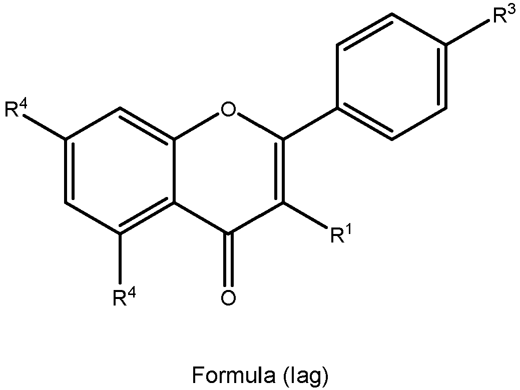 wherein each of R¹, R³, and R⁴ may be as defined according to any of the embodiments disclosed herein.

In various embodiments of the compound of Formula (Ia), m is 3, the R⁴ groups are at the C-5, C-6, C-7 positions of the fused ring, n is 1, and the R³ group is at the C-4' position of the phenyl group. In further embodiments, m is 3, the R⁴ groups are at the C-5, C-6, C-7 positions of the fused ring, n is 1, the R³ group is at the C-4' position of the phenyl group, and a double bond is present between positions C-2 and C-3 of the fused ring. Thus, in various embodiments the compound of Formula (Ia) is a compound of Formula (lah): 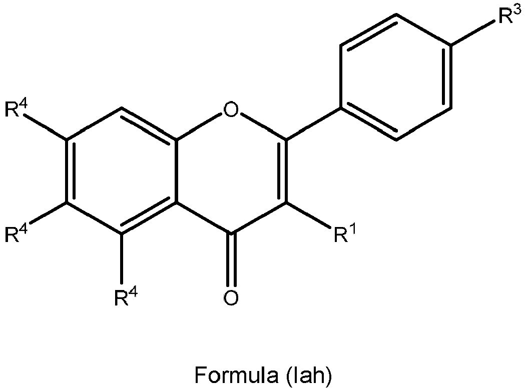 wherein each of R¹, R³, and R⁴ may be as defined according to any of the embodiments disclosed herein.

In any of the foregoing embodiments where the double bond between positions C-2 and C-3 of the fused ring is absent, the compound may be in the form of a racemic or scalemic mixture. In such scalemic mixtures, the S-enantiomer may be in excess. The enantiomeric excess is not necessarily limited. In various embodiments, the *S-enantiomer may* be present in an enantiomeric excess of at least 10%, at least 20%, at least 30%, at least 40%, at least 50%, at least 60%, at least 70%, at least 80%, or at least 90%.

In some embodiments of the compound of Formula (I), R¹ is an optionally substituted phenyl group; R² is H, OH, O-alkyl, O-alkenyl, SH, alkylthio, alkenylthio, NH₂, alkylamino, dialkylamino, alkenylamino, or dialkenylamino; and a double bond is present between positions C-2 and C-3 of the fused ring. In such embodiments, the compound of Formula (I) may be a compound of Formula (Ib): 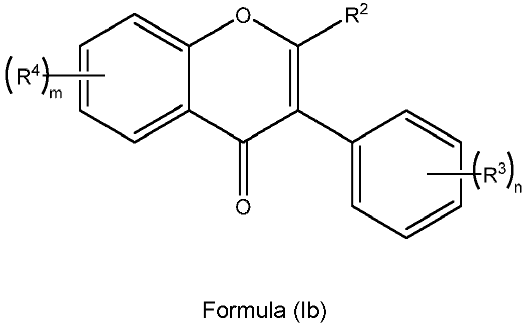 wherein each of R², R³, R⁴ and m may be as defined according to any of the embodiments disclosed herein and n is an integer from 0 to 5.

In various embodiments of the compound of Formula (Ib), m is an integer from 0 to 4. Thus, in various embodiments, m may be 0, 1, 2, 3, or 4. In various embodiments, m is an integer from 1 to 4, such as 1, 2, 3, or 4. In various embodiments, m is an integer from 0 to 3, such as 0, 1, 2, or 3. In various embodiments, m is an integer from 1 to 3, such as 1, 2, or 3. In other embodiments, m is an integer from 2 to 4, such as 2, 3, or 4. In some embodiments, m is 2 or 3. When m is 2, in various embodiments the R⁴ groups may be at the C-5 and C-7 positions of the fused ring. When m is 3, in various embodiments the R⁴ groups may be at the C-5, C-6, C-7 positions of the fused ring.

In various embodiments of the compound of Formula (Ib), n is an integer from 0 to 5. Thus, n may be 0, 1, 2, 3, 4, or 5. In various embodiments, n is an integer from 1 to 5, such as 1, 2, 3, 4, or 5. In various embodiments, n is an integer from 0 to 4, such as 0, 1, 2, 3, or 4. In various embodiments, n is an integer from 1 to 4, such as 1, 2, 3, or 4. In various embodiments, n is an integer from 0 to 3, such as 0, 1, 2 or 3. In various embodiments, n is an integer from 1 to 3, such as 1, 2, or 3., In various embodiments, n is an integer from 0 to 2, such as 0, 1, or 2. In various embodiments, n is an 1 or 2.

In various embodiments of the compound of Formula (Ib), m is 2 and n is 1. In various embodiments, m is 3 and n is 1. In various embodiments, m is 2 and n is 2. In various embodiments, m is 3 and n is 2.

In various embodiments of the compound of Formula (Ib), n is 2 and the R³ groups are at the C-3' and C-4' positions of the phenyl group.

In various embodiments of the compound of Formula (Ib), m is 2, the R⁴ groups are at the C-5 and C-7 positions of the fused ring, n is 1, and the R³ group is at the C-4' position of the phenyl group. In various embodiments of the compound of Formula (Ib), m is 2, the R⁴ groups are at the C-5 and C-7 positions of the fused ring, n is 2, and the R³ groups are at the C-3' and C-4' positions of the phenyl group.

In various embodiments of the compound of Formula (Ib), m is 3, the R⁴ groups are at the C-5, C-6, C-7 positions of the fused ring, n is 1, and the R³ group is at the C-4' position of the phenyl group. In various embodiments of the compound of Formula (Ib), m is 3, the R⁴ groups are at the C-5, C-6, C-7 positions of the fused ring, n is 2, and the R³ groups are at the C-3' and C-4' positions of the phenyl group.

In various embodiments of the compound of Formula (Ib), n is 1 and the R³ group is at the C-4' position of the phenyl group. Thus, in various embodiments, the compound of Formula (Ib) is a compound of Formula (Iba): 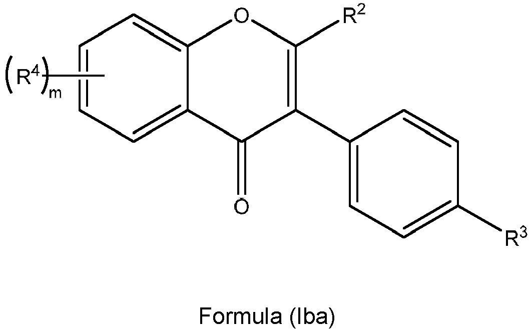 wherein each of R², R³, R⁴, and m may be as defined according to any of the embodiments disclosed herein.

In various embodiments of the compound of Formula (Ib), n is 1, the R³ group is at the C-4' position of the phenyl group, m is 2 and the R⁴ groups are at the C-5 and C-7 positions of the fused ring. Thus, in various embodiments, the compound of Formula (Ib) is a compound of Formula (Ibb): 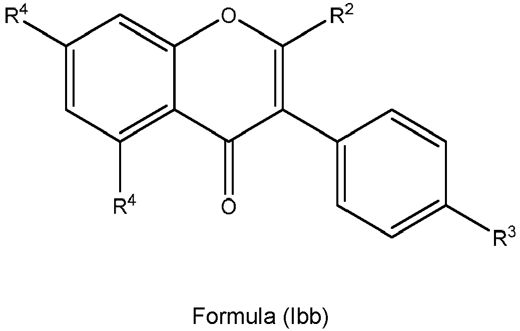 wherein each of R², R³, R⁴, and m may be as defined above.

In further embodiments of the Compound of Formula (Ia), (laa), (lab), (lac), (lad), (lae), (laf), (lag) or (lah), R¹ is OH, O-alkyl, O-alkenyl, SH, alkylthio, alkenylthio, NH₂, alkylamino, dialkylamino, alkenylamino or dialkenylamino. In various embodiments, R1 is H, OH, O-alkyl, O-alkenyl, alkylthio, alkenylthio, NH₂, alkylamino, dialkylamino, alkenylamino or dialkenylamino. In various embodiments, R1 is H, OH, O-alkyl, O-alkenyl, alkylthio, alkenylthio, alkylamino, dialkylamino, alkenylamino or dialkenylamino. In various embodiments, R1 is H, OH, O-alkyl, O-alkenyl, alkylamino, or dialkylamino. In various embodiments, R1 is H, OH, O-alkyl, or O-alkenyl. In various embodiments, R1 is H, OH or O-alkyl.

In any of such embodiments, the alkyl groups may be preferably C₁₋₆ alkyl groups and the alkenyl groups may be preferably C₂₋₆ alkenyl groups. More preferably, the alkyl groups may be C₁₋₃ alkyl groups and the alkenyl groups may be preferably C₂₋₃ alkenyl groups.

In various embodiments of the Compound of Formula (Ia), (laa), (lab), (lac), (lad), (lae), (laf), (lag) or (lah), R¹ is H, OH, OMe, methylthio, methylamino, or dimethylamino. In various embodiments, each R¹ is H, OH, OMe, methylamino, or dimethylamino. In some embodiments, R1 is H, OH or OMe. In any of the foregoing embodiments, R¹ is H, OH or OMe; preferably wherein R¹ is H or OH. In some embodiments, R¹ is H.

In further embodiments of the Compound of Formula (Ib), (Iba), or (Ibb), R² is H, OH, O-alkyl, O-alkenyl, SH, alkylthio, alkenylthio, NH₂, alkylamino, dialkylamino, alkenylamino or dialkenylamino. In various embodiments, R² is H, OH, O-alkyl, O-alkenyl, alkylthio, alkenylthio, NH₂, alkylamino, dialkylamino, alkenylamino or dialkenylamino. In various embodiments, R² is H, OH, O-alkyl, O-alkenyl, alkylthio, alkenylthio, alkylamino, dialkylamino, alkenylamino or dialkenylamino. In various embodiments, R² is H, OH, O-alkyl, O-alkenyl, alkylamino, or dialkylamino. In various embodiments, R² is H, OH, O-alkyl, or O-alkenyl. In various embodiments, R² is H, OH or O-alkyl.

In any of such embodiments, the alkyl groups may be preferably C₁₋₆ alkyl groups and the alkenyl groups may be preferably C₂₋₆ alkenyl groups. More preferably, the alkyl groups may be C₁₋₃ alkyl groups and the alkenyl groups may be preferably C₂₋₃ alkenyl groups.

In various embodiments of the Compound of Formula (Ib), (Iba), or (Ibb), R² is H, OH, OMe, methylthio, methylamino, or dimethylamino. In various embodiments, each R² is H, OH, OMe, methylamino, or dimethylamino. In some embodiments, R² is H, OH or OMe. In any of the foregoing embodiments, R² is H, OH or OMe; preferably wherein R² is H or OH. In some embodiments, R² is H.

In any of the foregoing embodiments of the Compound of Formula (Ia), (laa), (lab), (lac), (lad), (lae), (Iaf), (lag), (lah), (Iba), or (Ibb), each R⁴ may be independently selected from OH, O-alkyl, O-alkenyl, SH, alkylthio, alkenylthio, NH₂, alkylamino, dialkylamino, alkenylamino and dialkenylamino. In various embodiments, each R⁴ is independently selected from OH, O-alkyl, O-alkenyl, alkylthio, alkenylthio, NH₂, alkylamino, dialkylamino, alkenylamino and dialkenylamino. In various embodiments, each R⁴ is independently selected from OH, O-alkyl, O-alkenyl, alkylthio, alkenylthio, alkylamino, dialkylamino, alkenylamino and dialkenylamino. In various embodiments, each R⁴ is independently selected from OH, O-alkyl, O-alkenyl, alkylamino, and dialkylamino. In various embodiments, each R⁴ is independently selected from OH, O-alkyl, and O-alkenyl. In various embodiments, each R⁴ is independently selected from OH and O-alkyl. In any of such embodiments, the alkyl groups may be preferably C₁₋₆ alkyl groups and the alkenyl groups may be preferably C₂₋₆ alkenyl groups. More preferably, the alkyl groups may be C₁₋₃ alkyl groups and the alkenyl groups may be preferably C₂₋₃ alkenyl groups.

In any of the foregoing embodiments of the Compound of Formula (Ia), (laa), (lab), (lac), (lad), (lae), (Iaf), (lag), (lah), (Iba), or (Ibb), each R³ may be independently selected from OH, O-alkyl, O-alkenyl, SH, alkylthio, alkenylthio, NH₂, alkylamino, dialkylamino, alkenylamino, and dialkenylamino. In various embodiments, each R³ is independently selected from OH, O-alkyl, O-alkenyl, SH, alkylthio, alkenylthio, NH₂, alkylamino, dialkylamino, alkenylamino and dialkenylamino. In various embodiments, each R³ is independently selected from OH, O-alkyl, O-alkenyl, alkylthio, alkenylthio, NH₂, alkylamino, dialkylamino, alkenylamino and dialkenylamino. In various embodiments, each R³ is independently selected from OH, O-alkyl, O-alkenyl, alkylthio, alkenylthio, alkylamino, dialkylamino, alkenylamino and dialkenylamino. In various embodiments, each R³ is independently selected from OH, O-alkyl, O-alkenyl, alkylamino, and dialkylamino. In various embodiments, each R³ is independently selected from OH, O-alkyl, and O-alkenyl. In various embodiments, each R³ is independently selected from OH and O-alkyl. In any of such embodiments, the alkyl groups may be preferably C₁₋₆ alkyl groups and the alkenyl groups may be preferably C₂₋₆ alkenyl groups. More preferably, the alkyl groups may be C₁₋₃ alkyl groups and the alkenyl groups may be preferably C₂₋₃ alkenyl groups.

In various embodiments, each R³ is independently selected from OH, OMe, methylthio, methylamino, and dimethylamino. In various embodiments, each R³ is independently selected from OH, OMe, methylamino, and dimethylamino. In some embodiments, each R³ is independently selected from OH and OMe.

In any of the foregoing embodiments of the Compound of Formula (Ia), (laa), (lab), (lac), (lad), (lae), (Iaf), (lag), (lah), (Iba), or (Ibb), each R⁴ is independently selected from OH, O-C₁₋₆alkyl, O-C₂₋₆alkenyl, SH, C₁₋₆alkylthio, C₂₋₆alkenylthio, NH₂, C₁₋₆alkylamino, C₁₋₆dialkylamino, C₂₋₆alkenylamino, or C₂₋₆dialkenylamino, and each R³ is independently selected from OH, O-C₁₋₆alkyl, O-C₂₋₆alkenyl, SH, C₁₋₆alkylthio, C₂₋₆alkenylthio, NH₂, C₁₋₆alkylamino, C₁₋₆dialkylamino, C₂₋₆alkenylamino, or C₂₋₆dialkenylamino. In various embodiments of the compound of Formula (I) wherein (i) R² is an optionally substituted phenyl group, R¹ is H, OH, O-C₁₋₃alkyl, O-C₂₋₃alkenyl, SH, C₁₋₃alkylthio, C₂₋₃alkenylthio, NH₂, C₁₋₃alkylamino, C₁₋₃dialkylamino, C₂₋₃alkenylamino, or C₂₋₃dialkenylamino; each R⁴ is independently selected from OH, O-C₁₋₃alkyl, O-C₂₋₃alkenyl, SH, C₁₋₃alkylthio, C₂₋₃alkenylthio, NH₂, C₁₋₃alkylamino, C₁₋₃dialkylamino, C₂₋₃alkenylamino, or C₂₋₃dialkenylamino, and each R³ is independently selected from OH, O-C₁₋₃alkyl, O-C₂₋₃alkenyl, SH, C₁₋₃alkylthio, C₂₋₃alkenylthio, NH₂, C₁₋₃alkylamino, C₁₋₃dialkylamino, C₂₋₃alkenylamino, or C₂₋₃dialkenylamino. In various embodiments of the compound of Formula (I) wherein (i) R² is an optionally substituted phenyl group, R¹ is H, OH or O-C₁₋₆alkyl; each R⁴ is independently selected from OH and O-C₁₋₆alkyl, and each R³ is independently selected from OH and O-C₁₋₆alkyl. In various embodiments of the compound of Formula (I) wherein (i) R² is an optionally substituted phenyl group, R¹ is H, OH or O-C₁₋₃alkyl; each R⁴ is independently selected from OH and O-C₁₋₃alkyl, and each R³ is independently selected from OH and O-C₁₋₃alkyl. In various embodiments wherein (i) R² is an optionally substituted phenyl group, R¹ is H, OH or OMe; each R⁴ is independently selected from OH and OMe, and each R³ is independently selected from OH and OMe.

In any of the foregoing embodiments of the Compound of Formula (I), (Ia), (laa), (lab), (lac), (lad), (lae), (Iaf), (lag), (lah), (Ib), (Iba), or (Ibb), each R³ is independently H or OH. In any of the foregoing embodiments of the Compound of Formula (I), (Ia), (laa), (lab), (lac), (lad), (lae), (Iaf), (lag), (lah), (Ib), (Iba), or (Ibb), each R⁴ is independently H or OH. In any of the foregoing embodiments of the Compound of Formula (I), (Ia), (laa), (lab), (lac), (lad), (lae), (Iaf), (lag), (lah), (Ib), (Iba), or (Ibb), each R³ and each R⁴ is independently H or OH.

In various embodiments, the compound of Formula (I) is selected from flavone, genistein, hispidulin, kaempferol, naringenin, and quercetin. The structures of various compounds of Formula (I) are set out in the following Table 1.

**Table 1. Exemplary embodiments of compounds of Formula (I)**

| | | |
|---|---|---|
| Flavone | 2-phenylchromen-4-one | 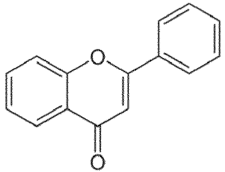 |
| Genistein | 5,7-Dihydroxy-3-(4-hydroxyphenyl)-4*H*-1-benzopyran-4-one | 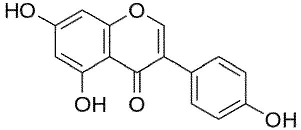 |
| Hispidulin | 5,7-Dihydroxy-2-(4-hydroxyphenyl)-6-methoxy-4*H*-1-benzopyran-4-one | 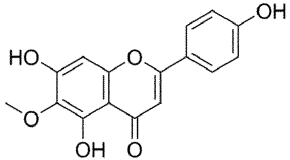 |
| Kaempferol | 3,5,7-Trihydroxy-2-(4-hydroxyphenyl)-4*H-*1-benzopyran-4-one | 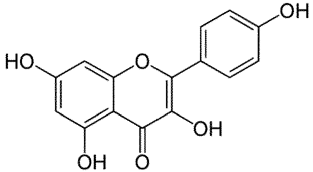 |
| Naringenin | (2S)-5,7-Dihydroxy-2-(4-hydroxyphenyl)-2,3-dihydro-4*H*-1-benzopyran-4-one | 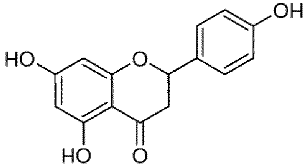 |
| Quercetin | 2-(3,4-Dihydroxyphenyl)-3,5,7-trihydroxy-4*H*-1-benzopyran-4-one | 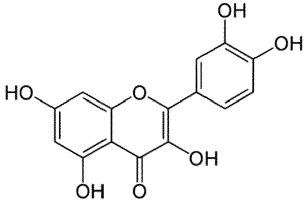 |

The compounds of formula (I), or derivatives thereof, may be naturally occurring or synthetically obtained. Compounds of Formula (I) as described herein are, for instance, commercially available from sources known to a person skilled in the art. The compounds may be provided in the form of a natural extract, for example a plant extract. The compounds may be obtained from natural sources, in particular plant sources. For example, the compounds may be obtained from plant extracts. In various embodiments, the compounds are isolated compounds, e.g. isolated from plant extracts. The purity of such isolated compound is not necessarily limited. In various embodiments, the purity of the isolated compound may be at least 20%, at least 30%, at least 40%, at least 50%, at least 60%, at least 70%, at least 80%, or at least 90%. In other embodiments, the compounds may be synthetically obtained, i.e. produced by chemical synthesis. In further embodiments, the compounds may be obtained by synthetic modification of isolated compounds, e.g. isolated compounds from natural sources such as plant extracts. In yet other embodiments, the compounds may be obtained from a genetically modified organism or cells thereof. For example, genetically modified prokaryotic or eukaryotic cells or organisms. In various embodiments, the compounds may be obtained from genetically modified bacteria, yeasts, plants or plant cells. In such embodiments, the compounds are preferably isolated from the genetically modified organism. The purity of such isolated compound is not necessarily limited. In various embodiments, the purity of the isolated compound may be at least 20%, at least 30%, at least 40%, at least 50%, at least 60%, at least 70%, at least 80%, or at least 90%.

In various embodiments, the compound of Formula (I) is selected from genistein, hispidulin, kaempferol, naringenin, and quercetin. In various embodiments, the compound of Formula (I) is selected from flavone, genistein, hispidulin, kaempferol, and naringenin. In various embodiments, the compound of Formula (I) is selected from flavone, genistein, hispidulin, kaempferol, and quercetin. In various embodiments, the compound of Formula (I) is selected from flavone, genistein, hispidulin, naringenin, and quercetin. In various embodiments, the compound of Formula (I) is selected from flavone, genistein, kaempferol, naringenin, and quercetin. In various embodiments, the compound of Formula (I) is selected from flavone, hispidulin, kaempferol, naringenin, and quercetin. In various embodiments, the compound of Formula (I) is selected from genistein, hispidulin, kaempferol, naringenin, and quercetin.

In various embodiments, the compound of Formula (I) is selected from flavone, genistein, hispidulin, and kaempferol. In various embodiments, the compound of Formula (I) is selected from flavone, genistein, hispidulin, and naringenin. In various embodiments, the compound of Formula (I) is selected from flavone, genistein, hispidulin, and quercetin. In various embodiments, the compound of Formula (I) is selected from flavone, genistein, kaempferol, and naringenin. In various embodiments, the compound of Formula (I) is selected from flavone, genistein, kaempferol, and quercetin. In various embodiments, the compound of Formula (I) is selected from flavone, genistein, naringenin, and quercetin. In various embodiments, the compound of Formula (I) is selected from flavone, hispidulin, kaempferol, and naringenin. In various embodiments, the compound of Formula (I) is selected from flavone, hispidulin, kaempferol, and quercetin. In various embodiments, the compound of Formula (I) is selected from flavone, hispidulin, naringenin, and quercetin. In various embodiments, the compound of Formula (I) is selected from flavone, kaempferol, naringenin, and quercetin. In various embodiments, the compound of Formula (I) is selected from genistein, hispidulin, kaempferol, and naringenin. In various embodiments, the compound of Formula (I) is selected from genistein, hispidulin, kaempferol, and quercetin. In various embodiments, the compound of Formula (I) is selected from genistein, hispidulin, naringenin, and quercetin. In various embodiments, the compound of Formula (I) is selected from genistein, kaempferol, naringenin, and quercetin. In various embodiments, the compound of Formula (I) is selected from hispidulin, kaempferol, naringenin, and quercetin.

In various embodiments, the compound of Formula (I) is selected from flavone, genistein, and hispidulin. In various embodiments, the compound of Formula (I) is selected from flavone, genistein, and kaempferol. In various embodiments, the compound of Formula (I) is selected from flavone, genistein, and naringenin. In various embodiments, the compound of Formula (I) is selected from flavone, genistein, and quercetin. In various embodiments, the compound of Formula (I) is selected from flavone, hispidulin, and kaempferol. In various embodiments, the compound of Formula (I) is selected from flavone, hispidulin, and naringenin. In various embodiments, the compound of Formula (I) is selected from flavone, hispidulin, and quercetin. In various embodiments, the compound of Formula (I) is selected from flavone, kaempferol, and naringenin. In various embodiments, the compound of Formula (I) is selected from flavone, kaempferol, and quercetin. In various embodiments, the compound of Formula (I) is selected from flavone, naringenin, and quercetin. In various embodiments, the compound of Formula (I) is selected from genistein, hispidulin, and kaempferol. In various embodiments, the compound of Formula (I) is selected from genistein, hispidulin, and naringenin. In various embodiments, the compound of Formula (I) is selected from genistein, hispidulin, and quercetin. In various embodiments, the compound of Formula (I) is selected from genistein, kaempferol, and naringenin. In various embodiments, the compound of Formula (I) is selected from genistein, kaempferol, and quercetin. In various embodiments, the compound of Formula (I) is selected from genistein, naringenin, and quercetin. In various embodiments, the compound of Formula (I) is selected from hispidulin, kaempferol, and naringenin. In various embodiments, the compound of Formula (I) is selected from hispidulin, kaempferol, and quercetin. In various embodiments, the compound of Formula (I) is selected from hispidulin, naringenin, and quercetin. In various embodiments, the compound of Formula (I) is selected from kaempferol, naringenin, and quercetin.

In various embodiments, the compound of Formula (I) is selected from flavone and genistein. In various embodiments, the compound of Formula (I) is selected from flavone and hispidulin. In various embodiments, the compound of Formula (I) is selected from flavone and kaempferol. In various embodiments, the compound of Formula (I) is selected from flavone and naringenin. In various embodiments, the compound of Formula (I) is selected from flavone and quercetin. In various embodiments, the compound of Formula (I) is selected from genistein and hispidulin. In various embodiments, the compound of Formula (I) is selected from genistein and kaempferol. In various embodiments, the compound of Formula (I) is selected from genistein and naringenin. In various embodiments, the compound of Formula (I) is selected from genistein and quercetin. In various embodiments, the compound of Formula (I) is selected from hispidulin and kaempferol. In various embodiments, the compound of Formula (I) is selected from hispidulin and naringenin. In various embodiments, the compound of Formula (I) is selected from hispidulin and quercetin. In various embodiments, the compound of Formula (I) is selected from kaempferol and naringenin. In various embodiments, the compound of Formula (I) is selected from kaempferol and quercetin. In various embodiments, the compound of Formula (I) is selected from naringenin and quercetin.

In various embodiments, combinations of compounds of Formula (I) or derivatives thereof are used in the aspects described herein. For instance, in various embodiments the present disclosure provides an oral product comprising a combination of compounds of Formula (I) or derivatives thereof. Said compounds may be according to any of the embodiments described herein. The oral product may be a liquid dosage form or a solid oral product as defined herein, and may comprise the combination of compounds of Formula (I) or derivatives thereof in combination with further active ingredients. For instance, the combination of compounds of Formula (I) or derivatives thereof may be comprised in a combination of active ingredients further comprising one or more B vitamins selected from B1, B2, B6 and B7; or a compound selected from maca root, ginseng, coenzyme Q10, caffeine, vitamin C, beta-alanine, theacrine, taurine, zinc or a salt thereof, L-theanine, theobromine, Echinacea, selenium, lemon balm, chamomile, L-tryptophan, piperine or a geometric isomer thereof, saffron, and combinations thereof.

In various embodiments, the combination comprises at least two compounds of Formula (I) or derivatives thereof. In such embodiments, the compounds of Formula (I) or derivatives thereof may be as defined according to any of the embodiments described herein. For example, the compound of Formula (I) may be a compound of Formula (Ia) and/or (Ib) according to any of the embodiments disclosed herein. The compound of Formula (I) may be a compound of Formula (laa), (lab), (lac), (lad), (lae), (Iaf), (lag), (lah), (Iba), and/or (Ibb) according to any of the embodiments disclosed herein. In various embodiments, the compound of Formula (I) may be a compound of Formula (laa), (lab), (lac), (lad), (lae), (laf), (lag), and/or (lah) according to any of the embodiments disclosed herein. In various embodiments, the compound of Formula (I) may be a compound of Formula (Iba) and/or (Ibb) according to any of the embodiments disclosed herein.

For example, in various embodiments the combination comprises at least two compounds selected from flavone, genistein, hispidulin, kaempferol, naringenin, and quercetin. In various embodiments, the combination comprises at least two compounds selected from genistein, hispidulin, kaempferol, naringenin, and quercetin. In various embodiments, the combination comprises at least two compounds selected from flavone, genistein, hispidulin, kaempferol, and naringenin. In various embodiments, the combination comprises at least two compounds selected from flavone, genistein, hispidulin, kaempferol, and quercetin. In various embodiments, the combination comprises at least two compounds selected from flavone, genistein, hispidulin, naringenin, and quercetin. In various embodiments, the combination comprises at least two compounds selected from flavone, genistein, kaempferol, naringenin, and quercetin. In various embodiments, the combination comprises at least two compounds selected from flavone, hispidulin, kaempferol, naringenin, and quercetin. In various embodiments, the combination comprises at least two compounds selected from genistein, hispidulin, kaempferol, naringenin, and quercetin.

In various embodiments, the combination comprises at least two compounds selected from flavone, genistein, hispidulin, and kaempferol. In various embodiments, the combination comprises at least two compounds selected from flavone, genistein, hispidulin, and naringenin. In various embodiments, the combination comprises at least two compounds selected from flavone, genistein, hispidulin, and quercetin. In various embodiments, the combination comprises at least two compounds selected from flavone, genistein, kaempferol, and naringenin. In various embodiments, the combination comprises at least two compounds selected from flavone, genistein, kaempferol, and quercetin. In various embodiments, the combination comprises at least two compounds selected from flavone, genistein, naringenin, and quercetin. In various embodiments, the combination comprises at least two compounds selected from flavone, hispidulin, kaempferol, and naringenin. In various embodiments, the combination comprises at least two compounds selected from flavone, hispidulin, kaempferol, and quercetin. In various embodiments, the combination comprises at least two compounds selected from flavone, hispidulin, naringenin, and quercetin. In various embodiments, the combination comprises at least two compounds selected from flavone, kaempferol, naringenin, and quercetin. In various embodiments, the combination comprises at least two compounds selected from genistein, hispidulin, kaempferol, and naringenin. In various embodiments, the combination comprises at least two compounds selected from genistein, hispidulin, kaempferol, and quercetin. In various embodiments, the combination comprises at least two compounds selected from genistein, hispidulin, naringenin, and quercetin. In various embodiments, the combination comprises at least two compounds selected from genistein, kaempferol, naringenin, and quercetin. In various embodiments, the combination comprises at least two compounds selected from hispidulin, kaempferol, naringenin, and quercetin.

In various embodiments, the combination comprises at least two compounds selected from flavone, genistein, and hispidulin. In various embodiments, the combination comprises at least two compounds selected from flavone, genistein, and kaempferol. In various embodiments, the combination comprises at least two compounds selected from flavone, genistein, and naringenin. In various embodiments, the combination comprises at least two compounds selected from flavone, genistein, and quercetin. In various embodiments, the combination comprises at least two compounds selected from flavone, hispidulin, and kaempferol. In various embodiments, the combination comprises at least two compounds selected from flavone, hispidulin, and naringenin. In various embodiments, the combination comprises at least two compounds selected from flavone, hispidulin, and quercetin. In various embodiments, the combination comprises at least two compounds selected from flavone, kaempferol, and naringenin. In various embodiments, the combination comprises at least two compounds selected from flavone, kaempferol, and quercetin. In various embodiments, the combination comprises at least two compounds selected from flavone, naringenin, and quercetin. In various embodiments, the combination comprises at least two compounds selected from genistein, hispidulin, and kaempferol. In various embodiments, the combination comprises at least two compounds selected from genistein, hispidulin, and naringenin. In various embodiments, the combination comprises at least two compounds selected from genistein, hispidulin, and quercetin. In various embodiments, the combination comprises at least two compounds selected from genistein, kaempferol, and naringenin. In various embodiments, the combination comprises at least two compounds selected from genistein, kaempferol, and quercetin. In various embodiments, the combination comprises at least two compounds selected from genistein, naringenin, and quercetin. In various embodiments, the combination comprises at least two compounds selected from hispidulin, kaempferol, and naringenin. In various embodiments, the combination comprises at least two compounds selected from hispidulin, kaempferol, and quercetin. In various embodiments, the combination comprises at least two compounds selected from hispidulin, naringenin, and quercetin. In various embodiments, the combination comprises at least two compounds selected from kaempferol, naringenin, and quercetin.

In various embodiments, the combination comprises flavone and genistein. In various embodiments, the combination comprises flavone and hispidulin. In various embodiments, the combination comprises flavone and kaempferol. In various embodiments, the combination comprises flavone and naringenin. In various embodiments, the combination comprises flavone and quercetin. In various embodiments, the combination comprises genistein and hispidulin. In various embodiments, the combination comprises genistein and kaempferol. In various embodiments, the combination comprises genistein and naringenin. In various embodiments, the combination comprises genistein and quercetin. In various embodiments, the combination comprises hispidulin and kaempferol. In various embodiments, the combination comprises hispidulin and naringenin. In various embodiments, the combination comprises hispidulin and quercetin. In various embodiments, the combination comprises kaempferol and naringenin. In various embodiments, the combination comprises kaempferol and quercetin. In various embodiments, the combination comprises naringenin and quercetin.

In various embodiments, the combination comprises at least three compounds of Formula (I) or derivatives thereof. In such embodiments, the compounds of Formula (I) or derivatives thereof may be as defined according to any of the embodiments described herein. For example, the compound of Formula (I) may be a compound of Formula (Ia) and/or (Ib) according to any of the embodiments disclosed herein. The compound of Formula (I) may be a compound of Formula (laa), (lab), (lac), (lad), (lae), (Iaf), (lag), (lah), (Iba), and/or (Ibb) according to any of the embodiments disclosed herein. In various embodiments, the compound of Formula (I) may be a compound of Formula (laa), (lab), (lac), (lad), (lae), (laf), (lag), and/or (lah) according to any of the embodiments disclosed herein. In various embodiments, the compound of Formula (I) may be a compound of Formula (Iba) and/or (Ibb) according to any of the embodiments disclosed herein.

For example, in various embodiments the combination comprises at least three compounds selected from flavone, genistein, hispidulin, kaempferol, naringenin, and quercetin.

In various embodiments, the combination comprises at least three compounds selected from genistein, hispidulin, kaempferol, naringenin, and quercetin. In various embodiments, the combination comprises at least three compounds selected from flavone, genistein, hispidulin, kaempferol, and naringenin. In various embodiments, the combination comprises at least three compounds selected from flavone, genistein, hispidulin, kaempferol, and quercetin. In various embodiments, the combination comprises at least three compounds selected from flavone, genistein, hispidulin, naringenin, and quercetin. In various embodiments, the combination comprises at least three compounds selected from flavone, genistein, kaempferol, naringenin, and quercetin. In various embodiments, the combination comprises at least three compounds selected from flavone, hispidulin, kaempferol, naringenin, and quercetin. In various embodiments, the combination comprises at least three compounds selected from genistein, hispidulin, kaempferol, naringenin, and quercetin.

In various embodiments, the combination comprises at least three compounds selected from flavone, genistein, hispidulin, and kaempferol. In various embodiments, the combination comprises at least three compounds selected from flavone, genistein, hispidulin, and naringenin. In various embodiments, the combination comprises at least three compounds selected from flavone, genistein, hispidulin, and quercetin. In various embodiments, the combination comprises at least three compounds selected from flavone, genistein, kaempferol, and naringenin. In various embodiments, the combination comprises at least three compounds selected from flavone, genistein, kaempferol, and quercetin. In various embodiments, the combination comprises at least three compounds selected from flavone, genistein, naringenin, and quercetin. In various embodiments, the combination comprises at least three compounds selected from flavone, hispidulin, kaempferol, and naringenin. In various embodiments, the combination comprises at least three compounds selected from flavone, hispidulin, kaempferol, and quercetin. In various embodiments, the combination comprises at least three compounds selected from flavone, hispidulin, naringenin, and quercetin. In various embodiments, the combination comprises at least three compounds selected from flavone, kaempferol, naringenin, and quercetin. In various embodiments, the combination comprises at least three compounds selected from genistein, hispidulin, kaempferol, and naringenin. In various embodiments, the combination comprises at least three compounds selected from genistein, hispidulin, kaempferol, and quercetin. In various embodiments, the combination comprises at least three compounds selected from genistein, hispidulin, naringenin, and quercetin. In various embodiments, the combination comprises at least three compounds selected from genistein, kaempferol, naringenin, and quercetin. In various embodiments, the combination comprises at least three compounds selected from hispidulin, kaempferol, naringenin, and quercetin.

In various embodiments, the combination comprises flavone, genistein, and hispidulin. In various embodiments, the combination comprises flavone, genistein, and kaempferol. In various embodiments, the combination comprises flavone, genistein, and naringenin. In various embodiments, the combination comprises flavone, genistein, and quercetin. In various embodiments, the combination comprises flavone, hispidulin, and kaempferol. In various embodiments, the combination comprises flavone, hispidulin, and naringenin. In various embodiments, the combination comprises flavone, hispidulin, and quercetin. In various embodiments, the combination comprises flavone, kaempferol, and naringenin. In various embodiments, the combination comprises flavone, kaempferol, and quercetin. In various embodiments, the combination comprises flavone, naringenin, and quercetin. In various embodiments, the combination comprises genistein, hispidulin, and kaempferol. In various embodiments, the combination comprises genistein, hispidulin, and naringenin. In various embodiments, the combination comprises genistein, hispidulin, and quercetin. In various embodiments, the combination comprises genistein, kaempferol, and naringenin. In various embodiments, the combination comprises genistein, kaempferol, and quercetin. In various embodiments, the combination comprises genistein, naringenin, and quercetin. In various embodiments, the combination comprises hispidulin, kaempferol, and naringenin. In various embodiments, the combination comprises hispidulin, kaempferol, and quercetin. In various embodiments, the combination comprises hispidulin, naringenin, and quercetin. In various embodiments, the combination comprises kaempferol, naringenin, and quercetin.

In various embodiments, the combination comprises at least four compounds of Formula (I) or derivatives thereof. In such embodiments, the compounds of Formula (I) or derivatives thereof may be as defined according to any of the embodiments described herein. For example, the compound of Formula (I) may be a compound of Formula (Ia) and/or (Ib) according to any of the embodiments disclosed herein. The compound of Formula (I) may be a compound of Formula (laa), (lab), (lac), (lad), (lae), (Iaf), (lag), (lah), (Iba), and/or (Ibb) according to any of the embodiments disclosed herein. In various embodiments, the compound of Formula (I) may be a compound of Formula (laa), (lab), (lac), (lad), (lae), (laf), (lag), and/or (lah) according to any of the embodiments disclosed herein. In various embodiments, the compound of Formula (I) may be a compound of Formula (Iba) and/or (Ibb) according to any of the embodiments disclosed herein.

For example, in various embodiments the combination comprises at least four compounds selected from flavone, genistein, hispidulin, kaempferol, naringenin, and quercetin.ln various embodiments, the combination comprises at least four compounds selected from genistein, hispidulin, kaempferol, naringenin, and quercetin. In various embodiments, the combination comprises at least four compounds selected from flavone, genistein, hispidulin, kaempferol, and naringenin. In various embodiments, the combination comprises at least four compounds selected from flavone, genistein, hispidulin, kaempferol, and quercetin. In various embodiments, the combination comprises at least four compounds selected from flavone, genistein, hispidulin, naringenin, and quercetin. In various embodiments, the combination comprises at least four compounds selected from flavone, genistein, kaempferol, naringenin, and quercetin. In various embodiments, the combination comprises at least four compounds selected from flavone, hispidulin, kaempferol, naringenin, and quercetin. In various embodiments, the combination comprises at least four compounds selected from genistein, hispidulin, kaempferol, naringenin, and quercetin.

In various embodiments, the combination comprises flavone, genistein, hispidulin, and kaempferol. In various embodiments, the combination comprises flavone, genistein, hispidulin, and naringenin. In various embodiments, the combination comprises flavone, genistein, hispidulin, and quercetin. In various embodiments, the combination comprises flavone, genistein, kaempferol, and naringenin. In various embodiments, the combination comprises flavone, genistein, kaempferol, and quercetin. In various embodiments, the combination comprises flavone, genistein, naringenin, and quercetin. In various embodiments, the combination comprises flavone, hispidulin, kaempferol, and naringenin. In various embodiments, the combination comprises flavone, hispidulin, kaempferol, and quercetin. In various embodiments, the combination comprises flavone, hispidulin, naringenin, and quercetin. In various embodiments, the combination comprises flavone, kaempferol, naringenin, and quercetin. In various embodiments, the combination comprises genistein, hispidulin, kaempferol, and naringenin. In various embodiments, the combination comprises genistein, hispidulin, kaempferol, and quercetin. In various embodiments, the combination comprises genistein, hispidulin, naringenin, and quercetin. In various embodiments, the combination comprises genistein, kaempferol, naringenin, and quercetin. In various embodiments, the combination comprises hispidulin, kaempferol, naringenin, and quercetin.

In various embodiments, the combination comprises at least five compounds of Formula (I) or derivatives thereof. In such embodiments, the compounds of Formula (I) or derivatives thereof may be as defined according to any of the embodiments described herein. For example, the compound of Formula (I) may be a compound of Formula (Ia) and/or (Ib) according to any of the embodiments disclosed herein. The compound of Formula (I) may be a compound of Formula (laa), (lab), (lac), (lad), (lae), (Iaf), (lag), (lah), (Iba), and/or (Ibb) according to any of the embodiments disclosed herein. In various embodiments, the compound of Formula (I) may be a compound of Formula (laa), (lab), (lac), (lad), (lae), (laf), (lag), and/or (lah) according to any of the embodiments disclosed herein. In various embodiments, the compound of Formula (I) may be a compound of Formula (Iba) and/or (Ibb) according to any of the embodiments disclosed herein.

For example, in various embodiments the combination comprises at least five compounds selected from flavone, genistein, hispidulin, kaempferol, naringenin, and quercetin. In various embodiments, the combination comprises genistein, hispidulin, kaempferol, naringenin, and quercetin. In various embodiments, the combination comprises flavone, genistein, hispidulin, kaempferol, and naringenin. In various embodiments, the combination comprises flavone, genistein, hispidulin, kaempferol, and quercetin. In various embodiments, the combination comprises flavone, genistein, hispidulin, naringenin, and quercetin. In various embodiments, the combination comprises flavone, genistein, kaempferol, naringenin, and quercetin. In various embodiments, the combination comprises flavone, hispidulin, kaempferol, naringenin, and quercetin. In various embodiments, the combination comprises genistein, hispidulin, kaempferol, naringenin, and quercetin.

In some embodiments, the combination comprises at least six compounds of Formula (I) or derivatives thereof. In such embodiments, the compounds of Formula (I) or derivatives thereof may be as defined according to any of the embodiments described herein. For example, the compound of Formula (I) may be a compound of Formula (Ia) and/or (Ib) according to any of the embodiments disclosed herein. The compound of Formula (I) may be a compound of Formula (laa), (lab), (lac), (lad), (lae), (Iaf), (lag), (lah), (Iba), and/or (Ibb) according to any of the embodiments disclosed herein. In various embodiments, the compound of Formula (I) may be a compound of Formula (laa), (lab), (lac), (lad), (lae), (laf), (lag), and/or (lah) according to any of the embodiments disclosed herein. In various embodiments, the compound of Formula (I) may be a compound of Formula (Iba) and/or (Ibb) according to any of the embodiments disclosed herein.

For example, in some embodiments the combination comprises flavone, genistein, hispidulin, kaempferol, naringenin, and quercetin.

As used herein, the term "derivative" includes salts and/or solvates (including hydrates).

Examples of salts of the compound of Formula (I) and embodiments thereof described herein include suitable acid addition or base salts thereof. Suitable salts include pharmaceutically acceptable salts, a review of which is found in Berge et al, J Pharm Sci, 66, 1-19 (1977). Suitable acid addition salts include carboxylate salts (e.g. formate, acetate, trifluoroacetate, propionate, isobutyrate, heptanoate, decanoate, caprate, caprylate, stearate, acrylate, caproate, propiolate, ascorbate, citrate, glucuronate, glutamate, glycolate, α-hydroxybutyrate, lactate, tartrate, phenylacetate, mandelate, phenylpropionate, phenylbutyrate, benzoate, chlorobenzoate, methylbenzoate, hydroxybenzoate, methoxybenzoate, dinitrobenzoate, o-acetoxybenzoate, salicylate, nicotinate, isonicotinate, cinnamate, oxalate, malonate, succinate, suberate, sebacate, fumarate, malate, maleate, hydroxymaleate, hippurate, phthalate or terephthalate salts), halide salts (e.g. chloride, bromide or iodide salts), sulfonate salts (e.g. benzenesulfonate, methyl-, bromo- or chlorobenzenesulfonate, xylenesulfonate, methanesulfonate, ethanesulfonate, propanesulfonate, hydroxyethanesulfonate, 1- or 2- naphthalene-sulfonate or 1,5-naphthalenedisulfonate salts) or sulfate, pyrosulfate, bisulfate, sulfite, bisulfite, phosphate, monohydrogenphosphate, dihydrogenphosphate, metaphosphate, pyrophosphate or nitrate salts. Suitable base salts include metal salts, e.g. sodium, calcium, and amine salts, e.g. tromethamine.

As used herein, the term "solvate" includes complexes of the compound of Formula (I) with one or more solvents. For example, hydrates, wherein the solvent complexed to the compound of Formula (I) is water. Suitable solvates include but are not limited to crystalline multi-component systems in which one or more solvents are accommodated by the crystal structure in a stoichiometric or non-stoichiometric manner.

In various embodiments, the term "derivative" includes compounds wherein the compound of Formula (I) has been subjected to one or more chemical reactions. In various embodiments, said one or more chemical reactions comprises substitution and/or addition at the C-5, C-6, C-7, C-8, C-2', C-3', C-4', C-5', and/or C-6' positions of the compound of Formula (I). Thus, in various embodiments the derivative of the compound of Formula (I) includes compounds wherein one or more of the C-5, C-6, C-7, C-8, C-2', C-3', C-4', C-5', and/or C-6' positions of the compound of Formula (I) is bonded to a substituent selected from alkyl, alkenyl, halo, haloalkyl, hydroxyl, carboxyl, acyl, acyloxy, amino, alkyl- or dialkylamino, amido, arylamino, alkoxy, aryloxy, nitro, cyano, azido, thiol, imino, sulfonic acid, sulfate, sulfonyl, sulfanyl, sulfinyl, sulfamoyl, ester, phosphonyl, phosphinyl, phosphoryl, phosphine, thioester, thioether, acid halide, anhydride, oxime, hydrazine, carbamate, phosphonic acid, phosphate, phosphonate, or any other viable functional group that does not inhibit the biological activity of the compounds of the present disclosure, either unprotected, or protected as necessary, as known to those skilled in the art, for example, as taught in Greene and Wuts, Protective Groups in Organic Synthesis, John Wiley and Sons, Third Edition, 1999. In the foregoing embodiments, up to 8, up to 7, up to 6, up to 5, up to 4, up to 3, up to 2, or 1 of the C-5, C-6, C-7, C-8, C-2', C-3', C-4', C-5', and/or C-6' positions of the compound of Formula (I) may be bonded to a substituent as defined herein. In any of the foregoing embodiments, the derivative may include compounds wherein the compound of Formula (I) has been subjected to up to 5, up to 4, up to 3, up to 2, or 1 chemical reaction(s), which may comprise substitution and/or addition at the C-5, C-6, C-7, C-8, C-2', C-3', C-4', C-5', and/or C-6' positions of the compound of Formula (I). In various embodiments, the resulting group formed by the addition/substitution reaction(s) may be selected from any of the foregoing substituents.

In various embodiments, the term "derivative" includes compounds wherein a hydrogen atom comprised in one or more hydroxyl (OH) groups of the compound of Formula (I) has been substituted. In such embodiments, the hydrogen atom of the hydroxyl group may be substituted to form a group selected from O-alkyl, O-alkenyl, O-acyl, sulfonyl, sulfonic acid, sulfate, and phosphate. For example, up to 8, up to 7, up to 6, up to 5, up to 4, up to 3, up to 2 or one hydroxyl groups may be modified by substituting the hydrogen atom of the hydroxyl group to form a group selected from O-alkyl, O-alkenyl, O-acyl, sulfonyl, sulfonic acid, sulfate, and phosphate. In further embodiments, the term "derivative" includes glycosides of the compound of Formula (I) defined herein. As used herein, the term "glycoside" refers to the case wherein a sugar group (glycosyl group) is bonded through its anomeric carbon to the compound of Formula (I) via a glycosidic bond. Such glycosides are known to occur naturally, but may also be obtained by chemical reaction or biosynthetically (e.g. *in vitro* by the use of enzymes such glycosyltransferases, or *in vivo e.g.* in genetically modified organisms). Exemplary and non-limiting examples of common sugar groups that may form glycosides with the compound of Formula (I) include glucose, rhamnose, galactose, xylose, arabinose, glucoronic acid, and apiose. In various embodiments, the glycoside of the compound of Formula (I) is a O-glycoside, an N-glycoside, an S-glycoside, or a C-glycoside; preferably an O-glycoside or C-glycoside, more preferably an O-glycoside. In further embodiments of the glycoside, the sugar molecule is bonded through a glycosidic bond formed from a hydroxyl group comprised in the compound of Formula (I). In further embodiments, the glycosyl group of the glycoside is bonded via a glycosidic bond at the C-5, C-6, C-7, C-8, C-2', C-3', C-4', C-5', or C-6' position of the compound of Formula (I). In any of the foregoing embodiments, the glycoside may comprise one or more other modifications as encompassed by the definition of "derivative" provided herein above. In particular, salts and/or solvates (including hydrates) of the glycosides of the present disclosure are also contemplated.

### Oral Product

As described herein, the compound of Formula (I), a derivative thereof, or a combination thereof as defined according to any of the embodiments disclosed herein may be used in an oral product. Typically, said compound of Formula (I), a derivative thereof, or a combination thereof may be comprised in a combination of active ingredients.

Accordingly, an aspect of the present disclosure provides an oral product comprising a combination of active ingredients, wherein the combination of active ingredients comprises: (a) a compound of Formula (I), a derivative thereof, or a combination thereof; and (b) one or more B vitamins selected from B1, B2, B6 and B7; or a compound selected from maca root, ginseng, coenzyme Q10, caffeine, vitamin C, beta-alanine, theacrine, taurine, zinc or a salt thereof, L-theanine, theobromine Echinacea, selenium, lemon balm, chamomile, L-tryptophan, piperine or a geometric isomer thereof, saffron, and combinations thereof,.

The oral product is configured for oral use, and thus for insertion into the user's mouth (i.e. oral cavity). In some embodiments, the oral product is a liquid oral product, such as a liquid dosage form. In such embodiments, the term "oral" in connection to said liquid product means that, in normal use, said product is suited to be ingested orally by the user. For example, the product may be in the form of a beverage with a volume of 250 mL or greater, or a liquid shot with a volume of from about 1 mL to about 200 mL, preferably about 20 mL to about 100 mL.

In other embodiments, the oral product is a solid oral product e.g. a lozenge or a chew. For example, the solid oral product may be a solid oral product in chewable form. In such embodiments, the oral product is configured for oral use, and thus for insertion into the user's mouth (i.e. oral cavity). In such embodiments, the term "oral" in connection to said solid oral product refers to a product which, in normal use, is suited to be ingested or placed somewhere in the oral cavity of the user. For example, the product may be placed in the mouth, e.g. chewed in the oral cavity, for example in the form of a chew or chewing gum.

The person skilled in the art will understand that the ranges provided herein may be taken alone or combined with one or more other component ranges to provide a preferred aspect of the invention.

### Combination of active ingredients

The product as disclosed herein includes active ingredients. In particular, the combination of active ingredients comprises the compound of Formula (I), derivative thereof, or combination thereof as disclosed herein. The combination of active ingredients comprises suitable active ingredients that cause a biological response in a human or animal. In particular, the combination of active ingredients may comprise (a) a compound of Formula (I), a derivative thereof, or a combination thereof; and (b) one or more B vitamins selected from B1, B2, B6 and B7; or a compound selected from maca root, ginseng, coenzyme Q10, caffeine, vitamin C, beta-alanine, theacrine, taurine, zinc or a salt thereof, L-theanine, theobromine, Echinacea, selenium, lemon balm, chamomile, L-tryptophan, piperine or a geometric isomer thereof, saffron and combinations thereof,. Each of the active ingredients may be present in an amount suitable to provide the desired biological response in a human animal. The active ingredients may be naturally occurring or synthetically obtained.

The compound of Formula (I), derivative thereof, or combination thereof may be present in any suitable amount, such as in an amount of at least about 0.0001% by weight, at least about 0.001% by weight, at least about 0.01% by weight, at least about 0.1% by weight, or at least about 1% by weight. In some embodiments, the compound of Formula (I), derivative thereof, or combination thereof may be present in an amount of no greater than about 20% by weight, no greater than about 15% by weight, no greater than about 10% by weight, no greater than about 5% by weight, or no greater than about 2% by weight of the oral product.

The compound of Formula (I), derivative thereof, or combination thereof may in various embodiments be present in an amount of from about 0.001% to about 20%, from about 0.001% to about 15%, from about 0.001% to about 10%, from about 0.001% to about 5%, from about 0.001% to about 2% by weight of the oral product.

The compound of Formula (I), derivative thereof, or combination thereof may in various embodiments be present in an amount of from about 0.0001% to about 20%, from about 0.001% to about 15%, from about 0.01% to about 10%, from about 0.1% to about 5%, or from about 1% to about 2% by weight of the oral product. In some embodiments, the compound of Formula (I), derivative thereof, or combination thereof may be present in an amount of from about 0.0001 % to about 2%, from about 0.001% to about 2%, from about 0.01% to about 2%, or from about 0.1% to about 2% by weight of the oral product.

Where a combination of compounds of Formula (I) and/or derivatives thereof is used in the oral product, it will be understood that the above amounts refer to the total amount of compounds of Formula (I) and/or derivatives thereof.

The combination of active ingredients may comprise one or more B vitamins selected from B1, B2, B6 and B7. In other embodiments, the combination of active ingredients may comprise a combination of at least two B vitamins selected from vitamin B1, vitamin B2, vitamin B3, vitamin B5, vitamin B6, vitamin B7, vitamin B9 and vitamin B12. Preferably the combination of B vitamins comprises vitamin B2 and vitamin B6, more preferably the combination of B vitamins further comprises one or more of vitamin B1 or vitamin B7.

The term "B vitamins" includes a group of eight water-soluble vitamins that have effect in cellular functioning; e.g. cell metabolism and synthesis of red blood cells. B vitamins act as coenzymes in a substantial proportion of the enzymatic processes for cellular physiological functioning. The B vitamins may generally be divided into two categories: those that act as co-enzymes for catabolic enzymatic reactions (leading to generation of energy) and those that act as co-enzymes for anabolic enzymatic reactions (leading to enhanced brain function).

B vitamins are considered to have roles in aspects of brain function and energy production. The eight water soluble B vitamins are vitamin B1 (thiamin), vitamin B2 (riboflavin), vitamin B3 (niacin), vitamin B5 (panthothenic acid or salt thereof, e.g. calcium pantothenate), vitamin B6 (pyridoxine hydrochloride), vitamin B7 (biotin), vitamin B9 (folate or folic acid), vitamin B12 (cyanocobalamin). Vitamins B2, B3, B6, B9 and B12 have been found to support anabolic metabolism and to boost brain function. Vitamins B1, B2, B3, B5 and B7 have been found to support catabolic metabolism and to boost energy levels.

In preferred embodiments, the oral product includes at least two of the B vitamins that support anabolic metabolism. In other words, according to a first aspect, the combination of at least two B vitamins is selected from vitamins B2, B3, B6, B9 and B12. It has been found that the inclusion of at least these B vitamins may reduce perceived physical and mental fatigue as well as enhance psychological and cognitive functions. In particularly preferred embodiments, the oral product includes all five B vitamins that support anabolic metabolism. In other words, according to a first aspect, the combination of active ingredients includes a combination of vitamins B2, B3, B6, B9 and B12. Each of these B vitamins may contribute to the reduction of tiredness and fatigue, and the inclusion of all five B vitamins may lead to a synergistic effect in this regard.

In some embodiments, the combination of B vitamins consists essentially of all anabolic vitamins; i.e. a combination of vitamins B2, B3, B6, B9 and B12. In some embodiments, the combination of B vitamins consists of all anabolic vitamins; i.e. a combination of vitamins B2, B3, B6, B9 and B12. The term "anabolic B vitamin" refers to those B vitamins that have an effect on anabolic pathways, while the term "catabolic B vitamin" refers to those B vitamins that have an effect on catabolic pathways.

In some embodiments, the combination of B vitamins further comprises at least one catabolic B vitamin selected from the group consisting of vitamin B1, vitamin B5, vitamin B7 and combinations thereof.

In any such embodiments, the ratio of the total amount of anabolic B vitamins (i.e. vitamins B2, B3, B6, B9 and B12) to the total amount of catabolic B vitamins (i.e. vitamins B1, B5 and B7) may be from about 5:1 to about 1:5, preferably from about 2:1 to about 1:2 and more preferably from about 2:1 to about 1:1.

In other embodiments, the combination of B vitamins may comprise at least vitamin B1 and vitamin B2, or at least vitamin B6 and vitamin B7, or at least vitamin B1 and B6, or at least vitamin B2 and vitamin B7, preferably the combination of B vitamins may comprise at least vitamin B1, vitamin B2 and vitamin B6. In such embodiments, the combination of B vitamins may comprise at least vitamin B1, vitamin B2, vitamin B6, and vitamin B7, preferably the combination of B vitamins may consist essentially of vitamin B1, vitamin B2, and vitamin B6, and vitamin B7.

The amounts of the B vitamins included in the composition may be any amount suitable to provide the desired effect while also providing a product that is safe to consume and has reduced side effects. The total amount of B vitamins included in the combination may be any suitable amount to provide the desired effect of boosting brain function. In some embodiments, the total amount of anabolic B vitamins (i.e. the total amount of the combination of vitamins B2, B3, B6, B9 and B12) may be at least about 0.0001% by weight, preferably at least about 0.001% by weight, and more preferably at least about 0.01% by weight of the oral product. For example, the total amount of anabolic B vitamins may be from about 0.0001% to about 5% by weight of the oral product, and preferably from about 0.001% to about 1%. For example, this amount may be from about 0.01% to about 0.5% by weight of the oral product, or from about 0.05% to about 0.5% by weight of the oral product.

Where present, the total amount of catabolic B vitamins (i.e. the total amount of the combination of vitamins B1, B5 and B7) may be at least about 0.0001% by weight, preferably at least about 0.001% by weight, and more preferably at least about 0.01% by weight of the oral product. For example, the total amount of catabolic B vitamins (e.g. where the catabolic B vitamins are vitamin B1, vitamin B2, vitamin B3, vitamin B5 and/or vitamin B7) may be from about 0.0001% to about 5% by weight of the oral product, and preferably from about 0.001% to about 1%. For example, this amount may be from about 0.01% to about 0.5% by weight of the oral product.

The total amount of B vitamins included in the combination may be any suitable amount to provide the desired effect of boosting brain function. In some embodiments, the total amount of B vitamins is from about 0.001% to about 10% by weight, and preferably from about 0.001% to about 5% by weight of the oral product. In some embodiments, the total amount of B vitamins may be from about 0.01% to about 4% by weight, such as from about 0.025% to about 3% by weight, preferably from about 0.05% to about 2% by weight of the oral product.

In some embodiments, vitamin B2 may be present in an amount of from about 1% to about 20% by weight of the total amount of B vitamin in the oral product. For example, vitamin B2 may be present in an amount of from about 5% to about 15% by weight of the total amount of B vitamin in the oral product. In other embodiments, vitamin B2 may be present in an amount of from about 1% to about 40% by weight of the total amount of B vitamin in the oral product. For example, vitamin B2 may be present in an amount of from about 10% to about 40% by weight of the total amount of B vitamin in the oral product.

In some embodiments, vitamin B3 may be present in an amount of from about 20% to about 80% by weight of the total amount of B vitamin in the oral product. For example, vitamin B3 may be present in an amount of from about 40% to about 70% by weight, and preferably from about 45% to about 60% by weight of the total amount of B vitamin in the oral product. In other embodiments, the combination of B vitamins do not include vitamin B3.

In some embodiments, vitamin B6 may be present in an amount of from about 1% to about 15% by weight of the total amount of B vitamin in the oral product. For example, vitamin B6 may be present in an amount of from about 5% to about 10% by weight of the total amount of B vitamin in the oral product. In other embodiments, vitamin B6 may be present in an amount of from about 1% to about 50% by weight of the total amount of B vitamin in the oral product. For example, vitamin B6 may be present in an amount of from about 5% to about 50% by weight of the total amount of B vitamin in the oral product.

In some embodiments, vitamin B9 may be present in an amount of from about 0.1% to about 10% by weight of the total amount of B vitamin in the oral product. For example, vitamin B9 may be present in an amount of from about 1% to about 5% by weight of the total amount of B vitamin in the oral product.

In some embodiments, vitamin B12 may be present in an amount of from about 0.01% to about 30% by weight, such as from about 0.1% to about 25% by weight, such as from about 1% to about 20% by weight of the total amount of B vitamin in the oral product. For example, vitamin B12 may be present in an amount of from about 5% to about 15% by weight of the total amount of B vitamin in the oral product.

Where present, vitamin B1 may be present in an amount of from about 1% to about 10% by weight of the total amount of B vitamin in the oral product. For example, where present, vitamin B1 may be present in an amount of from about 2.5% to about 7.5% by weight of the total amount of B vitamin in the oral product. In some embodiments, the oral product may be substantially free of (i.e. include no more than 0.0001% by weight of) vitamin B1, or may be free of (i.e. include 0%) vitamin B1. In other embodiments, vitamin B1 may be present in an amount of from about 1% to about 40% by weight of the total amount of B vitamin in the oral product. For example, vitamin B1 may be present in an amount of from about 5% to about 35% by weight of the total amount of B vitamin in the oral product.

Where present, vitamin B5 may be present in an amount of from about 1% to about 20% by weight of the total amount of B vitamin in the oral product. For example, where present, vitamin B5 may be present in an amount of from about 5% to about 15% by weight of the total amount of B vitamin in the oral product. In some embodiments, the oral product may be substantially free of vitamin B5, or may be free of vitamin B5.

Where present, vitamin B7 may be present in an amount of from about 0.01% to about 5% by weight of the total amount of B vitamin in the oral product. For example, where present, vitamin B7 may be present in an amount of from about 0.01% to about 1% by weight or about 0.01% to about 2% by weight of the total amount of B vitamin in the oral product. In some embodiments, the oral product may be substantially free of vitamin B7, or may be free of vitamin B7.

Maca or *Lepidium meyenii* is an edible herbaceous biennial plant of the family *Brassicaceae* that is native to South America. It is otherwise known as `Peruvian ginseng' and is mainly grown for consumption of its root. Traditionally, maca is processed by boiling, baking, and drying the root and then grinding the dried root material into powder. Various maca root powders are commercially available. It may be used for its presumed benefits including increasing fertility, boosting energy and endurance, improving mood, reducing blood pressure, and improving learning and memory. Fresh maca roots have different colours due to genetic variations. The colours include yellow, red, purple, blue, black and green. Yellow maca is the sweetest-tasting and is most commonly grown for food, while red and black maca are generally considered more medicinally potent. The maca root included in the oral product can be black maca root, yellow maca root, red maca root, or any combination thereof. The maca root may be provided in the form of an extract or in the form of a powder (e.g. a powder extract).

The maca extract is a concentrated form of maca that can be obtained by standard extraction techniques that are well known in the art. For example, maca extract may be obtained by taking dried maca root that has been cut into small pieces or, preferably, pulverized to a powder form and soaking in a solution. Although maca root can be extracted in organic solutions such as chloroform, to minimise safety concerns, it is preferable that the extraction solution is an aqueous or more preferably an aqueous/alcoholic solution, for example a water and ethanol solution. The extraction solution is usually provided in excess. Preferably, the extraction temperature is from room temperature to less than boiling temperature of the solution, such as from about 20°C up to about 100°C or from about 25°C up to about 80°C. Techniques such as sonication may be used to increase extraction efficiency. Techniques such as filtration are preferably used to reduce particulate in the maca extract. The extraction solution can then be dried, preferably to powder form, to give a concentrated maca extract. The maca root of the present invention may be a ratio of concentrated maca extract to filler. For example, a 10:1 maca root ingredient purity may be obtained by using 300 mg of concentrated maca extract to give 3 grams of maca root ingredient. The type of extract is not limited in the present disclosure.

The maca root may be present in an amount of from about 0.01% to about 20% by weight of the oral product. In some embodiments, the maca root is present in an amount of from about 0.1% to about 15% by weight of the oral product. The maca root may preferably be present in an amount of from about 0.25% to about 10% by weight of the oral product.

Ginseng is the root of plants in the genus *Panax,* which are characterised by the presence of unique steroid saponin phytochemicals (ginsenosides) and gintonin. The putative major active components of ginseng comprise more than 100 species specific triterpene saponins or 'ginsenosides'. Ginseng and ginseng extracts also contain a range of other potentially bioactive components, including alkaloids, phytosterols, sesquiterpenes, and polyphenols. Ginseng finds use as a dietary supplement in energy drinks or herbal teas, and in traditional medicine. The ginseng may include any suitable form of ginseng, such as *Panax ginseng* (Korean ginseng), *Panax notoginseng* (China ginseng) and *Panax quinquefolius* (American ginseng). The ginseng may also comprise Ashwagandha (Withania somnifera), commonly known as Indian Ginseng. The ginseng may be present in the form of ginseng extract, chopped ginseng, shredded ginseng or powdered ginseng. Preferably, the ginseng is included in the form of ginseng extract or powdered ginseng extract. The ginseng may white ginseng, fresh ginseng or red ginseng. In some embodiments, the ginseng is red ginseng, such as red ginseng extract or powdered red ginseng extract. The inclusion of ginseng has been found to be beneficial in improving the cognitive effects of the composition. Ginseng also has anti-inflammatory and antioxidant properties.

In some embodiments, the ginseng is present in an amount of from about 0.01% to about 5% by weight of the oral product, such as in an amount of from about 0.05% to about 2% by weight of the oral product. In preferred embodiments, the ginseng is present in an amount of from about 0.1% to about 1% by weight of the oral product.

Coenzyme Q10 is also known as ubiquinone-10; it is a 1,4-benzoquinone, where Q refers to the quinone chemical group and 10 refers to the number of isoprenyl chemical subunits in the tail, and has the following chemical formula:

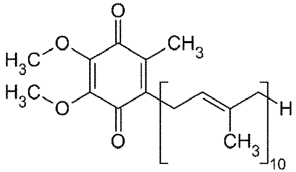

Coenzyme Q10 or CoQ₁₀ is a crystalline powder. It acts as an antioxidant, which protects cells from damage. It also plays an important part in the human metabolism and has been used to treat many different conditions including heart failure.

In some embodiments, Coenzyme Q10 is present in an amount of from about 0.001% to about 30% by weight of the oral product. In some embodiments, Coenzyme Q10 is present in an amount of from about 0.01% to about 20% by weight of the oral product. In some embodiments, the Coenzyme Q10 is present in an amount of from about 0.1% to about 10% by weight of the oral product. In some preferred embodiments, Coenzyme Q10 is present in an amount of from about 0.01% to about 5% by weight of the oral product, and particularly preferably from about 0.1% to about 1% by weight of the oral product.

Coenzyme Q10 may in some embodiments (e.g. where the oral product is a liquid oral dosage form) be present in an amount of from about 0.01% to about 5% by weight of the oral product. In some embodiments (e.g. where the oral product is a liquid oral dosage form), the Coenzyme Q10 is present in an amount of from about 0.1% to about 1% by weight, such as from about 0.1% to about 0.75% by weight, preferably from about 0.1% to about 0.5% by weight of the oral product.

The oral product may be caffeine-free, or may include caffeine in the combination of active ingredients. Caffeine is a central nervous system stimulant. Caffeine (1,3,7-trimethylxanthine) exists as a bitter substance, an alkaloid which is naturally found in a variety of plant species, including tea leaves, kola nuts, cocoa, guarana, and coffee beans. Once ingested, caffeine is metabolised through the liver *via* the cytochrome P450 enzyme system and is capable of entering all body tissues, as well as crossing the blood brain barrier. Caffeine may be used to enhance alertness, attention, cognitive performance as well as physical performance.

The caffeine may be included as a natural stimulant, meaning that it is naturally derived. By "naturally derived" is meant that the caffeine is in a purified form, outside its natural (e.g. botanical) matrix. For example, caffeine may be obtained by extraction and purification from botanical sources (e.g. tea). Alternatively, or in addition, the caffeine may be provided in synthetic form; i.e. obtained by chemical synthesis. The caffeine may be provided in the form of an extract or in the form of a powder (e.g. a powder extract). For example, the caffeine may be included in the form of a green tea powder extract, black tea powder extract. The caffeine may also be provided in the form of guarana seed powder extract. In some embodiments, the caffeine is present in an encapsulated form. An example of an encapsulated caffeine is Vitashure^{®}, available from Balchem Corp., 52 Sunrise Park Road, New Hampton, NY, 10958.

The caffeine may be present in any suitable amount, such as in an amount of at least about 0.01% by weight, at least about 0.1% by weight, at least about 1% by weight, or at least 2% by weight of the oral product. In some embodiments, the caffeine may be present in an amount of no greater than about 35% by weight, no greater than about 20% by weight, no greater than about 10% by weight, or no greater than about 5% by weight of the oral product.

The caffeine may be present in an amount of from about 0.01% to about 35% by weight of the oral product. In some embodiments, the caffeine is present in an amount of from about 0.1% to about 20% by weight of the oral product. In further embodiments the caffeine may be present in an amount of from about 0.1% to about 10% by weight of the oral product. In yet further embodiments, the caffeine may be present in an amount from about 0.1% to about 5% by weight of the oral product. The caffeine may alternatively be present in an amount of from about 1% to about 35% by weight.

As discussed herein, the compound of Formula (I), derivative thereof or combination thereof has been found to provide one or more of the desirable physiological effects of caffeine while reducing/avoiding one or more of the undesirable physiological effects of caffeine. Hence, in various embodiments the compound of Formula (I), derivative thereof or combination thereof is used in the oral product as a substitute for caffeine. In such embodiments, the oral product may not comprise caffeine, i.e. it is caffeine-free.

In other embodiments, the compound of Formula (I), derivative thereof or combination thereof may be used in combination with caffeine. For example, in various embdiments, the compound of Formula (I), derivative thereof or combination thereof is used in combination with caffeine, e.g. an amount according to the embodiments disclosed herein above, to enhance one or more desirable effects and/or reduce one or more undesirable effects of the caffeine. In some embodiments, the enhancement provided by the compound of Formula (I), derivative thereof or combination thereof may be additive. In some advantageous embodiments, the enhancement provided by the compound of Formula (I), derivative thereof or combination thereof may be synergistic, that is to say greater than additive. Such synergy is not predictable or expected when two actives are used in combination.

In further embodiments, the compound of Formula (I), derivative thereof or combination thereof may be used in combination with caffeine such that the amount of caffeine can be reduced relative to an equivalent oral product that does not contain the compound of Formula (I), derivative thereof or combination, i.e. while achieving the same desired physiological effects.

Thus, in various embodiments the compound of Formula (I), derivative thereof, or combination thereof may be present in an amount of from about 0.001% to about 20%, from about 0.001% to about 15%, from about 0.001% to about 10%, or from about 0.001% to about 5% by weight of the oral product and the caffeine may be present in an amount of from about 0.1% to about 20%, from about 0.1% to about 10%, or from about 0.1% to about 5% by weight of the oral product. In various embodiments the compound of Formula (I), derivative thereof, or combination thereof may be present in an amount of from about 0.001% to about 20%, from about 0.001% to about 15%, from about 0.001% to about 10%, or from about 0.001% to about 5% by weight of the oral product and the caffeine may be present in an amount of from about 0.01% to about 20%, from about 0.01% to about 10%, or from about 0.01% to about 5% by weight of the oral product. In various embodiments the compound of Formula (I), derivative thereof, or combination thereof may be present in an amount of from about 0.01% to about 20%, from about 0.01% to about 15%, from about 0.01% to about 10%, or from about 0.01% to about 5% by weight of the oral product and the caffeine may be present in an amount of from about 0.01% to about 20%, from about 0.01% to about 10%, or from about 0.01% to about 5% by weight of the oral product. In various embodiments the compound of Formula (I), derivative thereof, or combination thereof may be present in an amount of from about 0.1% to about 20%, from about 0.1% to about 15%, from about 0.1% to about 10%, or from about 0.1% to about 5% by weight of the oral product and the caffeine may be present in an amount of from about 0.01% to about 20%, from about 0.01% to about 10%, or from about 0.01% to about 5% by weight of the oral product.

The combination of active ingredients may further comprise vitamin C (ascorbic acid). Vitamin C acts as an antioxidant, helping to protect cells from the damage caused by free radicals. It is also used by the body to make collagen, a protein that may help wounds heal. In addition, vitamin C improves the absorption of iron from plant-based foods and helps the immune system work properly to protect the body from disease. The inclusion of vitamin C may reduce tiredness and fatigue.

The vitamin C may be present in the oral product in any suitable amount. In some embodiments, the vitamin C may be present in an amount of from about 0.01% to about 10% by weight, such as from about 0.05% to about 8% by weight, preferably in an amount of from about 0.1% to about 5% by weight of the oral product.

In some embodiments the combination of active ingredients may include beta-alanine. Beta-alanine or 3-aminopropanoic acid is a naturally occurring beta amino acid. There is evidence that beta-alanine supplements can increase energy and cognitive performance as well as prevent muscle soreness and fatigue. Beta-alanine is converted within muscle cells to carnosine which acts as a buffer for the lactic acid produced during high intensity exercise, and can thereby help delay the onset of neuromuscular fatigue.

The beta-alanine may be present in any suitable amount, such as an amount of from about 0.01% to about 60% by weight of the oral product. In some embodiments, the beta-alanine is present in an amount of from about 0.1% to about 50% by weight of the oral product. In other embodiments (for example when the oral product is in liquid dosage form), the beta-alanine is present in an amount of from about 1% to about 10% by weight of the oral product.

In some embodiments, the combination of active ingredients further comprises theacrine (1,3,7,9-tetramethyluric acid). Theacrine is a purine alkanoid found in *Theobroma grandiflorum* (commonly known as copoasu, a tropical rainforest tree related to cacao) and in a Chinese tea known as kucha. Theacrine shows anti-inflammatory and analgesic effects and appears to affect adenosine signaling in a manner similar to caffeine. Structurally theacrine is similar to caffeine; it has an additional methyl group in the 9-position, and a carbamide as a result of an additional carbonyl group at the 8-position. The inclusion of theacrine may reduce tiredness and fatigue, and improve energy metabolism.

Where present, theacrine may be present in any suitable amount, such as an amount of from about 0.001% to about 30% by weight, or from about 0.01% to about 20% by weight of the oral product. The theacrine may preferably be present in an amount of from about 0.1% to about 15% by weight of the oral product. In some embodiments, theacrine is present in an amount of from about 0.05% to about 5% by weight, and preferably from about 0.05% to about 1% by weight of the oral product.

Taurine or 2-aminoethanesulfonic acid is an organic compound widely distributed in animal tissues. Taurine is known to play a role in energy metabolism. The combination of taurine with caffeine may lead to taurine neutralising some of the negative side effects that excess caffeine may cause (e.g. the jitters or increased heart rate). The inclusion of taurine may improve muscle strength and endurance, and performance during physical activity. The addition of taurine may also enhance energy metabolism.

The taurine may be present in any suitable amount, such as an amount of from about 0.001% to about 20% by weight of the oral product. In some embodiments, the taurine is present in an amount of from about 0.01% to about 10% by weight of the oral product. The taurine may preferably be present in an amount of from about 0.05% to about 5% by weight of the oral product, or from about 0.05% to about 1% by weight of the oral product.

In any of the above embodiments, the oral product may further comprise zinc or a salt thereof. In various embodiments, the zinc salt may be zinc gluconate. In various embodiments, the zinc or salt thereof, e.g. zinc gluconate, may be present in an amount of from about 0.001% to about 2% by weight of the oral product. In preferred embodiments, zinc or a salt thereof, e.g. zinc gluconate, may be present in an amount of about 0.005% to about 1% by weight of the oral product.

L-theanine is an amino acid analogue that is also referred to as L-γ-glutamylethylamide and *N*⁵-ethyl-L-glutamine. It is a water-soluble amino acid isolated from green tea (*Camellia sinensis*) that has anti-inflammatory activity, antioxidative properties and hepatoprotective effect. The inclusion of L-theanine may improve the effects of the oral product on the immune system. For example, the inclusion of L-theanine may help to regulate immune response, prevent disease, and reduce anti-oxidation stress. Studies have shown that L-theanine can enhance innate immune function by regulating the secretion of immune cytokines.

The L-theanine may be present in the oral product in any suitable amount, such as in an amount of from about 0.01% to about 15% by weight of the oral product, such as in an amount of from about 0.05% to about 10% by weight of the oral product. In preferred embodiments, the L-theanine is present in an amount of from about 0.1% to about 8% by weight of the oral product.

In some embodiments, the combination of active ingredients further comprises theobromine (also known as xantheose or 3,7-dihydro-3,7-dimethyl-1H-purine-2,6-dione). Theobromine is the principal alkanoid found in *Theobroma* cacao (the cacao plant). It has no significant stimulant effect on the human central nervous system and compared with caffeine, is weaker in both its inhibition of cyclic nucleotide phosphodiesterases and its antagonism of adenosine receptors. Structurally theobromine is similar to caffeine; it lacks the methyl groups in the 4- and 9-position, and the carbonyl group at the 8-position. The latter means the second ring is an imidazole.

Where present, theobromine may be present in any suitable amount, such as an amount of from about 0.001% to about 10% by weight, or from about 0.01% to about 5% by weight of the oral product. The theobromine may preferably be present in an amount of from about 0.1% to about 1% by weight of the oral product. In some embodiments, theobromine is present in an amount of from about 0.05% to about 5% by weight, and preferably from about 0.05% to about 1% by weight of the oral product.

### Other active ingredients

In some embodiments the oral product may comprise one or more additional active ingredients. The additional active ingredients may be any suitable active ingredient that has a biological response in a human or animal. The additional active ingredient may be naturally occurring or synthetically obtained. Non-limiting examples of additional active ingredients include those falling in the categories of botanical ingredients, amino acids, nicotine components, and/or nutraceutical, and medicinal ingredients (e.g., vitamins, such as A, B1, B2, B3, B5, B6, B7, B9, B12, and/or cannabinoids, such as tetrahydrocannabinol (THC) and cannabidiol (CBD)). The additional active ingredient may comprise for example taurine, theanine, vitamins such as B vitamins (e.g. B1, B2, B3, B5, B6, B7, B9 and/or B12), vitamin A, vitamin E, or vitamin K, melatonin, gamma-aminobutyric acid (GABA), cannabinoids, or constituents, derivatives, or combinations thereof.

In some embodiments, the one or more additional active ingredients is selected from a botanical ingredient (e.g., lavender, peppermint, chamomile, basil, rosemary, ginger, maca, lemon balm, and tisanes), an amino acid (e.g., taurine, phenylalanine, tyrosine, GABA, and tryptophan), a cannabinoid, and/or a nutraceutical, or medicinal ingredient (e.g., a vitamin, such as a B vitamin).

In some embodiments, the combination of active ingredients further comprises lemon balm. Lemon balm is also referred to as Melissa officinalis and is a perennial herbaceous plant in the mint family. Lemon balm contains a complex mixture of terpenes, terpenoids, flavonoids, polyphenols, and other molecules. The lemon balm may be in the form of leaves, stems or roots from the lemon balm plant. In some embodiments, the lemon balm is included in the form of lemon balm leaves, such as chopped, shredded or powdered lemon balm leaves. In some embodiments, the lemon balm is included in the form of a lemon balm extract, such as an aqueous extract of lemon balm. In some embodiments, the lemon balm is included in the form of lemon balm essential oil. Lemon balm has previously been found to exert antioxidant, anti-inflammatory and neuroprotective properties.

The lemon balm may be present in any suitable amount, such as in an amount of from about 0.01% to about 3% by weight of the oral product, such as in an amount of from about 0.05% by weight to about 3% by weight. In preferred embodiments, the lemon balm is present in an amount of from about 0.1% to about 2% by weight of the oral product.

In some embodiments, the combination of active ingredients further comprises chamomile. Chamomile (Matricaria recuita) is a flowering plant in the daisy (Asteraceae) family. Native to Europe and Western Asia, it is now found around the world. There are two different chamomile plants, German chamomile and Roman chamomile. German chamomile is generally considered the more potent variety and the type most widely used for medicinal purposes. Different classes of bioactive constituents are present in chamomile, such as flavanols (apigenin, luteolin, quercetin) coumarins and terpenoids. Research has shown chamomile to have benefits when it comes to boosting immunity or the immune system to help fight infections. Chamomile is also known to have anti-inflammatory and anti-oxidant effects.

The chamomile may be in the form of chamomile extract. The chamomile (e.g. chamomile extract) may be present in an amount of at least about 0.001% by weight, at least about 0.005% by weight or at least about 0.01% by weight of the oral product. In some embodiments, the chamomile (e.g. chamomile extract) is present in an amount of no greater than about 1% by weight, no greater than about 0.5% by weight, or no greater than about 0.1% by weight of the oral product.

The combination of active ingredients may further comprise vitamin D. Vitamin D is a group of fat-soluble secosteroids responsible for increasing intestinal absorption of calcium, magnesium, and phosphate. In humans the most important compounds in this group are vitamin D3 (cholecalciferol) and D2 (ergocalciferol). Vitamin D as used herein thus refers to D2, D3 or both. The structural difference between vitamin D2 and D3 is in the side chain, which contains a double bond, between carbons 22 and 23, and a methyl group on carbon 24 in vitamin D2. As well as calcium, magnesium, and phosphate absorption, vitamin D affects the immune system and vitamin D receptors (VDRs) are expressed in several white blood cells, including monocytes and activated T and B cells. In general, vitamin D functions to activate the innate and dampen the adaptive immune systems with antibacterial, antiviral and anti-inflammatory effects.

When present in the oral product, the vitamin D may be used in any suitable amount, such as in an amount of from about 0.000001 % to about 0.1% by weight of the oral product, such as in an amount of from about 0.0000025% to about 0.005% by weight of the oral product. In preferred embodiments, the vitamin D is present in an amount of from about 0.00001 % to about 0.001 % by weight of the oral product.

The combination of active ingredients may further comprise selenium. Selenium is a trace element that is nutritionally essential for humans as a component of multiple selenoproteins that play critical roles in reproduction, thyroid hormone metabolism, DNA synthesis, and protection from oxidative damage and infection. When present in the oral product, the selenium may be used in any suitable amount such as in an amount of from about 0.0000001 % by weight to about 0.001 % by weight of the oral product, such as in an amount of from about 0.0000005% by weight to about 0.0005% by weight of the oral product. In preferred embodiments, the selenium is present in an amount of from about 0.000001% by weight to about 0.0001% by weight of the oral product.

The combination of active ingredients may further comprise Echinacea. The Echinacea is either Echinacea purpurea, Echinacea angustifolia or Echinacea pallida and the upper parts (e.g. flowers, fruit, leaves) and/or roots of the plant may be utilized. Echinacea plants contain a variety of active compounds including caffeic acid, alkamides, phenolic acids, rosmarinic acid, polyacetylenes, flavonoids, and cichoric acid. Studies have linked Echinacea and its compounds to many health benefits including reduced inflammation, improved immunity and lower blood sugar levels. The Echinacea may be present in the form of an extract, chopped or shredded or powdered. Preferably, the Echinacea is included in the form of an extract.

The Echinacea may be present in the oral product in any suitable amount, such as in an amount of about 0.001% to about 20% by weight of the oral product, such as in an amount of from about 0.01% to about 15% by weight of the oral product. In preferred embodiments, the Echinacea is present in an amount of from about 0.05% to about 10% by weight of the oral product.

The combination of active ingredients may further comprise a vitamin selected from vitamin A, vitamin E and vitamin K, or mixtures thereof.

The combination of active ingredients may further comprise an amino acid, such as an amino acid selected from the group consisting of alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine, gamma-aminobutyric acid (GABA), taurine (2-aminoethanesulfonic acid), hydroxyproline, and beta-alanine. In some embodiments, the combination of active ingredients comprises GABA.

In some embodiments, the combination of active ingredients comprises a cannabinoid. The cannabinoid may be a derivative or extract of cannabis. Cannabinoids are a class of natural or synthetic chemical compounds which act on cannabinoid receptors (i.e., CB1 and CB2) in cells that repress neurotransmitter release in the brain. Cannabinoids are cyclic molecules exhibiting particular properties such as the ability to easily cross the blood-brain barrier. Cannabinoids may be naturally occurring (Phytocannabinoids) from plants such as cannabis, (endocannabinoids) from animals, or artificially manufactured (synthetic cannabinoids). Cannabis species express at least 85 different phytocannabinoids, and these may be divided into subclasses, including cannabigerols, cannabichromenes, cannabidiols, tetrahydrocannabinols, cannabinols and cannabinodiols, and other cannabinoids. In some embodiments" the cannabinoid is selected from the group consisting of cannabigerol (CBG), cannabichromene (CBC), cannabidiol (CBD), tetrahydrocannabinol (THC), cannabinol (CBN) and cannabinodiol (CBDL), cannabicyclol (CBL), cannabivarin (CBV), tetrahydrocannabivarin (THCV), cannabidivarin (CBDV), cannabichromevarin (CBCV), cannabigerovarin (CBGV), cannabigerol monomethyl ether (CBGM), cannabinerolic acid, cannabidiolic acid (CBDA), Cannabinol propyl variant (CBNV), cannabitriol (CBO), tetrahydrocannabimolic acid (THCA), tetrahydrocannabivarinic acid (THCV A), and mixtures thereof. In some embodiments, the cannabinoid is or comprises at least tetrahydrocannabinol (THC). In some embodiments, the cannabinoid is or comprises at least cannabidiol (CBD).

In some embodiments, the combination of active ingredients comprises a cannabinoid (such as cannabidiol) in an amount of about 0.001% to about 10% by weight, such as from about 0.01% to about 5% by weight, such as from about 0.1% to about 2.5% by weight, such as from 0.5% to about 1% by weight of the oral product.

In some embodiments, the combination of active ingredients comprises magnesium glycinate. Where present, magnesium glycinate may be included in an amount of from about 0.1% to about 10% by weight, such as from about 0.5% to about 5% by weight, or from about 0.5% to about 1% by weight of the oral product.

In some embodiments, the combination of active ingredients further comprises piperine. Piperine is an alkaloid found in fruits of the pepper species in the family *Piperaceae,* such as black pepper *(Piper nigrum),* long pepper *(Piper longum),* red long pepper *(Piper retrofactum)* and voatsiperifery pepper (*Piper borbonense*). Piperine may be beneficial to include in the oral product because of its ability to enhance the bioavailability of one or more compounds included in the oral product. The origin of piperine is not limited; in some embodiments it may be obtained from a black pepper extract.

Piperine has, for example, been found to enhance the bioavailability of one or more P-glycoprotein substrate compounds obtained from saffron or Ashwagandha. Specifically, piperine has been found to enhance the bioavailability of crocetin, crocin, withanolide A, and withanone. Each of these compounds are P-glycoprotein substrates and their permeation across the blood-brain barrier has been found to be enhanced by combining said compounds with piperine. This is unexpected because the enhancement was not observed with another P-gp inhibitor (quercetin), and/or with other P-glycoprotein substrate compounds, e.g. ursolic acid obtained from Holy Basil. In some embodiments the combination of active ingredients may therefore include ginseng in the form of Ashwagandha and further include piperine. In such embodiments, piperine may enhance/improve the bioavailability of withanolide A and/or withanone provided by the Ashwagandha in the oral product. The enhancement/improvement is relative to an oral product without piperine, and refers to the blood-brain barrier permeation of the P-glycoprotein substrates withanolide A and/or withanone.

In some embodiments the combination of active ingredients may alternatively or additionally include crocin and/or crocetin. Crocin and/or crocetin may be obtained from a saffron extract. Saffron is disclosed herein as a possible flavouring agent for the oral product. In a similar manner to above, piperine may enhance/improve the bioavailability of crocin and/or crocetin in the oral product relative to an oral product without piperine. The enhancement/improvement refers to the blood-brain barrier permeation of the P-glycoprotein substrates crocin and/or crocetin.

The benefit of such enhancement of the blood-brain barrier permeation is that it may allow lower amounts of the active ingredients to be included in the oral product, thereby mitigating or preventing any issues that may arise from higher amounts, e.g. issues of solubility, sensorials, compliance, safety etc. The inclusion of lower amounts of actives is especially beneficial when the oral product is prepared as a liquid in the form of a shot, i.e. a drink that may be consumed quickly. The liquid oral product may, for example, have a volume of from about 10 mL to about 100 mL, such as from about 25 mL to about 75 mL. In such embodiments, the liquid oral product may be considered to be a shot. For example, the volume of liquid may be about 60 mL or about 30 mL. Inclusion of lower amounts should not, however, compromise the effect of the oral product and piperine may therefore be used for this purpose.

In some embodiments, the oral product may comprise about 0.0001 wt% to about 0.1 wt% of piperine, based on the total weight of the oral product. In preferred embodiments the oral product may comprise about 0.0005 wt% to about 0.05 wt% of piperine, based on the total weight of the oral product.

In any of the embodiments defined herein, the term "comprises" may be replaced with the term "consists essentially of" or "consists of".

For the avoidance of doubt, combinations of the above end points are explicitly envisaged by the present disclosure. This applies to any of the ranges disclosed herein

It will be understood that all disclosure herein concerning the oral product applies to these combinations of active ingredients. The disclosure is not repeated for conciseness.

### Additives

Depending on the type of oral product, the product may include one or more additional components in addition to the combination of active ingredients. For example, the oral product may further comprise an additive selected from the group consisting of a flavouring agent, sweetener, buffering agent, acidifying agent, thickener, filler, binder, humectant, preservative, salt, colouring agent, oral care additive, distintegration aid, antioxidant, water or mixtures thereof. In some embodiments, the oral product may further comprise water and one or more additives selected from the group consisting of a flavouring agent, sweetener, acidifying agent, preservative, and mixtures thereof. In other embodiments, the oral product may further comprise one or more additives selected from the group consisting of a flavouring agent, sweetener, acidifying agent, thickener, filler, binder, humectant, preservative, and mixtures thereof.

The moisture content (e.g., water content) of the oral product, prior to use by a consumer of the product, may vary according to the desired properties.

For the liquid oral product, the water content may be at least about 50%, such as at least about 60%, such as at least about 75% by weight of the oral product. Preferably, the water content of the liquid oral product may be at least about 90% by weight of the oral product. Preferably, the water content of a liquid oral product may be from about 60% to about 99.5% by weight, such as from about 75% to about 99% by weight, such as from about 80% to about 98% by weight of the oral product.

Typically, for a solid oral product, prior to insertion into the mouth of the user, the water content is less than about 60% by weight, and generally is from about 1 to about 60% by weight, for example, from about 5 to about 55% by weight, about 10 to about 50% by weight, about 20 to about 45% by weight, or about 25 to about 40% by weight of the oral product; including water amounts of at least about 5% by weight, at least about 10% by weight, at least about 15% by weight, and at least about 20% by weight of the oral product.

### Flavouring Agent

As used herein, the term "flavouring agent" (or "flavour" or "flavourant") refers to materials which, where local regulations permit, may be used to create a desired taste, aroma or other somatosensorial sensation in a product for adult consumers. Examples of sensory characteristics that can be modified by the flavoring agent include taste, mouthfeel, moistness, coolness/heat, and/or fragrance/aroma. Flavouring agents may be natural or synthetic, and the character of the flavours imparted thereby may be described, without limitation, as fresh, sweet, herbal, confectionary, floral, fruity, or spicy.

The flavouring agent may be selected from the group consisting of naturally occurring flavour materials, botanicals, extracts of botanicals, synthetically obtained materials, or combinations thereof (e.g., tobacco, cannabis, licorice (liquorice), hydrangea, eugenol, Japanese white bark magnolia leaf, chamomile, fenugreek, clove, maple, matcha, menthol, Japanese mint, aniseed (anise), cinnamon, turmeric, Indian spices, Asian spices, herb, wintergreen, cherry, berry, red berry, cranberry, raspberry, strawberry, peach, apple, orange, mango, pineapple, clementine, lemon, lime, tropical fruit, papaya, rhubarb, grape, durian, dragon fruit, cucumber, blueberry, mulberry, citrus fruits, Drambuie, bourbon, scotch, whiskey, gin, tequila, rum, spearmint, peppermint, lavender, aloe vera, cardamom, coconut, celery, cascarilla, nutmeg, sandalwood, bergamot, geranium, khat, naswar, betel, shisha, pine, honey essence, rose oil, vanilla, lemon oil, orange oil, orange blossom, cherry blossom, cassia, caraway, cognac, jasmine, ylang-ylang, sage, fennel, wasabi, piment, ginger, coriander, coffee, hemp, a mint oil from any species of the genus Mentha, eucalyptus, star anise, cocoa, lemongrass, rooibos, flax, ginkgo biloba, hazel, hibiscus, laurel, mate, orange skin, rose, tea such as green tea or black tea, thyme, juniper, elderflower, basil, bay leaves, cumin, oregano, paprika, rosemary, saffron, lemon peel, mint, beefsteak plant, curcuma, cilantro, myrtle, cassis, valerian, pimento, mace, damien, marjoram, olive, lemon basil, chive, carvi, verbena, tarragon, limonene, thymol, camphene), flavour enhancers, bitterness receptor site blockers, sensorial receptor site activators or stimulators, sugars and/or sugar substitutes (e.g., honey, sucralose, acesulfame potassium, aspartame, saccharine, cyclamates, lactose, sucrose, glucose, fructose, sorbitol, or mannitol), and other additives such as charcoal, chlorophyll, minerals, botanicals, or breath freshening agents. They may be imitation, synthetic or natural ingredients or blends thereof. They may be in any suitable form, for example, liquid such as an oil, solid such as a powder, or gas.

In some embodiments, the flavouring agent comprises a natural flavoring, such as berry (e.g. raspberry, blueberry or strawberry), honey, citrus (such as lemon, bergamot, orange or lime), or other botanical material.

In some embodiments, the flavouring agent comprises menthol, spearmint and/or peppermint. In some embodiments, the flavouring agent comprises flavour components of cucumber, blueberry, citrus fruits and/or redberry. In some embodiments, the flavouring agent comprises eugenol. In some embodiments, the flavouring agent comprises flavour components extracted from tobacco. In some embodiments, the flavouring agent comprises flavour components extracted from cannabis.

In some embodiments, the flavouring agent may comprise a sensate, which is intended to achieve a somatosensorial sensation which are usually chemically induced and perceived by the stimulation of the fifth cranial nerve (trigeminal nerve), in addition to or in place of aroma or taste nerves, and these may include agents providing heating, cooling, tingling, numbing effect. A suitable heat effect agent may be, but is not limited to, vanillyl ethyl ether and a suitable cooling agent may be, but not limited to eucalyptol, WS-3.

In some embodiments, the flavouring agent may comprise a terpene. In some embodiments, the flavouring agent may comprise a monoterpene and/or a diterpene and/or a sesquiterpene. In some embodiments, the flavouring agent may comprise a monoterpene. In some embodiments, the terpene is selected from pinene (alpha and beta), geraniol, linalool, limonene, carvone, eucalyptol, menthone, iso-menthone, piperitone, myrcene, beta-bourbonene, germacrene, thymol, citral, eugenol, and mixtures thereof. In some embodiments,, the flavouring agent is selected from the group consisting of geraniol, citronellol, nerol, maltol, ethylmaltol, fenchol, homofuraneol, furaneol, norfuraneol, 1-octen-3-ol, borneol, linalool, farnesol, hydroxycitronellol, 3,7-dimethyloctanol, myrcenol, lavandulol, nerolidol, terpineol, alpha-terpineol, menthol, thymol, carvacrol, myrtenol, carveol, santalol, piperitol, perillyl alcohol, patchouli alcohol, hexanol, 1-hexanol, 3-cis-hexanol, cis-3-hexen-1-ol, phenylethanol, eugenol, sesamol, sotolone, maple furanone, methyl anthranilate, guaiacol, raspberry ketone, 2-methoxy-4-vinylphenol, 4-ethylguaiacol, benzylalcohol, homofuraneol, vanillin, ethylvanillin, and combinations thereof. In some embodiments, the flavouring agent is selected from the group consisting of geraniol, citronellol, nerol, maltol, ethylmaltol, fenchol, homofuraneol, furaneol, norfuraneol, 1-octen-3-ol, borneol, linalool, farnesol, hydroxycitronellol, 3,7-dimethyloctanol, myrcenol, lavandulol, nerolidol, terpineol, alpha-terpineol, menthol, thymol, carvacrol, myrtenol, carveol, santalol, piperitol, perillyl alcohol, patchouli alcohol, hexanol, 1-hexanol, 3-cis-hexanol, cis-3-hexen-1-ol, phenylethanol, eugenol, sesamol, sotolone, maple furanone, methyl anthranilate, guaiacol, raspberry ketone, 2-methoxy-4-vinylphenol, 4-ethylguaiacol, benzylalcohol, homofuraneol, and combinations thereof.

Where present, a flavouring agent may be included in the oral product in an amount up to about 10% by weight, such as up to about 5% by weight, such as up to about 1% by weight of the oral product. In some embodiments, a flavouring agent is present in an amount of from about 0.01% to about 5% by weight, such as in an amount of from about 0.1% to about 2.5% by weight of the oral product, preferably in an amount of from about 0.25% to about 1% by weight of the oral product.

### Humectants

In some embodiments, the oral product comprises at least one humectant. Examples of suitable humectants that may be included in the product include, but are not limited to, glycerin, 1,2-propanediol (propylene glycol), 1,3-propanediol, dipropylene glycol, sorbitol, xylitol, mannitol, and the like. In some embodiments, the humectant is or comprises glycerin. In some embodiments, the oral product comprises glycerin. In some embodiments, the humectant is or comprises propylene glycol. In some embodiments, the oral product comprises propylene glycol. The amount of humectant utilised in the oral product can vary, but may be up to about 5% by weight, and certain embodiments can be characterised by a humectant content of at least about 1% by weight, such as about 2 to about 5% by weight of the oral product. In some embodiments, the humectant (such as glycerin and/or propylene glycol) may be present in an amount of from about 0.01% to about 25% by weight of the oral product, such as from about 0.1% to about 20% by weight of the oral product, such as from about 0.5% to about 15% by weight of the oral product, such as from about 1% to about 10% by weight of the oral product, such as from about 5% to about 10% by weight of the oral product.

### Sweeteners

In order to improve the sensory properties of the oral product, one or more sweeteners may be added. The sweeteners can be any sweetener or combination of sweeteners, in natural or artificial form, or as a combination of natural and artificial sweeteners. Examples of natural sweeteners include fructose, sucrose, glucose, maltose, mannose, galactose, lactose, stevia, honey, and the like. Examples of artificial sweeteners include sucralose, isomaltulose, maltodextrin, saccharin, aspartame, acesulfame K, neotame and the like.

In some embodiments, the sweetener comprises one or more sugar alcohols. The sugar alcohol may include erythritol, arabitol, ribitol, isomalt, maltitol, dulcitol, iditol, mannitol, xylitol, lactitol, sorbitol, or combinations thereof. In some embodiments, the sweetener is selected from the group consisting of fructose, sucrose, glucose, maltose, mannose, galactose, lactose, stevia, honey, sucralose, isomaltulose, maltodextrin, saccharin, aspartame, acesulfame K, neotame, erythritol, arabitol, ribitol, isomalt, maltitol, dulcitol, iditol, mannitol, xylitol, lactitol, sorbitol, and mixtures thereof.

In some embodiments, the sweetener is selected from the group consisting of sucralose, acesulfame K, aspartame, maltodextrin, mannitol, sucrose, and mixtures thereof. Preferably, the sweetener may be sucralose and/or acesulfame K. When present in the oral product, the sweetener (such as sucralose and/or acesulfame K) may be present in an amount of from about 0.001% to about 5% by weight, such as from about 0.01% to about 3% by weight, preferably from about 0.01% to about 1% by weight of the oral product.

### Buffering agents

Non-limiting examples of suitable buffering agents that may be included in the oral product include alkali metals acetates, glycinates, phosphates, glycerophosphates, citrates, carbonates, hydrogen carbonates, borates, or mixtures thereof. In some embodiments, in which a buffering agent is present, the buffering agent is selected from the group consisting of sodium carbonate, sodium bicarbonate, sodium phosphate, ammonium phosphate, dicalcium phosphate, tricalcium phosphate, and mixtures thereof. In some embodiments, the buffering agent is sodium bicarbonate and/or sodium carbonate. Where present, the buffering agent (e.g. sodium bicarbonate and/or sodium carbonate) may be included in an amount less than about 5% based on the weight of the oral product; for example, from about 0.5% to about 5%, such as, e.g., from about 0.75% to about 4%, from about 0.75% to about 3%, or from about 1% to about 2% by weight, based on the total weight of the oral product.

### Organic acid

In some embodiments, the product comprises an organic acid. As used herein, the term "organic acid" refers to an organic (i.e., carbon-based) compound that is characterised by acidic properties. Typically, organic acids are relatively weak acids (i.e., they do not dissociate completely in the presence of water), such as carboxylic acids (-CO2H) or sulfonic acids (-SO2OH). As used herein, reference to organic acid means an organic acid that is intentionally added. In this regard, an organic acid may be intentionally added as a specific mixture ingredient as opposed to merely being inherently present as a component of another mixture ingredient (e.g., the small amount of organic acid which may inherently be present in a mixture ingredient such as a tobacco material). In some embodiments, the one or more organic acids are added neat (i.e., in their free acid, native solid or liquid form) or as a solution in, e.g. water. In some embodiments, the one or more organic acids are added in the form of a salt.

In some embodiments, the organic acid is a carboxylic acid or a sulfonic acid. The carboxylic acid or sulfonic acid functional group may be attached to any alkyl, cycloalkyl, heterocycloalkyl, aryl, or heteroaryl group having, for example, from one to twenty carbon atoms (C1-C20). In some embodiments, the organic acid is an alkyl, cycloalkyl, heterocycloalkyl, aryl, or heteroaryl carboxylic or sulfonic acid.

In some embodiments, the organic acid is an alkyl carboxylic acid. Non-limiting examples of alkyl carboxylic acids include formic acid, acetic acid, propionic acid, octanoic acid, nonanoic acid, decanoic acid, undecanoic acid, dodecanoic acid, stearic acid, oleic acid, linoleic acid, linolenic acid, and the like. In some embodiments, the organic acid is an alkyl sulfonic acid. Non-limiting examples of alkyl sulfonic acids include propanesulfonic acid and octanesulfonic acid.

In some embodiments, the alkyl carboxylic or sulfonic acid is substituted with one or more hydroxyl groups. Non-limiting examples include glycolic acid, 4-hydroxybutyric acid, and lactic acid.

In some embodiments, an organic acid may include more than one carboxylic acid group or more than one sulfonic acid group (e.g., two, three, or more carboxylic acid groups). Non-limiting examples include oxalic acid, fumaric acid, maleic acid, and glutaric acid. In organic acids containing multiple carboxylic acids (e.g., from two to four carboxylic acid groups), one or more of the carboxylic acid groups may be esterified. Non-limiting examples include succinic acid monoethyl ester, monomethyl fumarate, monomethyl or dimethyl citrate, and the like.

In some embodiments, the organic acid may include more than one carboxylic acid group and one or more hydroxyl groups. Non-limiting examples of such acids include tartaric acid, citric acid, and the like. In some preferred embodiments, the organic acid is citric acid, sodium citrate, calcium citrate, or a combination thereof.

In some embodiments, the organic acid is an aryl carboxylic acid or an aryl sulfonic acid. Non-limiting examples of aryl carboxylic and sulfonic acids include benzoic acid, toluic acids, salicylic acid, benzenesulfonic acid, and p-toluenesulfonic acid.

Additional non-limiting examples of suitable organic acids include 2,2-dichloroacetic acid, 2-hydroxyethanesulfonic acid, 2-oxoglutaric acid, 4-acetamidobenzoic acid, 4-aminosalicylic acid, acetic acid, adipic acid, ascorbic acid (L), aspartic acid (L), camphoric acid (+), camphor-10-sulfonic acid (+), capric acid, caproic acid, caprylic acid, cinnamic acid, cyclamic acid, decanoic acid, dodecylsulfuric acid, ethane-1,2-disulfonic acid, ethanesulfonic acid, formic acid, fumaric acid, galactaric acid, gentisic acid, glucoheptonic acid, gluconic acid, glucuronic acid, glutamic acid, glycerophosphoric acid, glycolic acid, hippuric acid, isobutyric acid, lactobionic acid, lauric acid, malonic acid, mandelic acid, methanesulfonic acid, naphthalene-1,5-disulfonic acid, naphthalene-2-sulfonic acid, oleic acid, palmitic acid, pamoic acid, pyroglutamic acid, sebacic acid, stearic acid, and undecylenic acid.

Preferably, the organic acid is selected from the group consisting of citric acid, malic acid, lactic acid, benzoic acid, tartaric acid, and mixtures thereof. In some preferred embodiments, the organic acid is or comprises citric acid or a salt thereof.

In some embodiments, the product comprises an alkali metal salt of an organic acid. For example, at least a portion of the organic acid may be present in the product in the form of an alkali metal salt. Suitable alkali metal salts include lithium, sodium, and potassium. In some embodiments, the alkali metal is sodium or potassium. In some embodiments, the alkali metal is sodium. In some embodiments, the product comprises an organic acid and a sodium salt of the organic acid. In some embodiments, the organic acid is or comprises sodium citrate, such as trisodium citrate.

The amount of organic acid present in the product may vary. The oral product may comprise from about 0.01% to about 10% by weight of organic acid, present as one or more organic acids, based on the total weight of the oral product. In some embodiments, the oral product comprises at least about 0.01%, at least about 0.1 %, about 0.2%, about 0.3%, about 0.4%, about 0.5%, about 0.6%, about 0.7%, about 0.8%, about 0.9%, about 1%, about 2%, about 3%, about 4%, about 5%, about 6%, about 7%, about 8%, about 9%, or at least about 10% organic acid by weight, based on the total weight of the oral product. In some preferred embodiments, the oral product comprises from about 0.01% to about 5% by weight of organic acid based on the weight of the oral product. For example, the oral product comprises an organic acid in an amount of from about 0.1% to about 2.5% by weight of the oral product. In the case where a salt of an organic acid is added (e.g., citric acid anhydrate), the percent by weight is calculated based on the weight of the free acid, not including any counter-ion which may be present.

In certain embodiments the organic acid inclusion is sufficient to provide a product pH of from about 4.0 to about 9.0, such as from about 4.5 to about 7.0, or from about 5.5 to about 7.0, from about 4.0 to about 5.5, or from about 7.0 to about 9.0. In some embodiments, the organic acid inclusion is sufficient to provide a product pH of from about 4.5 to about 6.5, for example, from about 4.5, about 5.0, or about 5.5, to about 6.0, or about 6.5. In some embodiments,, the organic acid is provided in a quantity sufficient to provide a pH of the product of from about 5.5 to about 6.5, for example, from about 5.5, about 5.6, about 5.7, about 5.8, about 5.9, or about 6.0, to about 6.1, about 6.2, about 6.3, about 6.4, or about 6.5.

In other embodiments, a mineral acid (e.g., hydrochloric acid, sulfuric acid, phosphoric acid, or the like) is added to adjust the pH of the product to the desired value.

Organic acids (e.g., citric acid) may be added neat (i.e., as a solid) or in solution, for example, in water. In some embodiments, the organic acid is added as a 50% aqueous solution.

### Salts

The oral product may further comprise a salt. This may be included in an amount sufficient to provide desired sensory attributes to the product. Non-limiting examples of suitable salts include sodium chloride, potassium chloride, ammonium chloride, flour salt, sodium acetate, sodium citrate, and the like. The salt may be included in any suitable amount, such as at least about 0.5% by weight, such as at least about 1% by weight, such as at least about 1.5% by weight of the oral product. In some embodiments, the oral product may comprise salt in an amount of from about 0.5% to about 10% by weight, such as from about 1% to about 7.5% by weight, such as from about 1.5% to about 5% by weight, based on the total weight of the oral product.

### Colouring agents

A colouring agent may be employed in amounts sufficient to provide the desired physical attributes to the product. Examples of colouring agents include various dyes and pigments, such as caramel colouring and titanium dioxide. Natural colouring agents such as curcumin, beet juice extract, spirulina; also a variety of synthetic pigments may also be used. The amount of colorant utilised in the oral product can vary, but when present is typically up to about 3% by weight, such as from about 0.1 %, about 0.5%, or about 1%, to about 3% by weight, based on the total weight of the oral product.

### Filler or Bulking Agents

Depending on the form of the product, the oral product may comprise a filler or bulking agent. Fillers or bulking agents, for example, may fulfil multiple functions, such as enhancing certain organoleptic properties such as texture and mouthfeel, enhancing cohesiveness or compressibility of the product, and the like.

In some embodiments, the filler is a porous particulate material and is cellulose-based. For example, the filler or bulking agent may be a non-tobacco plant material or derivative thereof, including cellulose materials derived from such sources. Examples of cellulosic non-tobacco plant material include cereal grains (e.g., maize, oat, barley, rye, buckwheat, and the like), sugar beet (e.g., FIBREX^{®} brand filler available from International Fiber Corporation), bran fiber, and mixtures thereof. In some embodiments, the filler is a cellulose material selected from the group consisting of maize fiber, oat fiber, barley fiber, rye fiber, buckwheat fiber, sugar beet fiber, bran fiber, bamboo fiber, wood pulp fiber, cotton fiber, citrus pulp fiber, grass fiber, willow fiber, poplar fiber, cocoa fiber, derivatives thereof, and combinations thereof. In some embodiments, the filler is a cellulose material selected from the group consisting of sugar beet fiber, wood pulp fiber, bamboo fiber, derivatives thereof, and combinations thereof.

In some embodiments, the filler is derived from wood pulp fiber. One particularly suitable filler for use in the products described herein is microcrystalline cellulose ("MCC"). The MCC may be synthetic or semi-synthetic, or it may be obtained entirely from natural celluloses. The MCC may be selected from the group consisting of AVICEL^{®} grades PH-100, PH-101, PH-102, PH-103, PH-105, PH-112, PH-113, PH-200, PH-300, PH-301, PH-302, VIVACEL^{®} grades 101, 102, 12, 20 and EMOCEL^{®} grades 50M and 90M, and the like, and mixtures thereof.

In some embodiments, the filler is a non-tobacco plant material or a derivative thereof. Non-limiting examples of derivatives of non-tobacco plant material include starches (e.g., from potato, wheat, rice, corn), natural cellulose, and modified cellulosic materials. Additional examples of potential fillers include maltodextrin, dextrose, calcium carbonate, calcium phosphate, lactose, mannitol, xylitol, and sorbitol. Combinations of these fillers can also be used.

"Starch" as used herein may refer to pure starch from any source, modified starch, or starch derivatives. Starch is present, typically in granular form, in almost all green plants and in various types of plant tissues and organs (e.g., seeds, leaves, rhizomes, roots, tubers, shoots, fruits, grains, and stems). Starch can vary in composition, as well as in granular shape and size. Often, starch from different sources has different chemical and physical characteristics. A specific starch can be selected for inclusion in the product based on the ability of the starch material to impart a specific organoleptic property to product. Starches derived from various sources can be used. For example, major sources of starch include cereal grains (e.g., rice, wheat, and maize) and root vegetables (e.g., potatoes and cassava). Other examples of sources of starch include acorns, arrowroot, arracacha, bananas, barley, beans (e.g., favas, lentils, mung beans, peas, chickpeas), breadfruit, buckwheat, canna, chestnuts, colacasia, katakuri, kudzu, malanga, millet, oats, oca, Polynesian arrowroot, sago, sorghum, sweet potato, quinoa, rye, tapioca, taro, tobacco, water chestnuts, and yams. Certain starches are modified starches. A modified starch has undergone one or more structural modifications, often designed to alter its high heat properties. Some starches have been developed by genetic modifications, and are considered to be "modified" starches. Other starches are obtained and subsequently modified. For example, modified starches can be starches that have been subjected to chemical reactions, such as esterification, etherification, oxidation, depolymerization (thinning) by acid catalysis or oxidation in the presence of base, bleaching, transglycosylation and depolymerization (e.g., dextrinization in the presence of a catalyst), cross-linking, enzyme treatment, acetylation, hydroxypropylation, and/or partial hydrolysis. Other starches are modified by heat treatments, such as pregelatinization, dextrinization, and/or cold water swelling processes. Certain modified starches include monostarch phosphate, distarch glycerol, distarch phosphate esterified with sodium trimetaphosphate, phosphate distarch phosphate, acetylated distarch phosphate, starch acetate esterified with acetic anhydride, starch acetate esterified with vinyl acetate, acetylated distarch adipate, acetylated distarch glycerol, hydroxypropyl starch, hydroxypropyl distarch glycerol, and starch sodium octenyl succinate.

Other suitable fillers or bulking agents include sugar alcohols. Sugar alcohols are polyols derived from monosaccharides or disaccharides that have a partially or fully hydrogenated form. Sugar alcohols have, for example, about 4 to about 20 carbon atoms and include erythritol, arabitol, ribitol, isomalt, maltitol, dulcitol, iditol, mannitol, xylitol, lactitol, sorbitol, and combinations thereof. In some embodiments, where present, the filler may be selected from the group consisting of isomalt, maltitol and mixtures thereof.

### Binding agent

In some embodiments, the oral product may further comprise at least one binder. A binder (or combination of binders) may be employed in the product in certain embodiments, in amounts sufficient to provide the desired physical attributes and physical integrity to the product. Binders can be organic or inorganic, or a combination thereof. Representative binders include cellulose derivatives, povidone, sodium alginate, starch-based binders, pectin, carrageenan, pullulan, zein, and the like, and combinations thereof. The amount of binder utilised in the product can vary, but may be up to about 30% by weight, and certain embodiments are characterised by a binder content of at least about 0.1% by weight, such as from about 1% to about 30% by weight, or about 1% to about 10% by weight, based on the total weight of the oral product.

In some embodiments, the binder comprises pectin. Pectins are natural polymers related to carbohydrates and which are acidic heteropolysaccharides (polysaccharides comprising multiple monosaccharide units). As opposed to carbohydrates, the pectin C-6 position contains a carboxylic acid (or corresponding methyl ester or carboxamide) group instead of a hydroxymethyl group. The principal subunit is known as galacturonic acid, which can be copolymerised with L-rhamnose. Other sugars are featured as side-chain substituents. Pectin acts as a thickening and gelling agent. Pectin isolated from sources such as apple pomace, citrus peels, sugarbeet waste from sugar manufacturing, sunflower heads discarded from seed harvesting, mango waste, and other commercially available pectins may be used. In combination with certain sugars, under acidic conditions (e.g., a pH of from about 2.5 to about 5), or in the presence of a gelation agent (calcium or other divalent alkaline earth elements), pectins may provide a gel or gum consistency to products as disclosed herein. In some embodiments, the binder comprises low methoxy pectin. Suitable low methoxy pectins include, for example, "GENU^{®} pectin type LM-104 AS", available from CP Kelco, Atlanta, GA, USA In some embodiments, the binder comprises low methoxy pectin in combination with a gelation agent. In some embodiments, the gelation agent comprises calcium ions, such as, but not limited to, calcium diphosphate. In some embodiments, the binder comprises a high methoxy pectin in combination with an organic acid, described herein below. In some embodiments, the binder comprises a high methoxy pectin in combination with citric acid.

When present, a pectin binder is typically present in an amount of up to about 3% by weight, for example, from about 0.1, about 0.2, about 0.3, about 0.4, about 0.5, about 0.6, about 0.7, about 0.8, about 0.9, or about 1, to about 1.1, about 1.2, about 1.3, about 1.4, about 1.5, about 1.6, about 1.7, about 1.8. about 1.9, about 2, about 2.1, about 2.2, about 2.3. about 2.4, about 2.5, about 2.6, about 2.7, about 2.8, about 2.9, or about 3% by weight, based on the total weight of the oral product.

In some embodiments, the binder comprises a cellulose derivative. In certain embodiments, the cellulose derivative is a cellulose ether (including carboxyalkyl ethers), meaning a cellulose polymer with the hydrogen of one or more hydroxyl groups in the cellulose structure replaced with an alkyl, hydroxyalkyl, or aryl group. Non-limiting examples of such cellulose derivatives include methylcellulose, hydroxypropylcellulose ("HPC"), hydroxypropylmethylcellulose ("HPMC"), hydroxyethyl cellulose, and carboxymethylcellulose ("CMC"). In some embodiments, the cellulose derivative is or comprises HPC. In some embodiments, the cellulose derivative is a combination of HPC and HPMC. In some embodiments, the oral product comprises from about 1% to about 10% of the cellulose derivative (such as HPC) by weight of the oral product, with certain embodiments comprising from about 1% to about 5% by weight of cellulose derivative (such as HPC), based on the weight of the product.

In certain embodiments, the binder includes a gum, for example, a natural gum. As used herein, a natural gum refers to polysaccharide materials of natural origin that have binding properties, and which are also useful as a thickening or gelling agents. Representative natural gums derived from plants, which are typically water soluble to some degree, include xanthan gum, guar gum, gum arabic, ghatti gum, gum tragacanth, karaya gum, locust bean gum, gellan gum, and combinations thereof. When present, natural gum binder materials may be present in an amount of up to about 5% by weight, for example, from about 0.1, about 0.2, about 0.3, about 0.4, about 0.5, about 0.6, about 0.7, about 0.8, about 0.9, or about 1%, to about 2, about 3, about 4, or about 5% by weight, based on the total weight of the product.

### Thickeners

The oral product may comprise a thickening agent in some embodiments. Suitable thickening agents may include a hydrocolloid, such as xanthan gum, guar gum, konjac gum, gum tragacanth and gum Arabic. For example, xanthan gum is understood to be a thickening agent that thickens compositions when added during cold processing (i.e. when heat is not applied). Where present, a thickening agent (e.g. xanthan gum) may be included in an amount of from about 0.001% to about 5% by weight, and preferably from about 0.01% to about 1% by weight of the oral product.

### Other additives

Other ingredients such as preservatives (e.g., potassium sorbate), disintegration aids (e.g., croscarmellose sodium, crospovidone, sodium starch glycolate, pregelatinized corn starch, and the like), and/or antioxidants can also be used. Typically, such ingredients, where used, are used in amounts of up to about 10% by weight, for example at least about 0.1% by weight, such as about 0.5 to about 10% by weight of the oral product. A disintegration aid may be employed in an amount sufficient to provide control of desired physical attributes of the oral product such as, for example, by providing loss of physical integrity and dispersion of the various component materials upon contact of the formulation with water (e.g., by undergoing swelling upon contact with water).

Examples of further types of additives include zinc or magnesium salts selected to be relatively water soluble for compositions with greater water solubility (e.g., magnesium or zinc gluconate) or selected to be relatively water insoluble for compositions with reduced water solubility (e.g., magnesium or zinc oxide), or combinations thereof. See, for example, those representative components, combination of components, relative amounts of those components, and manners and methods for employing those components, set forth in US Pat. No. 9,237,769 to Mua et al., US Pat. No. 7,861,728 to Holton, Jr. et al., US Pat. App. Pub. No. 2010/0291245 to Gao et al., and US Pat. App. Pub. No 2007/0062549 to Holton, Jr. et al., each of which is incorporated herein by reference. Typical inclusion ranges for such additional additives can vary depending on the nature and function of the additive and the intended effect on the final product, with an example range of up to about 10% by weight, based on total weight of the oral product (e.g., about 0.1 to about 5% by weight).

In some embodiments, the oral product comprises a magnesium salt. A non-limiting example of a suitable magnesium salt is magnesium gluconate. In some embodiments, the oral product comprises magnesium in an amount by weight from about 0.1% to about 2%, or from about 0.2 to about 1%, based on elemental magnesium.

The aforementioned additives can be employed together (e.g., as additive formulations) or separately (e.g., individual additive components can be added at different stages involved in the preparation of the final product).

### Oral Product

The products or compositions as described herein are configured for oral use. The term "configured for oral use" as used herein means that the product is provided in a form such that during use, saliva in the mouth of the user causes one or more of the components of the product (e.g., active ingredients) to pass into the mouth of the user. In certain embodiments, the product is adapted to deliver active ingredients and optionally flavouring agent to a user through mucous membranes in the user's mouth, the user's digestive system, or both.

The combination of active ingredients may be present in an amount of from about 0.01% to about 20% by weight. For example, the combination of active ingredients may be present in an amount of from about 0.05% to about 15% by weight, such as from about 0.1% to about 10% by weight. Preferably, the combination of active ingredients is present in an amount of from about 0.1% to about 15% by weight of the oral product, more preferably from about 1% to about 10% by weight of the oral product.

The liquid oral product may be in the form of a shot; i.e. a drink that may be consumed quickly, e.g. in a single gulp or a couple of gulps. Formulating the oral product in the form of a shot may be advantageous as it provides a convenient and easy-to-use mode of administration that does not take up much room during storage (e.g. in a fridge or cupboard) and is easily portable by the user so can be taken on the go. Shots may also provide an efficient delivery vehicle for actives, whereby a level of active sufficient to provide good efficacy can be delivered to the user without the need to consume large volumes of liquid. Additionally, the shots may be formulated to have a pleasant taste. Drinking a shot may be considered to be more palatable and easier to a user than swallowing a tablet, for example.

The liquid oral product may also be in the form of a larger beverage that is consumed more slowly over several gulps. The liquid oral product may have a volume of from about 1 mL to about 250 mL, such as from about 1 mL to about 200 mL. The liquid oral product may have a volume of from about 100 mL to about 250 mL and be provided in a package such as a carton, cup, can or bottle of liquid.

The liquid oral product may have a volume of from about 10 mL to about 100 mL, such as from about 25 mL to about 75 mL. In such embodiments, the liquid oral product may be considered to be a shot. For example, the volume of liquid may be about 60 mL or about 30 mL.

As noted above, the oral product may further comprise water in an amount of from about 50% to about 99.9% by weight of the oral product. In some embodiments, the oral product comprises water in an amount of from about 75% to about 99.5% by weight, such as from about 80% to about 99% by weight, such as from about 90% to about 97.5% by weight of the oral product. In some embodiments, the oral product comprises water in an amount of from about 90% to about 99.5% by weight of the oral product, and may be from about 95% to about 99% by weight of the oral product. Where present, the water may include tap water, rain water, mineralised water, or distilled water.

The liquid oral product may comprise the combination of active ingredients in an amount of from about 0.1 % to about 15% by weight of the oral product, and water in an amount of from about 85% to about 99.9% by weight of the oral product. The liquid oral product may comprise the combination of active ingredients in an amount of from about 0.5% to about 12% by weight of the oral product, and water in an amount of from about 88% to about 99.5% by weight of the oral product.

In some embodiments, there is provided a package in the form of a bottle or can that contains the liquid oral product. The package may be a bottle containing the desired volume of liquid oral product.

### Solid oral dosage forms

In some embodiments, the oral product is a solid oral dosage form. A solid oral product as described herein may take various forms, including pastilles and chews. As used herein, the term "solid" means that the products can substantially sustain their physical shape when unsupported by external means, e.g. packaging etc. Thus, they are considered to be solid, solid-like, in solid form or in solid-like form at room temperature. For the avoidance of doubt the solid product remains substantially solid at up to 30°C. In some embodiments, the oral product is in solid form, such as in the form of chews or pastilles. In some embodiments, the oral product is a chew or pastille.

The oral products as disclosed herein can be formed into a variety of shapes, including pills, tablets, spheres, strips, films, sheets, coins, cubes, beads, ovoids, obloids, cylinders, bean-shaped, sticks, or rods. Cross-sectional shapes of the product can vary, and example cross-sectional shapes include circles, squares, ovals, rectangles, and the like. Such shapes can be formed in a variety of manners using equipment such as moving belts, nips, extruders, granulation devices, compaction devices, and the like.

In some embodiments, the solid oral product is in a form selected from the group consisting of a chew, or a pastille. In other embodiments, the oral product is in solid form, such as in the form of loose moist snuff, loose dry snuff, chewing tobacco-type form, pelletized pieces, extruded or formed strips, pieces, rods, or sticks, finely divided ground powders, finely divided or milled agglomerates of powdered pieces and components, flake-like pieces, molded processed pieces, gums, films, readily water-dissolvable or water-dispersible films or strips, capsule-like materials, tablets, lozenges. In some embodiments, the oral product is a tablet or lozenge. In some embodiments, the oral product may be a chewing gum product. In some embodiments, the oral product is in the form of moist snuff or snus, which may or may not contain tobacco.

### Chews

In some embodiments, the product can be chewable, meaning the product has a mild resilience or "bounce" upon chewing, and possesses a desirable degree of malleability. A product in chewable form may be entirely dissolving, or may be in the form of a non-dissolving gum in which only certain components (e.g., active ingredients, flavour, sweetener) dissolve, leaving behind a non-dissolving matrix.

As described herein a "chew" is a confectionary-type product that may be chewed by the user before dissolving in the mouth (i.e. a dissolvable chew) or may be a chewing gum. Preferably, the "chew" dissolves fully in the mouth of the user. The product may be dissolvable. As used herein, the terms "dissolve," "dissolving," and "dissolvable" refer to products having aqueous-soluble components that interact with moisture in the oral cavity and enter into solution, thereby causing gradual consumption of the product. According to one aspect, the dissolvable product is capable of lasting in the user's mouth for a given period of time until it completely dissolves. Dissolution rates can vary over a wide range, from about 1 minute or less to about 60 minutes. For example, fast release products typically dissolve and/or release the desired component(s) (e.g., active ingredient, flavour, and the like) in about 2 minutes or less, often about 1 minute or less (e.g., about 50 seconds or less, about 40 seconds or less, about 30 seconds or less, or about 20 seconds or less). Dissolution can occur by any means, such as melting, mechanical disruption (e.g., chewing), enzymatic or other chemical degradation, or by disruption of the interaction between the components of the product.

In other embodiments, the products do not dissolve during the product's residence in the user's mouth.

Chewable embodiments generally include a binding agent, such as a natural gum, pectin, agar, carrageenan, starch, or a combination thereof. In some embodiments, the binding agent comprises or is pectin.

In some embodiments, the chewable product (i.e. "chew") comprises the combination of active ingredients, at least one bulking agent selected from the group consisting of a sugar alcohol, or at least one a sugar, or a combination of at least one sugar alcohol and at least one sugar, and at least one binding agent. In some embodiments, the chewable product (i.e. "chew") comprises the combination of active ingredients in an amount of from about 0.1% to about 10% by weight of the oral product, at least one bulking agent selected from the group consisting of a sugar alcohol, or at least one a sugar, or a combination of at least one sugar alcohol and at least one sugar, and at least one binding agent. The chewable product or chew may also optionally include a sweetening agent and/or a flavouring agent.

Representative chew compositions and products may incorporate from about 0.1% to about 20% by weight of the combination of active ingredients, from about 0.1% to about 10% of a binding agent (e.g. pectin, agar, carrageenan, starch, or a combination thereof), and at least about 30% by weight of at least one bulking agent selected from the group consisting of a sugar alcohol, or at least one a sugar, or a combination of at least one sugar alcohol and at least one sugar, based on the total weight of the oral product. Optionally, the oral product may further comprise about 0.01 to about 2% by weight of a sweetening agent, and/or from about 0.1% to about 5% by weight of at least one flavoring agent, based on the total weight of the oral product. The particular percentages and choice of ingredients will vary depending upon the desired flavor, texture, and other characteristics. For example, the combination of active ingredients may be present in a chew in an amount of from about 0.1% to about 10% by weight of the oral product.

In some embodiments, the oral chew product comprises pectin and an organic acid, along with one or more sugar alcohols in an amount of at least 30% by weight of the oral product.

In some embodiments, the oral chew product comprises at least one bulking agent selected from the group consisting of a sugar alcohol, or a sugar, or a combination of at least one sugar alcohol and at least one sugar. In some embodiments, the at least one bulking agent is selected from a sugar alcohol, or a sugar. In some embodiments, the at least one bulking agent is selected from sugar alcohol(s). In some embodiments, the at least one bulking agent is selected from sugar(s). In some embodiments, the at least one bulking agent is selected from a combination of at least one sugar alcohol and at least one sugar.

The total amount of bulking agent(s) (preferably sugar alcohol) may be at least about 30% by weight, such as at least about 40% by weight, such as at least about 45% by weight of the oral product. In some preferred embodiments, the total amount of bulking agent(s) (preferably sugar alcohol) may be at least about 50% by weight of the oral product.

In some embodiments, the total amount of sugar alcohol is present in an amount of from about 30% to about 99% by weight, such as from about 40% to about 99% by weight, such as from about 45% to about 99% by weight, such as from about 50% to about 99% by weight, such as from about 60% to about 95% by weight, and preferably from about 70% to about 90% by weight of the oral product. The sugar alcohol may preferably be present in an amount of from about 80% to about 95% by weight of the oral product. In some preferred embodiments, the total amount of bulking agent(s) (preferably sugar alcohol) is present in an amount of from about 40% to about 60% by weight of the oral product. Alternatively, in some preferred embodiments, the total amount of sugar alcohol is from about 50% to about 90% by weight of the oral product. In such embodiments, the bulking agent may consist of sugar alcohol(s).

The sugar alcohol may be selected from the group consisting of isomalt, erythritol, sorbitol, arabitol, ribitol, maltitol, dulcitol, iditol, mannitol, xylitol, lactitol, or mixtures thereof. In some embodiments, the sugar alcohol is selected from the group consisting of maltitol, sorbitol, erythritol, mannitol, lactitol, xylitol, isomalt, and mixtures thereof. In some embodiments, the sugar alcohol is selected from the group consisting of maltitol, isomalt, and mixtures thereof. In some embodiments, the sugar alcohol is or comprises isomalt. In some embodiments, the sugar alcohol is or comprises maltitol. In some embodiments, the sugar alcohol comprises a combination of isomalt and maltitol.

In some embodiments, a sugar substitute may be an alternative to sugar alcohols, or used in combination with one or more sugar alcohols. Suitable sugar substitutes include allulose, soluble tapioca fiber, inulin, and combinations thereof. In some embodiments, sugar is included as an alternative to sugar alcohols, or is used in combination with one or more sugar alcohols. Where present, sugar may be included in the form of glucose, fructose, galactose, sucrose, or mixtures thereof. In some embodiments, the oral chew product comprises sugar (e.g. sucrose) in combination with at least one sugar alcohol (e.g. maltitol and/or isomalt). The sugar may be present in any suitable amount, such as from 10% to about 50%, such as from about 20% to about 40% by weight of the oral product.

In some embodiments the bulking agents consists of at least one sugar alcohol selected from maltitol and/or isomalt and sucrose. In some embodiments the at least one bulking agent consists of maltitol and sucrose. In some embodiments the at least one bulking agent consists of isomalt and sucrose. In some embodiments, the bulking agent comprises a combination of maltitol and sugar. The maltitol may be present in an amount of from about 20% to about 50%, and the sugar may be present in an amount of from about 10% to about 50% by weight of the oral product. In some embodiments, the bulking agent comprises a combination of isomalt and sugar. The isomalt may be present in an amount of from about 20% to about 50%, and the sugar may be present in an amount of from about 10% to about 50% by weight of the oral product.

In some embodiments, the binding agent is selected from the group consisting of pectin, agar, carrageenan, starch, and mixtures thereof. In some preferred embodiments, the binding agent is or comprises pectin. In some embodiments, the binding agent (e.g. pectin) is present in an amount of from about 0.1% to about 10% by weight of the oral product. Preferably, the binding agent (e.g. pectin) is present in an amount of from about 1% to about 5% by weight of the oral product.

The oral product may also comprise an organic acid, a gelation agent or both to crosslink the pectin.

In some embodiments, the oral chew product comprises an acidifying agent. The acidifying agent may be an organic or inorganic acid. The organic acid may be any suitable organic acid. Suitable organic acids are as described hereinabove in the section titled "Additives". For example, the organic acid may be selected from the group consisting of citric acid, malic acid, lactic acid, benzoic acid, tartaric acid, and mixtures or salts thereof. The organic acid may, for example, include a combination of citric acid and a salt of citric acid (e.g. a sodium salt).

Oral products of the present disclosure in the form of a chew may contain various amounts of water. For example, the water content of the chew product may be provided within a specified range so as to dictate the final form of the product. The water content of the chew products described herein, prior to use by a consumer of the product, may vary within such ranges according to the desired properties and characteristics, in addition to dictating the final form of the product. For example, chew-type products may possess a water content in the range of from about 1% to about 20%, such as from about 1% to about 10% by weight of the oral product.

The oral product in the form of a chew may comprise:
(a) the combination of active ingredients as described herein in an amount of from about 0.1% to about 10% by weight of the oral product;
(b) at least one bulking agent selected from the group consisting of a sugar alcohol, or a sugar, or a combination of at least one sugar alcohol and at least one sugar, wherein the total amount of sugar alcohol and/or sugar is from about 40% to about 99% by weight of the oral product;
(c) at least one binding agent in an amount of from about 0.1% to about 10% by weight of the oral product;
optionally (d) a flavouring agent, an organic acid and/or a sweetening agent.

The oral chew product may have a weight of from about 0.1 g to about 10 g, such as from about 0.5 g to about 5 g. Preferably, the oral chew product has a weight of from about 1 g to about 5 g. An exemplary chew product may have a weight of about 4 g.

In some embodiments, the chew may further comprise a coating, such as a coating oil. The coating may comprise an oil or wax; for example, sunflower oil and/or carnuba wax. Suitable coatings are described in further detail in respect of the pastille products, which discussion equally applies to the chews in full. For example, the chew may be coated with an overcoat material. Devices for providing outer coating layers to compacted pelletized compositions are available as CompuLab 24, CompuLab 36, Accela-Cota 48 and Accela-Cota 60 from Thomas Engineering.

When present, a coating may comprise a film-forming polymer, such as a cellulosic polymer, an optional plasticizer, and optional flavorants, colorants, salts, sweeteners or other additives of the types set forth herein. The coating compositions may be aqueous in nature and can be applied using any pellet or tablet coating technique known in the art, such as pan coating. Example film-forming polymers include cellulosic polymers such as methylcellulose, hydroxypropyl cellulose (HPC), hydroxypropyl methylcellulose (HPMC), hydroxyethyl cellulose, and carboxy methylcellulose. Example plasticizers include aqueous solutions or emulsions of glyceryl monostearate and triethyl citrate. Additional potential coatings include food grade shellac, oils, such as sunflower oils, waxes such as carnuaba wax, and combinations thereof.

### Pastilles

In some embodiments, the products disclosed herein may be in the form of a dissolvable and lightly chewable pastille product for oral use. As used herein, the term "pastille" refers to a dissolvable oral product made by solidifying a liquid or gel composition, such as a composition that includes a gelling or binding agent, so that the final product is a hardened solid gel. A pastille product may alternatively be referred to as a soft lozenge. In certain embodiments, the pastille products of the disclosure are characterised by sufficient cohesiveness to withstand light chewing action in the oral cavity without rapidly disintegrating. The pastille products of the disclosure typically do not exhibit a highly deformable chewing quality as found in conventional chewing gum. See, for example, the smokeless tobacco pastilles, pastille formulations, pastille configurations, pastille characteristics and techniques for formulating or manufacturing pastilles set forth in US Pat. Nos. 9,204,667 to Cantrell et al.; 9,775,376 to Cantrell et al.; 10,357,054 to Marshall et al.; which are incorporated herein by reference. Certain products can exhibit, for example, one or more of the following characteristics: crispy, granular, chewy, syrupy, pasty, fluffy, smooth, and/or creamy. In certain embodiments, the desired textural property can be selected from the group consisting of adhesiveness, cohesiveness, density, dryness, fracturability, graininess, gumminess, hardness, heaviness, moisture absorption, moisture release, mouthcoating, roughness, slipperiness, smoothness, viscosity, wetness, and combinations thereof.

Pastille products of the present disclosure typically comprise a combination of active ingredients in an amount of from about 0.1% to about 10% by weight of the oral product, a binding agent, and a sugar alcohol and/or sugar (e.g. as a filler component). Any active ingredient as discussed herein is meant to be suitable for use as an active ingredient in the pastille products according to the present disclosure.

In some embodiments, the active ingredients may be provided in the pastille in liquid form or in a dry powder or particulate form. In some embodiments, each of the active ingredients included in the combination of active ingredients is provided in the pastille in a dry powder form.

A binding agent (or combination of two or more binding agents) may be employed in amounts sufficient to provide the desired physical attributes and physical integrity to the pastille products. In some embodiments, the binding agent in a pastille may comprise pectin or a gum. A representative amount of binding agent (e.g. pectin or gum) may make up at least about 5% or at least about 10% of the total weight of the pastille product. In certain embodiments, the binding agent(s) (e.g. pectin or a gum) will be present in an amount of at least about 30% by weight, at least about 35% by weight, at least about 40% by weight, at least about 45% by weight, or at least about 50% by weight, based on the total weight of the oral product. In some embodiments, the binding agent (e.g. pectin or a gum) may be present in an amount of about 35% to about 55% by weight of the oral product. Preferably, the total amount of binding agent (e.g. pectin or a gum) within the pastille product will not exceed about 55% by weight of the oral product. Often, the amount of binding agent (e.g. pectin or a gum) within a desirable product will not exceed about 65%, and frequently will not exceed about 60% by weight of the oral product.

In some embodiments, the binding agent may comprise pectin. The pastille product may include pectin as the only binding agent, or pectin may be included in combination with a gum.

In certain embodiments, the binding agent is or comprises a gum. The gum may include a natural gum. Particularly, natural gums (e.g., such as gum arabic) may be incorporated into the pastille products as a softener. Advantageously, use of a natural gum as a softener provides the desired textural qualities necessary for forming pastille products, particularly those described herein. Particularly, it should be noted that increasing the amount of a natural gum (e.g., gum arabic) while, subsequently, decreasing the amount of sugar alcohol and/or sugar can advantageously increase softness in the resulting pastille product. As used herein, a natural gum refers to polysaccharide materials of natural origin that are useful as softening agents. Representative natural gums derived from plants, which are typically water soluble to some degree, include xanthan gum, guar gum, gum arabic, ghatti gum, gum tragacanth, karaya gum, locust bean gum, gellan gum, and combinations thereof. Preferably, gum arabic may be used as an example natural gum which provides the above noted softening characteristics when incorporated into the pastille products of the present disclosure.

As noted above, pastille products of the present disclosure may comprise at least one sugar, or at least one sugar alcohol, or a combination of at least one sugar and at least one sugar alcohol (e.g. in the form of a filler component). Sugar alcohols are particularly advantageous as filler components in the pastilles of the disclosure because such materials contribute some sweetness and do not disrupt the desired chewable characteristics of the final product. In some embodiments, the sugar alcohol may be selected from the group consisting of maltitol, sorbitol, erythritol, mannitol, lactitol, xylitol and isomalt. In some embodiments, isomalt may be incorporated as the sole filler component. A sugar alcohol and/or sugar is typically added to products of the disclosure in the form of an aqueous solution or suspension, such as a solution or suspension with a solids content of about 50 to about 90% by weight. Combinations of a sugar alcohol and/or sugar with a further filler component can also be used. A filler component often fulfills multiple functions, such as enhancing certain organoleptic properties such as texture and mouthfeel, enhancing cohesiveness or compressibility of the product, and the like. In some embodiments, the filler comprises a sugar substitute, such as one or more of allulose, soluble tapioca fiber, and inulin. Such sugar substitutes may be an alternative to sugar alcohols, or used in combination with one or more sugar alcohols.

### Melts

In some embodiments, the product can be meltable as discussed, for example, in US Patent App. Pub. No. 2012/0037175 to Cantrell et al., incorporated by reference herein in its entirety. As used herein, "melt," "melting," and "meltable" refer to the ability of the product to change from a solid state to a liquid state. That is, melting occurs when a substance (e.g., a product as disclosed herein) changes from solid to liquid, usually by the application of heat. The application of heat in regard to a product as disclosed herein is provided by the internal temperature of a user's mouth. Thus, the term "meltable" refers to a product that is capable of liquefying in the mouth of the user as the product changes phase from solid to liquid, and is intended to distinguish products that merely disintegrate in the oral cavity through loss of cohesiveness within the product that merely dissolve in the oral cavity as aqueous-soluble components of the product interact with moisture.

Generally, meltable products comprise a lipid. In some embodiments, the composition comprises a lipid. The lipid is typically a fat, oil, or wax substance derived from animal or plant material (e.g., plant-derived fats), and typically comprises mostly triglycerides along with lesser amounts of free fatty acids and mono- or diglycerides. In certain embodiments, the lipid is a solid or semi-solid at room temperature (i.e., 25°C) and capable of at least partially liquefying when subjected to the temperature of the oral cavity of the user (i.e., "melting"). Example plant-derived fats are comprised primarily of saturated or unsaturated fatty acid chains (most of which are bound within triglyceride structures) having a carbon length of about 10 to about 26 carbon atoms, or about 14 to about 20 carbon atoms, or about 14 to about 18 carbon atoms.

In some embodiments, the lipid comprises an oil and, in particular, a food grade oil, including fractionated oils. Such oils include, but are not limited to, vegetable oils (e.g., acai oil, almond oil, amaranth oil, apricot oil, apple seed oil, argan oil, avocado oil, babassu oil, beech nut oil, ben oil, bitter gourd oil, black seed oil, blackcurrant seed oil, borage seed oil, borneo tallow nut oil, bottle gourd oil, brazil nut oil, buffalo gourd oil, butternut squash seed oil, cape chestnut oil, canola oil, carob cashew oil, cocoa butter, cocklebur oil, coconut oil, corn oil, cothune oil, coriander seed oil, cottonseed oil, date seed oil, dika oil, egus seed oil, evening primrose oil, false flax oil, flaxseed oil, grape seed oil, grapefruit seed oil, hazelnut oil, hemp oil, kapok seed oil, kenaf seed oil, lallemantia oil, lemon oil, linseed oil, macadamia oil, mafura oil, marula oil, meadowfoam seed oil, mongongo nut oil, mustard oil, niger seed oil, nutmeg butter, okra seed oil, olive oil, orange oil, palm oil, papaya seed oil, peanut oil, pecan oil, perilla seed oil, persimmon seed oil, pequi oil, pili nut oil, pine nut oil, pistachio oil, pomegranate seed oil, poppyseed oil, pracaxi oil, prune kernel oil, pumpkin seed oil, quinoa oil, ramtil oil, rapeseed oil, rice bran oil, royle oil, sacha inchi oil, safflower oil, sapote oil, seje oil, sesame oil, shea butter, soybean oil, sunflower oil, taramira oil, tea seed oil, thistle oil, tigernut oil, tobacco seed oil, tomato seed oil, walnut oil, watermelon seed oil, wheat germ oil, and combinations thereof), animal oils (e.g., cattle fat, buffalo fat, sheep fat, goat fat, pig fat, lard, camel fat, tallow, liquid margarine, fish oil, fish liver oil, whale oil, seal oil, and combinations thereof), and mineral oils.

In certain embodiments, the plant-derived fats of the present disclosure include palm oil, (including fractionated palm oil) palm kernel oil, soybean oil, cottonseed oil, and mixtures thereof. In one embodiment, the lipid is a blend of palm oil and palm kernel oil. The lipid can be, for example, hydrogenated, partially hydrogenated, or non-hydrogenated. Example embodiments of lipids can be purchased under the brand names CEBES^{®}, CISAO^{®}, or CONF AO^{®}, available from AarhusKarlshamn USA Inc.

The melting point of the lipid is typically about 29°C or above, such as about 29°C to about 49°C, or about 36°C to about 45°C, or about 38°C to about 41 °C. In some embodiments, use of lipids with a melting point of less than about 36°C is not advantageous due to possible melting during product storage or handling. One test for determining the melting point of lipids is the Mettler dropping point method (ASTM D3954-15, Standard Test Method for Dropping Point of Waxes, ASTM International, West Conshohocken, PA, 2015, www.astm.org.).

When present, the amount of lipid within the composition may vary. In certain embodiments, the amount of lipid is at least about 10%, at least about 20%, or at least about 30%, on a dry weight basis of the composition. In certain embodiments, the amount of lipid is less than about 70%, less than about 60%, or less than about 50%, on a dry weight basis. Example lipid weight ranges include about 10 to about 70% dry weight, such as about 35 to about 50% dry weight. In some embodiments, the amount of lipid is about 35, about 40, about 45, or about 50% by weight of the total oral product.

In some embodiments, the oral product comprises a lipid. In one embodiment, the lipid is an oil selected from the group consisting of palm oil, palm kernel oil, soybean oil, sunflower oil, cottonseed oil, coconut oil, and combinations thereof, wherein the oil may be hydrogenated, partially hydrogenated, or non-hydrogenated. In one embodiment, the lipid is a trans- hydrogenated filling fat of medium hardness such as Confao^{®} 5, available from AarhusKarlshamn USA Inc., 131 Marsh Street, Port Newark, NJ 07114.

In some embodiments, the product in meltable form comprises a lipid in an amount of from about 35 to about 50% by weight of the oral product, and a sugar alcohol in an amount of from about 35 to about 55% by weight of the oral product. In some embodiments, the sugar alcohol is isomalt, erythritol, sorbitol, arabitol, ribitol, maltitol, dulcitol, iditol, mannitol, xylitol, lactitol, or a combination thereof. In some embodiments, the sugar alcohol is or comprises isomalt. In some embodiments, a sugar substitute may be an alternative to the sugar alcohol, or used in combination with one or more sugar alcohols. Suitable sugar substitutes include allulose, soluble tapioca fiber, inulin, and combinations thereof.

### Tablets

In certain embodiments, the product is in the form of a compressed or molded tablet. An exemplary tablet dosage form weighs from about 250 mg to about 1500 mg, such as about 250 mg to about 700 mg, or from about 700 mg to about 1500 mg. The tablet can have any of a variety of shapes, including traditional pill or tablet shapes. Generally, the product in tablet form comprises a glucose-polysaccharide blend and a sugar alcohol. In some embodiments, the glucose-polysaccharide blend is present in an amount of from about 35 to about 50% by weight, based on the total weight of the product; and the sugar alcohol is present in an amount of from about 30 to about 45% by weight, based on the total weight of the product. In some embodiments, the sugar alcohol is isomalt, erythritol, sorbitol, arabitol, ribitol, maltitol, dulcitol, iditol, mannitol, xylitol, lactitol, or a combination thereof. In some embodiments, the sugar alcohol is or comprises isomalt.

When in the form of a tablet, the product may be dissolvable. As used herein, the terms "dissolve," "dissolving," and "dissolvable" refer to products having aqueous-soluble components that interact with moisture in the oral cavity and enter into solution, thereby causing gradual consumption of the product. According to one aspect, the dissolvable product is capable of lasting in the user's mouth for a given period of time until it completely dissolves. Dissolution rates can vary over a wide range, from about 1 minute or less to about 60 minutes. For example, fast release products typically dissolve and/or release the desired component(s) (e.g., active ingredient, flavour, and the like) in about 2 minutes or less, often about 1 minute or less (e.g., about 50 seconds or less, about 40 seconds or less, about 30 seconds or less, or about 20 seconds or less). Dissolution can occur by any means, such as melting, mechanical disruption (e.g., chewing), enzymatic or other chemical degradation, or by disruption of the interaction between the components of the product. In other embodiments, the products do not dissolve during the product's residence in the user's mouth.

### Lozenge

In some embodiments, the products disclosed herein may be in the form of a dissolvable lozenge product configured for oral use. Example lozenge-type products of the invention have the form of a lozenge, tablet, microtab, or other tablet-type product. See, for example, the types of nicotine-containing lozenges, lozenge formulations, lozenge formats and configurations, lozenge characteristics and techniques for formulating or manufacturing lozenges set forth in US Pat. Nos. 4,967,773 to Shaw; 5,110,605 to Acharya; 5,733,574 to Dam; 6,280,761 to Santus; 6,676,959 to Andersson et al.; 6,248,760 to Wilhelmsen; and 7,374,779; US Pat. Pub. Nos. 2001/0016593 to Wilhelmsen; 2004/0101543 to Liu et al.; 2006/0120974 to Mcneight; 2008/0020050 to Chau et al.; 2009/0081291 to Gin et al.; and 2010/0004294 to Axelsson et al.; which are incorporated herein by reference.

Lozenge products are generally described as "hard", and are distinguished in this manner from soft lozenges (i.e., pastilles). Hard lozenges are mixtures of sugars and/or carbohydrates in an amorphous state. Although they are made from aqueous syrups, the water, which is initially present, evaporates as the syrup is boiled during processing so that the moisture content in the finished product is very low, such as 0.5% to 1.5% by weight. To obtain lozenges that are hard and not tacky, the temperature of the melt generally must reach the hard crack stage, with an example temperature range of 149° to 154°C.

Lozenge-type products, in some embodiments, may exhibit translucence or transparency. The desired transparency or translucency of the product can be quantified by any known method. For example, optical methods such as turbidimetry (or nephelometry) and colorimetry may be used to quantify the cloudiness (light scattering) and the color (light absorption), respectively, of the products. Translucency can also be confirmed by visual inspection by simply holding the product up to a light source and determining if light travels through the material or product in a diffuse manner.

The lozenge-type products of the present disclosure may incorporate various different additives in addition to the combination of active ingredients, and may be prepared according to a variety of different methods commonly known in the art for preparing lozenge-type products. Example compositions, products, and methods of preparing such products will be detailed herein below.

Lozenge products of the present disclosure typically include a composition comprising the combination of active ingredients in an amount of less than about 2% by weight, a sugar substitute in an amount of at least about 80% by weight, and a sugar alcohol syrup. Any active ingredient as discussed herein is suitable for use as an active ingredient in the lozenge products provided herein. In some embodiments, the active ingredient may be provided in liquid form or in a dry powder or particulate form. As noted above, the active ingredient typically is present in an amount from about 0.1% to about 10% by weight, such as, e.g., from about 0.1% to about 10% by weight, such as, e.g., from about 0.1%, about 0.5%, about 1%, about 1.5%, about 2%, about 2.5%, about 3%, about 3.5%, about 4%, or about 4.5% by weight, to about 5.5%, about 6%, about 6.5%, about 7%, about 7.5%, about 8%, about 8.5%, about 9%, about 9.5%, or about 10% by weight, based on the total weight of the product. In some embodiments, the active ingredient may be present in an amount of less than about 10%, less than about 9%, less than about 8%, less than about 7%, less than about 6%, less than about 5%, less than about 4%, less than about 3%, less than about 2%, or less than about 1% by weight, based on the total weight of the product.

In some embodiments, the lozenge product comprises a sugar substitute. The sugar substitute is typically provided in pure, solid form (e.g., granular or powdered form). In certain embodiments, the sugar substitute is dry, comprising a very low water content. For example, the sugar substitute can comprise less than about 5% water by weight, less than about 3% water by weight, less than about 2% water by weight, or less than about 1% water by weight. In certain embodiments, the sugar substitute is capable of forming a glassy matrix. The formation of a glassy matrix is commonly characterized by a translucent/transparent appearance.

Typically, the sugar substitute is substantially non-hygroscopic. Non-hygroscopic materials typically do not absorb, adsorb, and/or retain a significant quantity of moisture from the air. Non-hygroscopic materials can provide the benefit of reducing the tendency of the lozenge product to tackify upon exposure to humidity. The sugar substitute can be any sugarless material (i.e., sucrose-free material) and can be natural or synthetically produced. The sugar substitute used in the products described herein can be nutritive or non-nutritive. For example, the sugar substitute is commonly a sugar alcohol. Sugar alcohols that may be useful according to the present invention include, but are not limited to, erythritol, threitol, arabitol, xylitol, ribotol, mannitol, sorbitol, dulcitol, iditol, isomalt, maltitol, lactitol, polyglycitol, and mixtures thereof. For example, in certain embodiments, the sugar alcohol is selected from the group consisting of erythritol, sorbitol, and isomalt. The amount of sugar substitute in the lozenge products can vary, but is typically at least about 75%, at least about 80%, at least about 85%, or at least about 90%, or at least about 95% by weight of the product.

In certain embodiments, the sugar substitute comprises one or more sugar alcohols. For example, in one embodiment, the sugar substitute is isomalt. In some embodiments, the sugar substitute is one or more of allulose, soluble tapioca fiber, and inulin. Such sugar substitutes may be an alternative to sugar alcohols, or used in combination with one or more sugar alcohols.

In some embodiments, the lozenge products of the present disclosure may comprise a syrup, e.g., a sugar syrup or a sugar alcohol syrup. "Sugar alcohol syrup" as used herein is intended to refer to a thick solution of sugar alcohol in water, e.g., having greater than about 40% solids, preferably having greater than about 50% solids, greater than about 60% solids, greater than about 70% solids, or greater than about 80% solids. Typically, the solid content of the sugar alcohol syrup primarily comprises the named sugar alcohol (i.e., maltitol syrup typically comprises greater than about 80%, greater than about 85%, or greater than about 90% by weight maltitol on a dry basis). Sugar alcohol syrups are generally prepared by heating a solution of the sugar alcohol in water and cooling the mixture to give a viscous composition. The resulting syrup is typically characterized by a relatively high concentration of sugar alcohol and relatively high stability (i.e., the sugar alcohol typically does not crystallise from solution, e.g., at room temperature).

The syrup, e.g., sugar alcohol syrup, desirably is capable of affecting the recrystallisation of a melted sugar substitute. One example sugar alcohol syrup that is particularly useful according to the present disclosure is maltitol syrup. Other sugar alcohol syrups can be used, including, but not limited to, com syrup, golden syrup, molasses, xylitol, mannitol, glycerol, erythritol, threitol, arabitol, ribitol, mannitol, sorbitol, dulcitol, iditol, isomalt, lactitol, and polyglycitol syrups. Such sugar alcohol syrups can be prepared or can be obtained from commercial sources. For example, maltitol syrups are commercially available from such suppliers as Corn Products Specialty Ingredients. Although sugar alcohol syrups may be preferred, sugar syrups can, in certain embodiments, be used in place of or in combination with the sugar alcohol syrup. For example, in some embodiments, corn syrup, golden syrup, and/or molasses can be used.

The amount of sugar alcohol syrup added to the lozenge composition mixture is typically that amount required to slow recrystallisation of the sugar substitute in melted form. It should be noted that it may be possible to vary the amount of sugar alcohol syrup depending on the composition of the remaining ingredients to ensure that the recrystallisation is sufficiently slow to provide a material with the desired characteristics (e.g., a desired level of translucency/transparency). Accordingly, the amount of sugar alcohol syrup can vary, but typically ranges from about 0.1% to about 2%, often from about 0.5% to about 1.5%, and more often about 1% by weight of the lozenge product mixture. In certain embodiments, the amount of sugar alcohol syrup is higher, for example, up to about 2% by weight of the mixture, up to about 5% by weight of the mixture, up to about 10% by weight of the mixture, or up to about 20% by weight of the mixture.

Representative lozenge compositions and products may incorporate about 10% by weight or less of the combination of active ingredients, about 0.01 to about 2% artificial sweetener, about 1% to about 5% humectant, about 1% to about 5% natural sweetener, at least about 80% of a sugar substitute, about 0.1% to about 10% of a sugar alcohol syrup, one or more flavorants in an amount of up to about 5%, and salt in an amount up to about 3%, based on the total weight of the product. The particular percentages and choice of ingredients will vary depending upon the desired flavor, texture, and other characteristics.

Oral products of the present disclosure in the form of a lozenge may contain various amounts of water. The water content of the lozenge described herein, prior to use by a consumer of the product, may vary within such ranges according to the desired properties and characteristics, in addition to dictating the final form of the product. For example, lozenge-type products typically possess a water content in the range of about 0.1 to about 5% by weight of the product. Preferably, the moisture content of a lozenge product, as present within a single unit of product prior to insertion into the mouth of the user, is less than about 5%, less than about 3%, less than about 2%, or less than about 1% by weight of the product. In some embodiments, the moisture content of a lozenge product as described herein may be within the range of about 0.1% to about 5%, about 0.5 to about 3%, or about 1 to about 2 % by weight of the product.

### Powders or pouched products

In some embodiments, the oral product may be in the form of a powder. The powder may be a free-flowing powder. The powder may be contained in loose form within a container, and may thus be used in a form similar to tobacco snuff where the user takes a pinch of powder from the container and places the powder in the oral cavity. Alternatively, or additionally, the powder may be incorporated into a moisture-permeable (e.g. saliva-permeable) pouch, similar to a snus-type product. The pouched product may be configured for insertion into the oral cavity of a user; i.e. it may be a pouched oral product.

In some embodiments, the product of the present disclosure is in the form of a pouched oral product. Such a pouched product comprises the oral product as described herein, disposed within a moisture-permeable container (e.g., a water-permeable pouch or saliva-permeable pouch). For example, the pouched product may comprise the oral product in a powder form incorporated within the saliva-permeable pouch.

Such compositions in the moisture-permeable pouch format are typically used by placing one pouch containing the composition in the mouth of a human subject/user. Generally, the pouch is placed somewhere in the oral cavity of the user, for example under the lips, in the same way as moist snuff products are generally used. The pouch preferably is not chewed or swallowed. Exposure to saliva then causes some of the components of the composition therein (e.g., the active ingredients and/or any flavours) to pass through e.g., the moisture-permeable pouch and provide the user with flavour and satisfaction, and the user is not required to spit out any portion of the composition. After about 10 minutes to about 60 minutes, typically about 15 minutes to about 45 minutes, of use/enjoyment, substantial amounts of the composition have been ingested by the human subject, and the pouch may be removed from the mouth of the human subject for disposal.

In some embodiments, the pouch is saliva-permeable. This means that the pouch is made of a saliva-permeable pouch material. The pouch material used in oral pouched products is typically a dry-laid bonded nonwoven comprising viscose rayon fibres (i.e. regenerated cellulose) and an acrylic polymer that acts as binder in the nonwoven material and provides for heat-sealing of the pouches during manufacturing thereof. The pouch material may also comprise synthetic fibres (e.g. polyester) in addition to viscose fibres. The viscose nonwoven material normally used for smokeless tobacco pouches is similar to the fabric used in tea bags. Nonwovens are fabrics that are neither woven nor knitted. Methods for the manufacturing of nonwoven materials are commonly known in the art. Further information on nonwovens is found in "Handbook of Nonwovens" by S. Russel, published by Woodhead Pub I. Ltd., 2007. In some embodiments, the pouch material is a fleece material. In some embodiments, the pouch material is a non-woven material. In some embodiments, the pouch material is a non-woven fleece material. In some embodiments, the pouch material comprises viscose, such as viscose rayon fibres. In some embodiments, the pouch material comprises regenerated cellulose fibres. In some embodiments, the pouch material comprises polyester fibres; the polyester fibres may constitute the pouch material or may be included in combination with viscose (such as regenerated cellulose fibres).

In some embodiments, the pouch material comprises a binder that provides for heat sealing of the pouches during manufacture. In some embodiments, the pouch material comprises an acrylic binder. In some embodiments, the pouch material comprises an acrylic binder in combination with viscose and/or polyester fibres.

Suitable packets, pouches or containers of the type used for the manufacture of smokeless tobacco products are available under the tradenames CatchDry, Ettan, General, Granit, Goteborgs Rape, Grovsnus White, Metropol Kaktus, Mocca Anis, Mocca Mint, Mocca Wintergreen, Kicks, Probe, Prince, Skruf and TreAnkrare. The composition may be contained in pouches and packaged, in a manner and using the types of components used for the manufacture of conventional snus types of products. The pouch provides a moisture-permeable container of a type that may be considered to be similar in character to the mesh-like type of material that is used for the construction of a tea bag. Components of the composition readily diffuse through the pouch and into the mouth of the user. Non-limiting examples of suitable types of pouches are set forth in, for example, US Pat. Nos. 5,167,244 to Kjerstad and 8,931,493 to Sebastian et al.; as well as US Patent App. Pub. Nos. 2016/0000140 to Sebastian et al.; 2016/0073689 to Sebastian et al.; 2016/0157515 to Chapman et al.; and 2016/0192703 to Sebastian et al., each of which is incorporated herein by reference. Pouches can be provided as individual pouches, or a plurality of pouches (e.g., 2, 4, 5, 10, 12, 15, 20, 25 or 30 pouches) can be connected or linked together (e.g., in an end-to-end manner) such that a single pouch or individual portion can be readily removed for use from a one-piece strand or matrix of pouches.

An example pouch may be manufactured from materials, and in such a manner, such that during use by the user, the pouch undergoes a controlled dispersion or dissolution. Such pouch materials may have the form of a mesh, screen, perforated paper, permeable fabric, or the like. For example, pouch material manufactured from a mesh-like form of rice paper, or perforated rice paper, may dissolve in the mouth of the user. As a result, the pouch and composition each may undergo complete dispersion within the mouth of the user during normal conditions of use, and hence the pouch and composition both may be ingested by the user. Other examples of pouch materials may be manufactured using water dispersible film forming materials (e.g., binding agents such as alginates, carboxymethylcellulose, xanthan gum, pullulan, and the like), as well as those materials in combination with materials such as ground cellulosics (e.g., fine particle size wood pulp). Preferred pouch materials, though water dispersible or dissolvable, may be designed and manufactured such that under conditions of normal use, a significant amount of the composition contents permeate through the pouch material prior to the time that the pouch undergoes loss of its physical integrity. If desired, flavoring ingredients, disintegration aids, and other desired components, may be incorporated within, or applied to, the pouch material.

The amount of the oral product contained within each pouched product unit, for example, a pouch, may vary. In some embodiments, the weight of the composition within each pouch is at least about 50 mg, for example, from about 50 mg to about 1 gram (1,000 mg), such as from about 100 mg to about 900 mg, such as from about 200 mg to about 800 mg, such as from about 500 mg to about 700 mg. In some smaller embodiments, the weight of the composition within each pouch may be from about 100 mg to about 300 mg. For a larger embodiment, the weight of the composition within each pouch may be from about 300 mg to about 700 mg. If desired, other components can be contained within each pouch.

The moisture content of the oral product may vary depending on the format in which the composition is provided. In some embodiments as described hereinabove, the oral product may be in the form of moist snuff or snus and/or may be provided in pouched formats. In some embodiments (e.g. for snus-type products), the moisture content of the composition (before insertion of the product into the user's mouth) may be at least about 20% by weight, such as at least 30% by weight, such as at least 40% by weight, such as at least 50% by weight of the oral product. In some embodiments (for example, for snus-type products; e.g. non-pouched or pouched snus products), the moisture content of the composition (before insertion of the product into the user's mouth) may be from about 20% to about 70% by weight, such as from about 30% to about 60% by weight, such as from about 40% to about 55% by weight of the oral product.

In some embodiments, the oral product may be a snus-type or snuff-type product that is in 'dry' form. In such embodiments, the moisture content of the oral product may be no greater than about 10% by weight, such as no greater than about 5% by weight of the oral product. For example, the moisture content may be from about 0.1% to about 10% by weight, such as from about 1% to about 5% by weight of the oral product.

When in the form of a pouched oral product, the oral product typically comprises a filler. The filler may preferably be a cellulose material selected from the suitable materials described hereinabove. In some preferred embodiments, the filler is or comprises at least MCC. The amount of filler can vary, but is typically at least about 5% to about 95% by weight of the oral product, based on the total weight of the oral product. In some embodiments, the filler (such as a cellulose material, such as MCC) may be present in the oral product in an amount of from about 5% to about 95% by weight, such as from about 10% to about 90% by weight, such as from about 15% to about 85% by weight, such as from about 20% to about 80% by weight, such as from about 25% to about 75% by weight, such as from about 30% to about 70% by weight, such as from about 35% to about 65% by weight, such as from about 40% to about 60% by weight of the oral product. In some embodiments, the filler (such as a cellulose material, such as MCC) may be present in an amount of from about 45% to about 55% by weight of the oral product.

### Package

According to some embodiments described herein, there is provided a package containing an oral product as described herein. For example, the package may contain the oral product in powdered form. In such embodiments, the package may be in the form of a tin or plastic container. Alternatively or additionally, the package may contain the oral product in the form of a chew, lozenge, pastille, tablet, or the like. The package may be in the form of a blister pack, tin or plastic container containing such oral dosage forms.

According to some embodiments described herein, there is provided a package containing at least one pouched oral product as described herein. A pouched product as described herein can be packaged within any suitable inner packaging material and/or outer container. See also, for example, the various types of containers for smokeless types of products that are set forth in US Pat. Nos. 7,014,039 to Henson et al.; 7,537,110 to Kutsch et al.; 7,584,843 to Kutsch et al.; 8,397,945 to Gelardi et al., D592,956 to Thiellier; D594,154 to Patel et al.; and D625,178 to Bailey et al.; US Pat. Pub. Nos. 2008/0173317 to Robinson et al.; 2009/0014343 to Clark et al.; 2009/0014450 to Bjorkholm; 2009/0250360 to Bellamah et al.; 2009/0266837 to Gelardi et al.; 2009/0223989 to Gelardi; 2009/0230003 to Thiellier; 2010/0084424 to Gelardi; and 2010/0133140 to Bailey et al; 2010/0264157 to Bailey et al.; and 2011/0168712 to Bailey et al. which are incorporated herein by reference. For example, the package may be a tin or plastic container which contains a plurality of the pouched oral products.

### Processes

The manner by which the various components of the composition (e.g., active ingredients and any additives) are combined may vary. The components noted above, which may be in liquid or dry solid form, can be admixed in a pre-treatment step prior to mixture with any remaining components of the product, or simply mixed together with all other liquid or dry ingredients.

The various components of the product may be contacted, combined, or mixed together using any mixing technique or equipment known in the art. Any mixing method that brings the product ingredients into intimate contact can be used, such as a mixing apparatus featuring an impeller or other structure capable of agitation. Examples of mixing equipment include casing drums, conditioning cylinders or drums, liquid spray apparatus, conical-type blenders, ribbon blenders, mixers available as FKM130, FKM600, FKM1200, FKM2000 and FKM3000 from Littleford Day, Inc., Plough Share types of mixer cylinders, Hobart mixers, and the like. See also, for example, the types of methodologies set forth in US Pat. Nos. 4,148,325 to Solomon et al.; 6,510,855 to Korte et al.; and 6,834,654 to Williams, each of which is incorporated herein by reference. Manners and methods for formulating products will be apparent to those skilled in the art. See, for example, the types of methodologies set forth in US Pat. No. 4,148,325 to Solomon et al.; US Pat. No. 6,510,855 to Korte et al.; and US Pat. No. 6,834,654 to Williams, US Pat. Nos. 4,725,440 to Ridgway et al., and 6,077,524 to Bolder et al., each of which is incorporated herein by reference.

### Method of preparing liquid products

In accordance with some embodiments described herein, there is provided a process for preparing an oral product as described herein, the oral product preferably being in the form of a liquid, the process comprising the steps of:
(a) combining active ingredients,
(b) contacting the active ingredients with water, and
(c) mixing the active ingredients with water to prepare an oral product.

The combination of active ingredients may be as described hereinabove.

The active ingredients may be provided in the form of a liquid extract, a liquid oil or a powder. When in the form of a powder, step (c) may comprise mixing the active ingredients with water until said active ingredients have dissolved in the water.

Step (b) and/or step (c) may preferably be carried out at ambient or room temperature (20-25°C). Alternatively, the temperature may be elevated in order to assist with dispersion or dissolution of the active ingredients in the water. For example, step (b) may comprise contacting the active ingredients with water at a temperature of from about 20-100°C, such as from about 30-90°C, or from about 40-80°C. Step (b) may comprise mixing the active ingredients with water at a temperature of from about 20-100°C, such as from about 30-90°C, or from about 40-80°C.

The process may comprise adding any additional additives at any stage. For example, any additives may be added to the active ingredients prior to combination with water and/or after the active ingredients have been combined with water. The additives may also be added to the water prior to contacting the water with the combination of active ingredients. Step (a) may comprise an optional step of combining any additives (e.g. acidifying agent) with the combination of active ingredients. Step (b) may comprise an optional step of contacting the active ingredients with water and an additive. Step (c) may comprise an optional step of adding additives to the mixture and mixing the additives with the combination of active ingredients and water.

In some embodiments, the oral product further comprises one or more additives. In such embodiments, the one or more additives may be combined with water prior to addition of the active ingredients. Optionally, the step of combining the one or more additives with water may comprise heating the combination, e.g. to a temperature of from about 60°C to about 80°C, to achieve dissolution of the additive(s). Further additives, such as a sweetener, humectant, colouring agent or the like, may be added at this stage or may be added during and/or after the step of combining the active ingredients with the water.

The resulting liquid product may be in the form of a solution or dispersion of active ingredients in water. Preferably the liquid product is in the form of a solution of active ingredients in water.

### Method of preparing chew products

As described herein, the product may be in chewable form. In such embodiments, the process may comprise the steps of:
(a) contacting at least one binding agent and at least one bulking agent selected from the group consisting of a sugar alcohol, or a sugar, or a combination of at least one sugar alcohol and at least one sugar, and optionally adding water;
(b) heating the mixture of binding agent, sugar alcohol and/or sugar, and optionally water;
(c) adding a combination of active ingredients;
(d) allowing the resulting mixture to set to provide an oral chew product.

The combination of active ingredients may be as described hereinabove.

Step (b) of heating the mixture may comprise heating the mixture to a temperature of about 70°C to about 150°C, such as from about 80°C to about 125°C, such as from about 90°C to about 100°C. Step (b) may comprise heating the mixture to boiling, optionally while stirring the mixture. Step (d) preferably comprises a cooling step. In some embodiments, the resulting mixture from step (c) is allowed to cool in order to set to provide an oral chew product. For example, after the active ingredients are added in step (c), the resulting mixture may be deposited into a mold and the heat removed. The mixture may be allowed to cool to room temperature passively. Alternatively, the mixture may be placed into a cool water bath, fridge or freezer in order to reduce the temperature. Preferably, cooling happens at room temperature to allow the product to set.

The combination of active ingredients may be added during the heating step (b). In some embodiments, the combination of active ingredients is added while the mixture of binding agent, sugar alcohol and/or sugar and optionally water is boiling. Alternatively, the combination of active ingredients may be added after cooling the mixture of binding agent, sugar alcohol and/or sugar and optionally water.

Any additives may be added at any stage during the above process. In some embodiments, an acidifying agent is added after removing the mixture from the heat; i.e. during step (c) or (d). In some embodiments, a flavouring agent and/or colouring agent is added after removing the mixture from the heat; i.e. during step (c) or (d). In some embodiments, a sweetener is added during step (a). The sweetener may therefore be included in the mixture that is heated in step (b).

For the preparation of the product in chewable form, generally, a binder (e.g. pectin, agar, carrageenan, starch, or a combination thereof) is pre-blended with all or a portion of the sugar alcohol/sugar, sweetener, or combination thereof). Water is added, and the mixture heated to boiling with stirring. Any remaining sugar alcohol or sweetener is added to the boiling mixture, along with the active ingredients, followed by any buffering agent. The mixture is cooked to a degrees brix from about 50 to about 80. Heat is removed, and any acidifying agent and/or flavorant added, along with any colorant, and the mixture thoroughly combined. The composition is deposited into molds for storage at ambient temperature.

In some embodiments, the composition is deposited in a starch mold. Starch trays with molded shapes are prepared and pre-heated at 60°C for at least 1- 2 hours. The starch can be any starch as disclosed herein above. In some embodiments, the starch is corn starch. In some starch molded embodiments, pectin binder is pre-blended with a portion of the sugar alcohol (e.g. isomalt or maltitol). Water is added, and the mixture heated to boiling with stirring. Further sugar alcohol (e.g. maltitol syrup and/or isomalt) are added to the boiling mixture, along with the active ingredients. The mixture is cooked to around 78 brix. Heat is removed, and any sweetener (e.g., sucralose or acesulfame K) and flavorant added, along with any colorant and acidifying agent (e.g. citric acid solution), and the mixture thoroughly combined. The hot mixture is deposited into starch molds for storage at ambient temperature. The resulting chews are removed from the starch mold, and any excess starch removed.

In other starch molded embodiments, a gum powder (e.g. pectin, agar, carrageenan, starch, or a combination thereof) is mixed with water until lump-free. Sugar alcohol and/or sugar (e.g. isomalt and/or maltitol syrup) and any sweetener (e.g. sucralose) are mixed together and the mixture heated to 82-104 °C. The gum powder solution is added into the sugar alcohol/sugar and mixed thoroughly. The active ingredient(s), and any colour and flavour are added to above solution and mixed thoroughly. The mixture is cooked at 93-104°C until a degrees brix of 50-80 is achieved. A solution of acidifying agent (e.g. citric acid and/or trisodium citrate dihydrate) in water is prepared and added to the hot mixture. Any gelling agents (e.g. dicalcium phosphate solution) is then added into the mixture if necessary. The hot mixture is deposited into the prepared starch molds and kept in an oven at 60°C overnight, or until proper setting is achieved. The resulting chews are removed from the starch mold, and any excess starch removed. In some embodiments, the chews are coated, e.g. with coating oil or CAPOL 410 (available from Centerchem, Inc.). The coating process may be carried out in the same manner as described in detail below with respect to pastille products.

In other embodiments, the composition is deposited in a starchless mold. In such embodiments, a gum powder (e.g. pectin, agar, carrageenan, starch, or a combination thereof) is mixed with water until lump free. Maltitol syrup, sucralose, and optionally isomalt, are mixed together and the mixture heated to 82-104 °C. The gum powder solution is added into the maltitol solution and mixed thoroughly. The active ingredient(s), and any colour and flavour are added to and the mixture mixed thoroughly. The mixture is cooked at 93-104°C until a degrees brix of 50-80 is achieved. A solution of citric acid and optionally trisodium citrate dihydrate in water is prepared and added to the hot mixture to achieve a pH between 2.5 and 4. Any gelling agents (e.g. dicalcium phosphate solution) is then added into the mixture if necessary. The hot mixture is deposited into the starchless molds and left at room temperature, until proper setting is achieved.

The chew product may be held in the mold (starch or starchless) for a predetermined duration of time such as, for example, about 10 minutes to about 24 or even 48 hours, so as to allow the chew product to cure and solidify.

According to other aspects of the present disclosure, rather than using molds to prepare the chew product, an extrusion process may be employed in which the final chew product is extruded as described hereinbelow with respect to pastille extrusion methods.

### Method of preparing pastille products

The manners and methods used to formulate and manufacture a pastille product as described herein can vary. For example, the compositions forming the pastille products may be prepared such that the mixture thereof may be used in a starch molding process for forming the pastille product. Example pastille production processes are set forth in US Pat. Nos. 4, 725,440 15 to Ridgway et al and 6,077,524 to Bolder et al., which are incorporated by reference herein. In some embodiments, the compositions for forming the pastille products may be prepared such that the mixture thereof may be used in a starchless molding process (e.g., not including a starch-based component in the molding process) for forming the pastille product.

In some embodiments, the process comprises heating a gum, and optionally hydrating that gum component with water, and then stirring the combination of active ingredients into the heated gum component. Generally, the gum may be heated to a temperature in the range of about 60°C to about 80°C for a period of a few seconds to a few minutes. In some embodiments, the gum may be heated to a temperature of about 71°C before stirring in the combination of active ingredients, to allow the active ingredients to dissolve therein. In some instances, an aqueous mixture is formed in a separate container by mixing one or more additives (e.g., such as salts, sweeteners, humectants, emulsifiers, flavouring agents, and others) with water to form the aqueous mixture. Then, the aqueous mixture may be admixed with the heated gum (including the at least one active ingredient that has been added therein) to form a mixture in the form of a slurry. In some embodiments, the at least one sugar alcohol/sugar component may be added separately to this mixture, or, in other embodiments, the at least one sugar alcohol/sugar may be combined with the gum and the active ingredient prior to addition to the mixture. In some instances, the at least one sugar alcohol/sugar may be heated in yet another separate container and added to the mixture separately. For example, in some embodiments, the at least one sugar alcohol/sugar (which may optionally include isomalt/maltitol/erythritol) may be heated to a temperature in the range of about 160°C to about 190°C before addition to the mixture. In some embodiments, the at least one sugar alcohol/sugar may be heated to a temperature of at least about 160°C, at least about 170°C, at least about 180°C, or at least about 190°C. In some instances, the heated sugar alcohol/sugar may be allowed to cool to a temperature in the range of about 120°C to about 160°C prior to addition to the mixture. In some embodiments, for example, the heated sugar alcohol/sugar may be cooled to a temperature of about 160°C or less, about 150°C or less, about 140°C or less, or about 130°C or less prior to addition to the mixture.

In some instances, the heated (and optionally cooled) sugar alcohol/sugar may be combined with the mixture (e.g., including the heated gum, the at least one active ingredient, and the aqueous mixture) and stirred using a high shear mixer or a Hobart mixing bowl with a whipping attachment to provide a pastille composition, which may also be in the form of a slurry. The pastille composition may then be heated to an elevated temperature for a period of time, for example, heated to between about 40°C to about 80°C, and typically heated to about 71 °C, for a period of about 1 to about 3 minutes, for example, to dissolve any dry ingredient within the pastille composition. The heating step can be characterized as heating at a temperature of at least about 50°C, at least about 60°C, or at least about 70°C. The pastille composition may typically have a moisture content of at least about 40 percent by weight water, based on the total weight of the composition.

According to some aspects, the pastille composition, in the form of a slurry, may optionally be put through a deaerating step or process prior to being received in a mold or being subjected to other processing steps, so as to reduce or eliminate air bubbles present in the slurry mixture. Air bubbles entrapped within the slurry may affect the final weight of the pastille product, which could lead to a lack of weight uniformity between units of the final product. As such, any deaerating methods and systems may be employed for removing such air bubbles from the slurry material. For example, the slurry may be placed under reduced pressure (i.e., below atmospheric pressure) to pull the air bubbles out of the slurry mixture. In some instances, a vacuum deaerating process may be employed in which the slurry mixture is placed in a vacuum deaerator for deaerating the slurry mixture using pressure reduction. In some instances, the slurry mixture may be under vacuum for about 1 to about 10 minutes, and typically for about 3 to about 5 minutes. The deaerating step may be observed and adjusted accordingly in order to controllably remove the gaseous components from the slurry mixture.

The viscosity of the heated and deaerated slurry mixture may be measured using, for example, a Brookfield viscometer HA Series, SC4 water jacket, 27/13R sample chamber and a No. 27 spindle. The pastille composition may have a viscosity of about 5.7 Pascal-seconds (Pa·s) to about 6.2 Pa·s when heated to a temperature of about 38°C, about 4.9 Pa·s to about 5.4 Pa·s when heated to a temperature of about 43°C, and about 4.2 Pa·s to about 4.7 Pa·s when heated to a temperature of about 50°C. In some instances, extra water may be added to the pastille composition so as to provide a desired viscosity thereof. Once the desired viscosity is achieved, the heated pastille composition may then be deposited into a mold, such as, for example, a starch mold. While the process as further described herein is directed to forming a pastille product using a starch mold, it is noted that other types of molds may be used in the process, such as, for example, starchless molds, pectin molds, plastic tray molds, silicone tray molds, metallic tray molds, neoprene tray molds, and the like.

In instances involving the use of starch molds, the starch molds may be pre-dried to remove moisture content from the starch mold itself. That is, prior to receiving the slurry or viscous pastille composition, the starch mold may be subjected to an elevated temperature to drive out moisture in the starch mold. For example, in some instances, the starch mold may initially have a moisture content of about 10-15% by weight. Such levels of moisture could potentially have an effect on the uniformity of the resultant product. In this regard, certain moisture levels in the starch mold could potentially have a wrinkling or pruning effect on the product such that the final product has a shrivelled or otherwise wrinkled appearance. As such, the starch mold may be dried at an elevated temperature to reduce the moisture content of the starch mold to between about 4 and about 10% by weight, and preferably between about 6 and about 8% by weight, based on the total weight of the starch mold. By taking such steps, the product may, in some instances, be more uniformly consistent in appearance. Furthermore, the starch mold may be heated to an elevated temperature prior to receiving the pastille composition such that the starch mold itself is at an elevated temperature when receiving the pastille composition.

The pastille composition may remain in the starch mold at an elevated temperature such as, for example, at between about 40°C to about 80°C (e.g., at least about 40°C or at least about 50°C), and typically at about 60°C. The pastille composition may be held at the elevated temperature for a predetermined duration of time such as, for example, about 12- 48 hours, and typically about 24 hours, so as to allow the pastille composition to cure and solidify into pastille form, while driving the moisture content of the pastille composition to a desired final moisture level. As noted above, in some embodiments, the desired final moisture level of the pastille product may be within the range of about 5 to about 25% by weight, or about 8 to about 20% by weight, or about 10 to about 15% by weight, based on the total weight of the product unit. In this regard, curing generally refers to the solidification process in which moisture loss occurs, the viscosity of the composition is raised, and chemical and physical changes begin to occur (e.g., crystallisation, cross-linking, gelling, film forming, etc.). The pastille composition is allowed to cool and thereafter removed from the starch mold. In some instances, the pastille composition may be allowed to cool at refrigerated or below ambient temperatures. An air blower I shaker device can be used to remove starch remnants from the pastille composition after being removed from the starch mold.

The pastille product is then allowed to post-cure for a time and at a temperature suitable to allow the product to become equilibrated to a desired moisture, shape and form. The time and temperature can vary without departing from the invention and depend in part on the desired final characteristics of the product. In one embodiment, the post-cure is conducted at ambient temperature for at least about 20 hours after being removed from the mold.

A pastille product may be provided in individual pieces weighing between about 0.5 grams to about 5 grams, although aspects of the present disclosure are not limited to such weights.

The curing times and temperatures of the pastille product can be varied as desired. In this regard, such variables may affect the final visual appearance of the pastille product. For example, extended curing times and/or low curing temperatures may affect the final outer configuration or contours of the pastille product. That is, the rate of drying and/or curing of the product can affect the final properties of the product. In some instances, for example, lowering the curing temperature and extending the curing time may cause the pastille product to have a relatively smooth outer surface. In contrast, curing at higher temperatures for shorter period of times can lead to a roughened or wrinkled appearance in the product.

According to other aspects of the present disclosure, rather than using molds to prepare the pastille product, an extrusion process may be employed in which the final pastille product is extruded. In some instances, the pastille composition in slurry form may be formed into a sheet and allowed to dry to a moisture content, for example, of about 15 percent to about 25 percent by weight water to form a tacky or otherwise pasty material, which is in a form capable of physical handling. The material may then be chopped or otherwise cut into smaller pieces using, for example, a mixer. The chopped material may then be extruded through an extrusion device to any shape/size desired, including shapes that may be difficult or impossible to achieve with a mold. In some instances, the extruded product may then be dried to achieve a desired moisture content. A similar type process is described, for example, in U.S. Pat. No. 3,806,617 to Smylie et al., which is incorporated herein by reference in its entirety. Further, the pastille composition may be subjected to a co-extrusion process with another composition.

Shapes such as, for example, rods and cubes can be formed by first extruding the material through a die having the desired cross-section (e.g., round or square) and then optionally cutting the extruded material into desired lengths. Techniques and equipment for extruding tobacco materials are set forth in US Pat. Nos. 3,098,492 to Wursburg; 4,874,000 to Tamol et al.; 25 4,880,018 to Graves et al.; 4,989,620 to Keritsis et al.; 5,072,744 to Luke et al.; 5,829,453 to White et al.; and 6,182,670 to White et al.; each of which is incorporated herein by reference. Example extrusion equipment suitable for use include food or gum extruders, or industrial pasta extruders such as Model TP 200/300 available from Emiliomiti, LLC of Italy. In some instances, a single machine may be capable of achieving multiple steps of the processes described herein, such as, for example, kneader systems available from Buss AG.

The pastille product can be provided in any suitable predetermined shape or form, and most preferably is provided in the form having a general shape of a pill, pellet, tablet, coin, bead, ovoid, obloid, cube, or the like. The mouthfeel of the pastille product preferably has a slightly chewable and dissolvable quality with a mild resilience or "bounce" upon chewing that gradually leads to greater malleability during use. According to one aspect, the pastille product is preferably capable of lasting in the user's mouth for about 10-15 minutes until it completely dissolves. Preferably, the products do not, to any substantial degree, leave any residue in the mouth of the user thereof, and do not impart a slick, waxy, or slimy sensation to the mouth of the user.

According to some embodiments, the pastille composition may be coated with a coating substance after being removed from the starch mold and prior to drying. For example, a glazing or anti-sticking coating substance, such as, for example, CAPOL 410 (available from Centerchem, Inc.), may be applied to the pastille composition to provide free-flowing properties. The coating composition may comprise an oil or wax; for example, sunflower oil and/or carnuba wax.

Outer coatings can also help to improve storage stability of the pastille products of the present disclosure as well as improve the packaging process by reducing friability and dusting. Devices for providing outer coating layers to the products of the present disclosure include pan coaters and spray coaters, and particularly include the coating devices available as CompuLab 24, CompuLab 36, Accela-Cota 48 and Accela-Cota 60 from Thomas Engineering. An example outer coating comprises a film-forming polymer, such as a cellulosic polymer, an optional plasticizer, and optional flavorants, colorants, salts, sweeteners or other additives of the types set forth herein. The coating compositions are usually aqueous in nature and can be applied using any pellet or tablet coating technique known in the art, such as pan coating. Example film-forming polymers include cellulosic polymers such as methylcellulose, hydroxypropyl cellulose (HPC), hydroxypropyl methylcellulose (HPMC), hydroxyethyl cellulose, and carboxy methylcellulose. Example plasticizers include aqueous solutions or emulsions of glyceryl monostearate and triethyl citrate.

In one embodiment, the coating composition comprises up to about 75% by weight of a film-forming polymer solution (e.g., about 40 to about 70% by weight based on total weight of the coating formulation), up to about 5% by weight of a plasticizer (e.g., about 0.5 to about 2% by weight), up to about 5% by weight of a sweetener (e.g., about 0.5 to about 2% by weight), up to about 10% by weight of one or more colorants (e.g., about 1 to about 5% by weight), up to about 5% by weight of one or more flavorants (e.g., about 0.5 to about 3 % by weight), up to about 2% by weight of a salt such as NaCl (e.g., about 0.1 to about 1 5 % by weight), and the balance water. Example coating compositions and methods of application are described in U.S. Application No. 12/876,785 to Hunt et al.; filed September 7, 2010, and which is incorporated by reference herein.

Although the foregoing description focuses on compositions that are uniform throughout each product unit, products can also be formed with multiple different formulations having different properties in the same product unit. For example, two different compositions can be deposited in a single mold to produce a layered product. Still further, two different compositions could be co-extruded to form a product with different characteristics across its cross-section. Such a process could be used to provide a product with two different compositions featuring different dissolution rates such that a first portion of the product dissolves at a first rate (e.g., a faster rate) and a second portion dissolves at a second, slower rate.

### Method of preparing tablet products

In some embodiments, the product is in the form of a compressed pellet or tablet. In one embodiment, the process for making the pellet or tablet involves first mixing the bulk filler (e.g., EMDEX^{®}) and the active ingredients. The remaining composition ingredients (e.g., sugar alcohol and any other desired components, such as binders, colorants, sweeteners, flavors, and the like) are then added. Optionally, a colorant can may be added to one of the composition components in a separate step prior to mixing with the remaining components of the composition. The mixing of the composition can be accomplished using any mixing device. The final composition is then compressed into pellet or tablet form using conventional tableting techniques and optionally coated. Compressed composition pellets can be produced by compacting the composition, including any associated formulation components, in the form of a pellet, and optionally coating each pellet with an overcoat material. Example compaction devices, such as compaction presses, are available as Colton 2216 and Colton 2247 from Vector Corporation and as 1200i, 2200i, 3200, 2090, 3090 and 4090 from Fette Compacting. Devices for providing outer coating layers to compacted pelletized compositions are available as CompuLab 24, CompuLab 36, Accela-Cota 48 and Accela-Cota 60 from Thomas Engineering.

When present, a coating typically comprises a film-forming polymer, such as a cellulosic polymer, an optional plasticizer, and optional flavorants, colorants, salts, sweeteners or other additives of the types set forth herein. The coating compositions are usually aqueous in nature and can be applied using any pellet or tablet coating technique known in the art, such as pan coating. Example film-forming polymers include cellulosic polymers such as methylcellulose, hydroxypropyl cellulose (HPC), hydroxypropyl methylcellulose (HPMC), hydroxyethyl cellulose, and carboxy methylcellulose. Example plasticizers include aqueous solutions or emulsions of glyceryl monostearate and triethyl citrate. Additional potential coatings include food grade shellac, waxes such as carnuaba wax, and combinations thereof.

### Methods of preparing lozenge products

The manners and methods used to formulate and manufacture a lozenge product as described herein above can vary. For example, the compositions can be prepared via any method commonly used for the preparation of hard boiled confections. Example methods for the preparation of hard confections can be found, for example, in LFRA Ingredients Handbook, Sweeteners, Janet M. Dalzell, Ed., Leatherhead Food RA (Dec. 1996), pp. 21-44, which is incorporated herein by reference.

Typically, a first mixture of ingredients is prepared. The composition of the first mixture of ingredients can vary; however, it typically comprises a sugar substitute and may contain various additional substances (e.g., the sugar alcohol syrup, NaCl, preservatives, further sweeteners, water, and/or flavourings). In certain embodiments, it comprises the sugar substitute, salt, and vanillin. In other embodiments, the first mixture comprises the sugar substitute and the sugar alcohol syrup. Typically, the first mixture of ingredients does not contain the active ingredients; although, it some embodiments, the active ingredients may be incorporated into the first mixture of ingredients.

The first mixture of ingredients is heated until it melts; subsequently, the mixture is heated to or past the hard crack stage. In confectionary making, the hard crack stage is defined as the temperature at which threads of the heated mixture (obtained by pulling a sample of cooled syrup between the thumb and forefinger) are brittle or as the temperature at which trying to mold the syrup results in cracking. According to the present method, the temperature at which the hard crack stage is achieved can vary, depending on the specific makeup of the product mixture but generally is between about 145°C and about 170°C. Typically, the mixture is not heated above about 171 °C, which is the temperature at which caramelisation begins to occur. In the processes of the present disclosure, the mixture is typically heated to the hard crack stage temperature or above and then allowed to cool. The heating can be conducted at atmospheric pressure or under vacuum. Typically, the method of the present invention is conducted at atmospheric pressure.

In one example embodiment, the first mixture of ingredients comprises a high percentage of isomalt and the mixture is heated to about 143°C. Once all components are dissolved, the temperature is raised past the hard crack stage (e.g., to about 166°C). The mixture is heated to this temperature and then removed from the heat to allow the mixture to cool.

In certain embodiments, the active ingredients and, optionally, additional components (e.g., additional sweeteners, fillers, flavouring agents, and water) as described above are separately combined in a second mixture. The second mixture is added to the first mixture of ingredients, typically after the first mixture of ingredients has been removed from the heat. The addition of the second mixture may, in some embodiments, occur only after the heated first mixture of ingredients has cooled to a predetermined temperature (e.g., in certain embodiments, to about 132 °C). In certain embodiments, one or more flavouring agents are added to the second mixture immediately prior to adding the mixture to the first, heated mixture of ingredients. Certain flavouring agents are volatile and are thus preferably added after the mixture has cooled somewhat. The combined mixture is then formed into the desired shape. In certain embodiments, the mixture is poured directly into molds, formed (e.g., rolled or pressed) into the desired shape, or extruded. If desired, the mixture can be extruded or injection molded. In certain embodiments, the mixture is formed or extruded into a mold of desired shape in an enclosed system, which may require decreased temperature and which may limit evaporation of certain mixture components. For example, such a system may limit the evaporation of volatile components including, but not limited to, flavorants. Other methods of producing lozenges are also intended to be encompassed herein.

Typical conditions associated with manufacture of food-grade lozenge products such as described herein include control of heat and temperature (i.e., the degree of heat to which the various ingredients are exposed during manufacture and the temperature of the manufacturing environment), moisture content (e.g., the degree of moisture present within individual ingredients and within the final composition), humidity within the manufacturing environment, atmospheric control (e.g., nitrogen atmosphere), airflow experienced by the various ingredients during the manufacturing process, and other similar types of factors. Additionally, various process steps involved in product manufacture can involve selection of certain solvents and processing aids, use of heat and radiation, refrigeration and cryogenic conditions, ingredient mixing rates, and the like. The manufacturing conditions also can be controlled due to selection of the form of various ingredients (e.g., solid, liquid, or gas), particle size or crystalline nature of ingredients of solid form, concentration of ingredients in liquid form, or the like. Ingredients can be processed into the desired composition by techniques such as extrusion, compression, spraying, and the like.

In certain embodiments, the lozenge product may be transparent or translucent. As used herein, "translucent" or "translucency" refers to materials allowing some level of light to travel therethrough diffusely. In certain embodiments, lozenge products of the present disclosure can have such a high degree of clarity that the material can be classified as "transparent" or exhibiting "transparency," which is defined as a material allowing light to pass freely through without significant diffusion. The clarity of the lozenge product is such that there is some level of translucency as opposed to opacity (which refers to materials that are impenetrable by light).

Transparency/translucency can be determined by any means commonly used in the art; however, it is commonly measured by spectrophotometric light transmission over a range of wavelengths (e.g., from about 400-700 nm). Alternatively, optical methods such as turbidimetry (or nephelometry) and colorimetry may be used to quantify the cloudiness (light scattering) and the color (light absorption), respectively, of the lozenge products provided herein. Translucency can also be confirmed by visual inspection by simply holding the material (e.g., extract) or product up to a light source and determining if light travels through the product in a diffuse manner.

### Method of preparing melt products

In some embodiments, the product is in meltable form. For preparation of meltable products, the lipid is typically heated to slightly above the melting temperature such that the lipid is liquefied. Optionally, active ingredients, flavoring agents, and/or lecithin can be added to the liquefied lipid at this stage. Thereafter, all or a portion of the liquefied lipid can be blended with the dry blend and mixed until the product reaches the desired level of homogeneity or until the desired textural properties are achieved. The mixture is milled (e.g., in a dry roll mill) until the particle size is less than about 20 microns. The milled isomalt-palm oil is combined with any remaining lipid, and the dry ingredients and flavor mixed in. The base is generally warmed to a fluid consistency.

In some embodiments, a sugar alcohol (e.g., isomalt) is added to a mixer bowl, and a portion of the total lipid (e.g., melted palm oil) is added, along with salt and emulsifier.

Additional lipid is added with mixing until adhesive clumps form. The clumped mixture is transferred portion-wise to a 3 roll mill and processed to a particle size of less than 50 microns, or about 20 microns. The refined mixture is transferred to a mixer bowl, and the remaining lipid added with mixing. The mixture is warmed as necessary to maintain a fluid consistency.

Sweetener, flavor, and active ingredient(s) are added with mixing. Mixing is continued until a homogenous composition is obtained. The mixture is allowed to rest for a period of time, such as about 10 to 15 minutes. The composition can be divided into discrete portions, such as by pouring the composition into a sheet-like structure, cooling, and then cutting the structure into individual portions, or by depositing the composition into molds and allowing to cool. The molds may be starch molds or starchless molds. In particular embodiments, the molds are starchless.

The melt composition may be held in the mold (starch or starchless) for a predetermined duration of time such as, for example, from about 1 to about 15 minutes, to allow the melt composition to cool and solidify. Optionally, the molds containing the melt product may be cooled by refrigeration to accelerate solidification.

According to other aspects of the present disclosure, rather than using molds to prepare the melt product, an extrusion process may be employed in which the final melt product is extruded as described herein above with respect to pastille extrusion methods.

### Methods of preparing pouched oral products

Where the product is in the form of a pouched oral product, the process may comprise the steps of:
(a) combining active ingredients;
(b) contacting the combination of active ingredients and at least one filler to provide the oral product.

The combination of active ingredients may be as described hereinabove.

In some embodiments, the step (b) comprises mixing the at least one filler and the combination of active ingredients. In some embodiments, the combination of active ingredients is in solid form (e.g. in the form of a powder). The combination of active ingredients may be mixed directly with the filler to provide the oral product. In some embodiments, the combination of active ingredients may be dissolved in a hydrophilic solvent (e.g. water and/or alcohol) prior to contacting the filler. For example, the combination of active ingredients may be dissolved in water or alcohol (e.g. ethanol or propylene glycol) before being mixed with the filler. The process may, in such embodiments, comprise the step of drying the product so as to remove the solvent. For example, the product may be dried via heating, freeze-drying, spray-drying, or simply leaving the product at room temperature for a certain period of time. Preferably, the drying step comprises leaving the product at room temperature for a period of 1 hour to 48 hours to remove the solvent.

The process may then further comprise the step of pouching the oral product using a pouch material as described hereinabove.

### EXAMPLES

Aspects of the present invention are more fully illustrated by the following examples, which are set forth to illustrate certain aspects of the present invention and are not to be construed as limiting thereof.

### Example 1 - Radioligand binding assays of adenosine A1 and A2A receptors

### Materials and Methods

*Materials*

| | **Code** | **Supplier** | **Solvent** |
|---|---|---|---|
| CCPA (2-Chloro-N6cyclopentyladenosine) | C7938 | SigmaAldrich (Taufkirchen, Germany) | H₂O_{Milli-Q} |
| DPCPX (8-Cyclopentyl-1,3-dipropylxanthine) | C101 | SigmaAldrich (Taufkirchen, Germany) | DMSO |
| Caffeine (1,3,7-Trimethylxanthine0 | C0750 | SigmaAldrich (Taufkirchen, Germany) | |

| | | | |
|---|---|---|---|
| Flavone | F2003 | SigmaAldrich (Taufkirchen, Germany) | H₂O_{Milli-Q} |
| Genistein | BS0082 | Biotrend (Cologne, Germany) | DMSO |
| ZM 241385 (4-(-2-[7-amino-2-{2-furyl}{1,2,4}triazolo{2,3-a}{1,3,5}triazin-5-yl-amino]ethyl)phenol) | 1036 | Tocris (Bio-Techne GmbH, Wiesbaden-Nordenstedt, Germany) | DMSO |
| Galangin | 15948 | Cayman Chemical (Ann Arbor, USA) | DMSO |
| Hispidulin | 26383 | Cayman Chemical (Ann Arbor, USA) | DMSO |
| Quercetin (hydrate) | 10005169 | Cayman Chemical (Ann Arbor, USA) | DMSO |
| Apigenin | 10010275 | Cayman Chemical (Ann Arbor, USA) | DMSO |
| Naringenin | 14173 | Cayman Chemical (Ann Arbor, USA) | DMSO |
| Kaempferol | 11852 | Cayman Chemical (Ann Arbor, USA) | DMSO |
| Cirsimaritin | FC69723 | Biosynth (Carbosynth, Compton, Great Britain) | DMSO |
| Scutallarein tetramethyl ether | FT75183 | Biosynth (Carbosynth, Compton, Great Britain) | DMSO |

Stock solutions (10 mM) were prepared with the solvents indicated and aliquots stored frozen at -80°C. All further dilutions were done in H₂O_{Milli-Q}.

Human adenosine A1 receptors (code: ES-010-M400UA, Lot: 2399756) and human adenosine A_{2A} receptors (code: RBHA2AM400UA) were from PerkinElmer (Rodgau, Germany). [³H]-DPCPX (code: HART-0424) was from Hartmann Analytic (Braunschweig, Germany). ZM 241385 (code: 1036) was from Tocris (Bio-Teche GmbH, Wiesbaden, Germany). ZM 241385 was iodinated with ¹²⁵I by standard methods and purified by HPLC.

All other chemicals were purchased either from SigmaAldrich or VWR.

### Adenosine A 1 receptor binding method

Binding experiments were performed according to the data sheet provided by the supplier of the receptor (Perkin Elmer) with modifications:

| ligand | conc. | non specific | incubation | detection method |
|---|---|---|---|---|
| [³H]-DPCPX | 1 nM | DPCPX (10 µM) | 60 min | scintillation counting |
| | | | 27°C | |

Approximately 2.4-3 µg protein-containing membranes were incubated for 60 min in a total volume of 100 µL at 27°C:
assay buffer:

| | |
|---|---|
| 25 mM | Hepes-NaOH (pH 7.4) |
| 100 mM | NaCl |
| 5 mM | MgCl₂ |
| 1 mM | CaCl₂ |

The assay was terminated by transfer of the samples on GF/B filter plates (Millipore), presoaked in 0.1% PEI. Filters were washed five times with 200 µL of ice-cold 50 mM Tris-HCl pH 7.4 / 0.5 M NaCl and filter-bound radioactivity was determined by a microplate reader (Microbeta, Wallac, Finland).

The assay was validated with the reference compound DPCPX, which is a selective and potent A1 receptor antagonist that competes for the binding of [³H]-DPCPX to membranes of CHO-K1 cells expressing the human adensoine A1 receptor in a concentration dependent manner. The bound radioactivity in the presence of 10 µM DPCPX was defined as non-specific binding of the radioligand.

### Adenosine A2A receptor binding

Binding experiments were performed as published by Palmer et al. (1995) Mol. Pharmacol. 48, 970-974, with modifications:

| Ligand | conc. | non specific | incubation | detection method |
|---|---|---|---|---|
| [¹²⁵I]-ZM241385 | 20000 cpm | ZM241385 (1 µM) | 60 min | scintillation counting |
| | | | 37°C | |

Approximately 0.4-0.9 µg protein-containing membranes were incubated for 60 min in a total volume of 100 µL at 37°C.
assay buffer:

| | |
|---|---|
| 50 mM | Hepes-NaOH (pH 6.8) |
| 10 mM | MgCl₂ |

The assay was terminated by transfer of the samples on GF/B filter plates (PE), presoaked in 0.05 % Pluronic F127. Filters were washed four times with 200 µL of ice-cold 50 mM Tris-HCl pH 7.4, 0,5 M NaCl and filter-bound radioactivity was determined by a microplate reader (Microbeta, Wallac, Finland).

The assay was validated using the potent and highly selective A2A adenosine antagonist ZM 241385, which competes for the binding of [¹²⁵I]-ZM 241385 to membranes of HEK293 cells expressing the human adenosine A2A receptor in a concentration dependent manner. The bound radioactivity in the presence of 1 µM) ZM 241385 was defined as non-specific binding of the radioligand.

### Data analysis and expression of results

The data of the reference compounds (subsections: assay validations) are presented as total bound radioactivity (in cpm).

Test compound data are presented as specific radioligand binding to the receptor. Specific binding is defined as the difference between the total binding and the non-specific binding determined in the presence of the reference compound as detailed in the previous sections.

The IC50 values (concentration causing half-maximal inhibition of control specific binding) were determined by non-linear regression analysis of the competition curves using the algorithm "sigmoidal dose-response" (GraphPadPrism, San Diego, USA). In case of ill-defined curves or algorithm-generated minima below the defined non-specific binding, the IC50 value was determined by graphical extrapolation.

### Results - Adenosine A1 receptor binding assay

The determined IC50 values by the Adenosine A1 receptor binding assay are summarised in the following Table 2:

**Table 2. IC50 values determined by Adenosine A1 receptor binding assay**

| | **1.M** | **2.M** | **Mean** |
|---|---|---|---|
| CCPA ¹ | 77 nM | 100 nM | **98 nM** |
| DPCPX ² | 10 nM | 10 nM | **10 nM** |
| ZM 241385 ³ | 3.4 µM | 1.7 µM | **2.6 µM** |
| Caffeine | 93 µM | 60 µM | **77 µM** |
| Galangin | 6.2 µM | 5.1 µM | **5.7 µM** |
| Hispidulin | 34 µM | 36 µM | **35 µM** |
| Quercetin | 7.2 µM | 4.1 µM | **5.7 µM** |
| Apigenin | 32 µM | 45 µM | **39 µM** |
| Flavone | 16 µM | 16 µM | **16 µM** |
| Naringenin | 27 µM | 32 µM | **30 µM** |
| Genistein | 25 µM | 25 µM | **25 µM** |
| Kaempferol | 13 µM | 12 µM | **13 µM** |
| Cirsimaritin ⁴ | 617 µM | 122 µM | **1.2 µM** |
| Tetramethylscutallarein | 4.8 µM | 9.3 µM | **7.1 µM** |

| | | | |
|---|---|---|---|
| ¹ Reference compound: 2-Chloro-N6cyclopentyladenosine ² Reference compound: 8-Cyclopentyl-1,3-dipropylxanthine ³ Reference compound: 4-(-2-[7-amino-2-{2-furyl}{1,2,4}triazolo{2,3-a}{1,3,5}triazin-5-yl-amino]ethyl)phenol ⁴ Literature value determined in rat - Ji,X.D., Melman,N. & Jacobson,K.A. Interactions of flavonoids and other phytochemicals with adenosine receptors. J Med. Chem. 39, 781-788 (1996). | | | |

As can be seen from Table 2, the compounds according to the present disclosure were found to have at least comparable affinities for the Adenosine A1 receptor when compared to caffeine.

### Results - Adenosine A2A receptor binding assay

The determined IC50 values by Adenosine A2A receptor binding assay are summarised in the following Table 3:

**Table 3. IC50 values determined by Adenosine A2A receptor binding assay**

| | **1.M** | **2.M** | **Mean** |
|---|---|---|---|
| CCPA ¹ | 512 nM | 426 nM | **470 nM** |
| DPCPX ² | 56 nM | 31 nM | **44 nM** |
| ZM 241385 ³ | 6 nM | 3.8 nM | **5 nM** |
| Caffeine | 7.2 µM | 6.8 µM | **7 µM** |
| Galangin | 0.6 µM | 0.83 µM | **0.7 µM** |
| Hispidulin | 1.8 µM | 3.2 µM | **2.5 µM** |
| Quercetin | 0.6 µM | 1.1 µM | **0.9 µM** |
| Apigenin | 32 µM | 34 µM | **33 µM** |
| Flavone | 4.1 µM | 5.6 µM | **4.9 µM** |
| Naringenin | 30 µM | 23 µM | **27 µM** |
| Genistein | 3.7 µM | 5.3 µM | **4.5 µM** |
| Kaempferol | 4.8 µM | 3.5 µM | **4.2 µM** |
| Cirsimaritin | 1.3 µM | 2.1 µM | **1.7 µM** |
| Tetramethylscutallarein | 1.9 µM | 1.3 µM | **1.6 µM** |

| | | | |
|---|---|---|---|
| ¹ Reference compound: 2-Chloro-N6cyclopentyladenosine ² Reference compound: 8-Cyclopentyl-1,3-dipropylxanthine ³ Reference compound: 4-(-2-[7-amino-2-{2-furyl}{1,2,4}triazolo{2,3-a}{1,3,5}triazin-5-yl-amino]ethyl)phenol | | | |

As can be seen from Table 3, the compounds according to the present disclosure were found to have generally comparable affinities for the Adenosine A2A receptor when compared to caffeine.

The results of the receptor binding assays confirm that compounds according to the present disclosure have at least comparable affinities for the Adenosine A1 and Adenosine A2A receptors. In order to supplement this finding with information on the pharmacology of said compounds against said receptors, e.g. agonism and/or antagonism, receptor functional assays were developed and tested as described in the following Example 2.

### Example 2 - Investigation of test compounds in A1 and A2A adenosine receptor functional assays

### Materials and Methods

### Materials

Compounds, solvents and supplier details are as already detailed above for Example 1.

Stock solutions (10 mM) were prepared with the solvents indicated and aliquots stored frozen at -80°C. All further dilutions were done in H₂O_{Milli-Q}.

GTP[γ-³⁵S] (Code: NEG030H, specific activity: 1250 Ci/mmol), human adenosine A1 receptors (code: ES-010-M400UA, Lot: 2399756) and human adenosine A2A receptors (code: RBHA2AM400UA) were from PerkinElmer (Rodgau, Germany). ZM 241385 (code: 1036) was from Tocris (Bio-Teche GmbH, Wiesbaden, Germany). CGS 21680 (code: C141, DMSO), DPCPX (8-Cyclopentyl-1,3-dipropylxanthine, code: C101, DMSO) and CCPA (2-Chloro-N6cyclopentyladenosine, code: C7938) were from SigmaAldrich (Taufkirchen, Germany).

All other chemicals were purchased either from SigmaAldrich or VWR.

### Rat striatal membrane preparation

Male Sprague Dawley rats were sacrificed by decapitation, rat striata were dissected on ice, frozen in liquid nitrogen and stored at -80°C. The tissue was homogenized (20 strokes with a glass-teflon homogenizer, 2000 rounds per minute) at 4°C in 10 mL of 10 mM Tris-HCl pH 7.4, 1 mM EDTA, and centrifuged for 15 min at 25,000 x g. The centrifugation step was repeated twice and the pellet finally resuspended in 50 mM Tris-HCl pH 7.4, 4 mM MgCl₂ and 1 mM EDTA, frozen in liquid nitrogen and stored at-80°C until usage.

### [³⁵S]-GTPγS binding assay

### Assay conditions I

[³⁵S]-GTPγS-binding assays as well as the initial assay optimization/adapation was in principle carried out as described (Bidlack and Parkhill, Methods Mol Biol 237, 135-143 (2004)):

| radioligand | conc. | non-specific | incubation | detection method |
|---|---|---|---|---|
| [³⁵S]GTPγS | 200 pM | GTPγS (10 µM) | 60 min 30°C | scintillation counting |

Membranes were preincubated with the respective effectors for 15 min, the incubation was initiated by the addition of 200 pM [³⁵S]-GTPγS (60 min incubation at 30°C in a final volume of 200 µL assay buffer).
assay buffer:

| | |
|---|---|
| 20 mM | HEPES-NaOH pH 7.4 |
| 100 mM | NaCl |
| 10 mM | MgCl₂ |
| 2 mM | EGTA |
| 10 µM | GDP |
| 10 µg/mL | Saponin |

The assay was terminated by transfer of the samples on GF/C filter plates (PerkinElmer). Filters were washed four times with 200 µL of ice-cold 50 mM Tris-HCl pH 7.4 and bound radioactivity was determined by a microplate reader (Microbeta, Wallac, Finland).

Assay conditions I were used for initial optimisation of the ratio of specific to non-specific binding of the compounds. Assay conditions II were used for all subsequent work.

### Assay conditions II

[³⁵S]-GTPγS-binding assays as well as the initial assay optimization/adapation was in priciple carried out as described (Gilliland et al., Eur J Pharmacol 392, 125-128 (2000)):

| radioligand | conc. | non-specific | incubation | detection method |
|---|---|---|---|---|
| [³⁵S]GTPγS | 200 pM | GTPγS (10 µM) | 60 min 30°C | scintillation counting |

Membranes were preincubated with the respective effectors for 15 min, the incubation was initiated by the addition of 200 pM [³⁵S]-GTPγS (60 min incubation at 30°C in a final volume of 200 µL assay buffer).
assay buffer:

| | |
|---|---|
| 20 mM | HEPES-NaOH pH 7.4 |
| 100 mM | NaCl |
| 3 mM | MgCl₂ |
| 1 mM | DTT |
| 300 µM | GDP |

The assay was terminated by transfer of the samples on GF/C filter plates (PerkinElmer). Filters were washed four times with 200 µL of ice-cold 50 mM Tris-HCl pH7.4 and bound radioactivity was determined by a microplate reader (Microbeta, Wallac, Finland).

### Data analysis and expression of results

From every data point the non-specific binding (binding of [³⁵S]-GTPγS in the presence of 10 µM GDP) was subtracted and the value-normalized to the respective control signal of the respective plate. For CCPA and CGS 21680 agonist mode results, all data points were normalized to the basal signal (signal in the absence of any effector = 100%, agonist mode). For DPCPX antagonist mode results in the A1 assay, all data points were normalized to the maximal stimulation of the reference agonist (= 0% change, antagonist mode and -100% change: basal signal - signal in the absence of any effector).

Because of effects of the test compounds on the basal signal (see Discussion below), a different method was chosen to present the test compound data. Namely, concentration-response curves of the test compound extracts in the agonist and antagonist mode were normalized to 0% change. In the agonist mode 0% change corresponds to the level of the basal signal; in the antagonist mode 0% change corresponds to the maximal stimulation of the receptor by the reference agonist. In the agonist mode -100% change correponds to 0% specific binding and in the antagonist mode to the basal signal (specific signal without any effector added).

EC50 and IC50 values (concentration causing half-maximal stimulation/inhibition of control specific binding) were determined by non-linear regression analysis of the competition curves using the algorithm "sigmoidal dose-response" (GraphPadPrism, San Diego, USA). In case of ill-defined curves, the IC50 was determined by graphical exploration of the algorithm-generated curve.

### The [³⁵S]-GTPγS-binding assay- principles

The [³⁵S]-GTPγS-binding assay is a functional test which measures the activation of G protein coupled receptors (GPCRs). The extracellular binding of an agonist to the GPCR leads to a conformational change of the receptor triggering the activation of intracellular G proteins. The first step in the activation process of a G protein in the exchange of bound GDP to GTP. If radioactive [³⁵S]-GTPγS is used instead of GTP, this activation process can be monitored by conventional receptor binding assay methods.

### The agonist mode

In principle, the [³⁵S]-GTPγS-binding assay always measures the activation state of all GPCRs present in the homogenates. Even without addition of any effector some GPCRs are activated leading to the basal signal of G protein activation. One reason for effector-independent activation is constitutive activity of receptors, another reason is the putative biological activity of membrane preparations (so could e.g. effectors/agonists be generated *in situ* by endogenous enzymes of the membrane preparations). A third reason might be that the membrane preparations themselves contain endogenous agonists.

### Increase of the basal signal by test compounds

If a test compound increases the basal signal in a concentration-dependent manner, the test compound has an agonistic action on at least one GPCR present in the membrane. If the test is performed on membrane preparations containing recombinant receptors, it is very likely that the test compound is an agonist to the recombinant receptor. The receptor specific action is proven if co-incubation with a receptor-specific antagonist reduces the signal in a concentration-dependent manner. Likewise, if a receptor-specific reference compound increases the basal signal in a concentration-dependent manner, it is indicated that this receptor is present in the membrane preparation.

### Decrease of the basal signal by test compounds

If a test compound decreases the basal signal in a concentration-dependent manner, several interpretations are possible: (i) The test compound has an antagonistic action on at least one GPCR present in the membrane preparation displaying constitutive activity. Constitutive activity at the investigated receptor is proven, if a reference antagonist reduces the basal signal in a concentration-dependent manner (see also Discussion below); or (ii) The test compound has receptor-independent effects on the assay system, e.g. by interfering with/destroying the membrane integrity or prohibiting the process of G protein activation.

### The antagonist mode

This method is especially suitable to identify antagonistic actions of test compounds. A receptor is maximally pre-stimulated by a receptor-specific agonist (meaning all G proteins coupled to this receptor are maximally activated). If the addition of a test compound decreases the control binding in a concentration-dependent manner, the standard interpretation would be that the test compound has an antagonistic action at the pre-stimulated receptor. But this interpretation is only valid if the test compound does not alter the basal signal, or in the case of constitutive receptor activity, the test compound and a reference antagonist change the basal signal to a similar degree. If a test compound inhibits the basal signal (agonist mode) and the stimulated signal (antagonist mode) to a similar extent, it needs to be concluded that the test compound does not have any receptor-specific effects within the detection limit.

### Detection limit under general inhibition conditions

In separate experiments in which test compounds which lead to a general inhibition were spiked with different concentrations of reference agonists and antagonists (data not shown), it was shown that the detection limit for receptor agonists is better than 10 times the concentration leading to half-maximal general inhibition (10 x IC50). If a receptor-specific agonist is present in a test compound mixture the inhibition curve in the agonist mode becomes biphasic and the IC50 is higher than in the antagonist mode. Possible receptor-specific agonistic effects of a test compound mixture with EC50s 10x or lower than 10x the IC50 of the general inhibition are detected. On the other hand, agonistic effects with EC50s higher than 10x the IC50 are masked by the general inhibition.

Similarly, receptor-specific antagonistic actions of a test compound mixture are masked above the detection limit of one tenth of the IC50 of the general inhibition (0.1 × IC50). If a receptor-specific antagonist is present in a test compound mixture the IC50 in the antagonist mode is lower than in the agonist mode.

### Constitutive activity at the adenosine receptors

Both reference antagonists (DPCPX and ZM-241385) were found to reduce the basal signal by about 25%. The standard explanation would conclude a constitutive activation of adenosine receptors in these membranes (Laitinen, Neuroscience 90, 1265-1279 (1999)). In case of the adenosine receptor this basal signal is probably not caused by a constitutive activity of the adenosine receptor, but rather by tonic activation via endogenous adenosine and a cryptic adenosine deaminase (ADA)-resistent pool (Savinainen et al., Br J Pharmacol 140, 1451-1459, (2003)).

### A1 validation

The potent and selective adenosine A1 receptor agonist CCPA stimulated the [³⁵S]-GTPyS binding to human adenosine A1 receptors in a concentration-dependent manner (agonist mode). The EC50 value for CCPA-stimulated [³⁵S]-GTPγS binding was 2.2 nM. In this experiment a maximal stimulation of 328% was detected. The selective A1 antagonist DPCPX reduced the CCPA-evoked signal (100 nM CCPA, =0% change) in a concentration-dependent manner to the level of the basal signal (no effector added, -100% change, antagonist mode). The IC50 value for the inhibition of CCPA-stimulated [³⁵S]-GTPγS binding by DPCPX in this experiment was 56 nM

It is concluded that the developed [³⁵S]-GTPγS binding assay is suitable to identify adenosine receptor A1 agonists in the agonist mode (an agonistic action of a test compound is identified by a concentration-dependent increase of the bound [³⁵S]-GTPγS) and antagonists in the antagonist mode (an antagonistic action of a test compound is identified by a concentration-dependent decrease of the CCPA-evoked signal to the level of the basal signal).

### A2A validation

The adenosine A2A receptor agonist CGS 21680 stimulated the [³⁵S]-GTPγS binding to adenosine receptors in rat striatum in a concentration-dependent manner (agonist mode). The EC50 value for CGS 21680-stimulated [³⁵S]-GTPγS binding was 10 µM. In this experiment a maximal stimulation of 248% was detected.The potent and highly selective A2a antagonist ZM 241385 reduced the CGS 21680-evoked signal in a concentration-dependent manner to the level of the basal signal (no effector added, -100% change, antagonist mode) and below. The IC50 value for the inhibition of CGS 21680-stimulated [³⁵S]-GTPγS binding by ZM 241385 in this experiment was 33 nM.

It is concluded that the developed [³⁵S]-GTPγS binding assay is suitable to identify adenosine receptor agonists in the agonist mode (an agonistic action of a test compound is identified by a concentration-dependent increase of the bound [³⁵S]-GTPγS) and antagonists in the antagonist mode (an antagonistic action of a test compound is identified by a concentration-dependent decrease of the CGS 21680-evoked signal to the level of the basal signal).

### Summary of Results

A summary of IC50 values determined in the Adenosine A1 receptor functional assay is shown in Table 4.

**Table 4. Summary of Results from Adenosine A1 Receptor Functional Assay**

| **Test compound** | | **IC₅₀ in µM** | |
|---|---|---|---|
| | | **agonist mode** | **antagonist mode** |
| Caffeine | 1.Measurement | >1000 | 250 |
| | 2.Measurement | >1000 | 310 |
| Galangin | 1.Measurement | 10 | 7 |
| | 2.Measurement | 12 | 3 |
| Hispidulin | 1.Measurement | 50 | 40 |
| | 2.Measurement | 40 | 20 |
| Quercetin | 1.Measurement | 20 | 5 |
| | 2.Measurement | 150 | 8 |
| Flavone | 1.Measurement | 20 | 4 |
| | 2.Measurement | 90 | 8 |
| Genistein | 1.Measurement | 180 | 40 |
| | 2.Measurement | 80 | 30 |
| Kaempferol | 1.Measurement | 140 | 20 |
| | 2.Measurement | 210 | 20 |
| Tetramethylscutallarein | 1.Measurement | 2 | 9 |
| | 2.Measurement | 7 | 13 |
| DPCPX¹ | 1.Measurement | | 76 |
| | 2.Measurement | | 30 |

| | | | |
|---|---|---|---|
| ¹ Reference compound: 8-Cyclopentyl-1,3-dipropylxanthine | | | |

A summary of IC50 values determined in the Adenosine A2A receptor functional assay is shown in Table 5.

**Table 5: Summary of Results from Adenosine A2A Receptor Functional Assay**

| **Test compound** | | **IC₅₀ in µM** | |
|---|---|---|---|
| | | **agonist mode** | **antagonist mode** |
| Caffeine | 1.Measurement | >1000 | 80 |
| | 2.Measurement | >1000 | 9 |
| Galangin | 1.Measurement | 80 | 0.7 |
| | 2.Measurement | >316 | 0.4 |
| Hispidulin | 1.Measurement | >1000 | 1.9 |
| | 2.Measurement | >316 | 6.0 |
| Quercetin | 1.Measurement | 90 | 1.0 |
| | 2.Measurement | >316 | 4.0 |
| Flavone | 1.Measurement | 80 | 1.0 |
| | 2.Measurement | >316 | 6.0 |
| Genistein | 1.Measurement | 620 | 3.0 |
| | 2.Measurement | >316 | 1.0 |
| Kaempferol | 1.Measurement | >316 | 1.6 |
| | 2.Measurement | 70 | 1.0 |
| Tetramethylscutallarein | 1.Measurement | >1000 | 0.3 |
| | 2.Measurement | >316 | 0.9 |

### Discussion

### Functional Adenosine A2A Assay

Adenosine A1 receptors couple to G_{i/o} proteins (Nanoff et al., Mol Pharmacol 48, 806-817 (1995)), Adenosine A2A receptors couple to Gₛ proteins (Laitinen, Neuroscience 90, 1265-1279 (1999)). Both signal transduction pathways are in principle detectable by the GTPγS technique. In this project, the the use of rat striatal membranes was found to be more favourable than the use of human recombinant adenosine A2A receptors. While the results with human recombinant adenosine A2A receptors cannot be interpreted as adenosine A2A data, they did reveal interesting observations that are discussed below (this assay is referred to herein as the "human recombinant A2A assay").

The rat striatum is known for having a high density of adenosine A1 and A2A receptors. Under the conditions detailed above, CGS 21680 was found to be stimulatory, with the signal being inhibited in a concentration-dependent manner by ZM 241385 (a potent and highly selective A2a antagonist) with an IC50 of 33 nM (see results of validation above). While the IC50 value for the inhibition by ZM 241385 of the CGS 21680 signal is approximately in the expected range, the EC50 value of CGS 21680 appears very high. There are reports that CGS 21680 has also some affinity for the adenosine A1 receptor (Casadá et al., J Neurochem 114, 972-980 (2010); Hide et al., Mol Pharmacol 41, 352-359 (1992)). On the other hand, it is well-known that different assay conditions favour different pathways in the [³⁵S]-GTPγS-binding assay (DeLapp et al., GTPgammaS Binding Assays, (2012); PMID: 22553877). Interestingly, the assay system is much less disturbed by non-specific interactions and is in good accordance with published data (Hide et al., Mol Pharmacol 41, 352-359 (1992)) who found an IC50 value of about 43 µM for caffeine for a CGS21680 stimulated signal and an EC50 for CGS21680 of 0.3 µM at the rat adenosine A2A receptor in an adenylate cyclase assay.

### Discussion of Findings

Due to the shortcomings of the the human recombinant A2A assay, and the likelihood that the rat striatal membrane assay also targets the (native, rat) adenosine A1 receptor, it is not clear whether any of the antagonists display selectivity between subtypes, for which an adenylate cyclase assay would be conclusive. Nonetheless, from the results herein, all test compounds investigated in this study have to be classified as adenosine receptor antagonists. Further discussion of the findings for specific compounds are included below.

### (i) Caffeine

The action of caffeine seems to be very "clean"; the basal signal is reduced by about 25% by caffeine as observed for the pure antagonists, therefore caffeine seems to have no antagonistic effect on any other receptors that contribute to the basal signal. In contrast to the rat striatum A2A assay, caffeine had no effect in the human recombinant A2A assay.

### (ii) Galangin/Tetramethylscutallarein/Hispidulin

Both galangin and tetramethylscutallarein affect the basal signal and the stimulated signal in the human A1 assay with affinities below 10 µM. The basal signal in the human recombinant A2A assay is affected with EC50 values around 300 µM. In the rat assay, tetramethylscutallarein has almost no effect on the basal signal, while the effect of galangin is above 80 µM. Since both compounds display an at least 100-fold difference between the agonist and antagonist mode in the rat adenosine assay (which is not so much influenced by the cellular background), both compounds can be classified as adenosine A1 antagonist with affinities in the low µM range. Both compounds have strong antagonistic effects (also in the low µM range) on at least one receptor displaying constitutive activity in CHO-K1 cells; the effects on consititutively active receptors in HEK293 cells are moderate.

Hispidulin displays a small difference between the agonist mode and the antagonist mode in the human adenosine A1 assay (50 µM vs 40 µM, respectively), in HEK293 cells the effect on the background is moderate (around 400 µM). In the rat adenosine assay the difference is at least 500-fold, therefore hispudulin has to be classified as an antagonist to the adensosine A1 receptor as well.

### (iii) Flavone/Quercetin

Both compounds affect the basal signal in the human A1 assay with affinities around 20 µM, the basal signal in the human recombinant A2A assay is affected with EC50 values around 200 µM and in the rat assay with IC50 values around 80 µM. Because of the very strong effects especially in the rat adenosine assay (80-90 fold over basal), both compounds have to be classified as adenosine receptor antagonists.

### (iv) Genistein/Kaempferol

Both compounds display a reasonable difference (5-7 fold) between the agonist and the anatagonist mode in the adenosine A1 assay and an over 200-fold difference in the rat adenosine assay. which is not so prone to receptor background. Therefore both compounds have to be classified as antagonists of the adenosine A1 receptor.

In summary, and similar to caffeine, all test compounds according to Formula (I) investigated in this study have to be classified as antagonists of adenosine receptors.

### Example 3 - Transcriptomics studies

To investigate whether compounds according to Formula (I) might be associated with additional physiological effects other than those associated with adenosine receptor antagonism, transcriptomics studies were performed.

Cells from human neuroblastoma cell line SHSY5Y were treated with 20 nM of each of the compounds in cell culture media for a 24 hr incubation under normal cell culture conditions. A dose-response study had been carried out to identify the most suitable concentration (i.e. 20 nM) as used for the transcriptomics study. DMSO was used as control treatment. Total RNA was isolated using the Qiagen RNAeasy kit according to the manufacturer's protocols and sequenced using short-read Illumina sequencing. The experiment was repeated in three separate biological experiments. Libraries were prepared with the NEBNext Ultra II Directional RNA Library Prep Kit for Illumina protocol according to supplier recommendations.

Briefly, the key stages of this protocol are successively: the purification of polyA-containing mRNA molecules using poly-T oligo-attached magnetic beads from 100 ng total RNA (using the Magnetic mRNA Isolation Kit from NEB), a fragmentation using divalent cations under elevated temperature to obtain approximately 300 bp pieces, double-stranded cDNA synthesis, and finally Illumina adapter ligation and cDNA library amplification by PCR for sequencing. Sequencing was then carried out on paired-end 100 b using the NovaSeq 6000 system. (NovaSeq Control Software V1.7.5). Image analysis and base calling was performed using Illumina Real Time Analysis with default parameters.

Raw data was mapped to reference human genome using STAR pipeline (1) for alignment and quantification of transcripts. Differential gene expression analysis was carried out on read count data using Deseq2 package in R (2). Gene set enrichment analysis was carried out using R package on differentially expressed gene (DEG) data using R package clusterProfiler (3,4) to understand biological, molecular and KEGG processes affected with compound treatment. Comparison was made based on treatment with a compound according to Formula (I) vs control (DMSO) and/or treatment with a compound according to Formula (I) vs caffeine. Based on this analysis, common themes were identified as summarised in Table 6. Gene Set Enrichment Analysis (GSEA) was used to interpret gene expression data by focussing on groups of genes that share common biological functions, chromosomal locations, or regulation (5). Genome-wide expression profiles were analysed and genes were ranked based on the correlation between their expression and the treatment compound used in samples belonging to two comparative groups (e.g. caffeine vs treatment; DMSO vs treatment). These rankings were further analysed to understand differences in biological function attributed to gene groups and subsequent effects on cell function and response by treatment with compounds according to the present disclosure.

**Table 6. Common themes identified from gene set enrichment analysis of DEG data.**

| **Compound** | **Comparison** | **Effect** |
|---|---|---|
| Flavone | Caffeine vs Treatment | Suppression of myofibril and contractile fiber |
| Flavone | Caffeine vs Treatment | Activation of carbohydrate digestion and absorption |
| Flavone | DMSO vs Treatment | Activation of positive regulation of receptor catabolic process |
| Flavone | DMSO vs Treatment | Activation of carbohydrate digestion and absorption |
| Genistein | Caffeine vs Treatment | Suppression of myofibril, contractile fiber, muscle contraction and muscle system process. Activation of midbrain dopaminergic neurin differentiation |
| Genistein | Caffeine vs Treatment | Suppression of vasopressin-regulated water reabsorption |
| Genistein | DMSO vs Treatment | Suppression of negative regulation of smooth muscle cell differentiation |
| Genistein | DMSO vs Treatment | Suppression of neuroactive ligand-receptor interaction |
| Hispdulin | Caffeine vs Treatment | Suppression of blood circulation, muscle contraction. Activation of negative regulation of muscle cell apoptosis |
| Hispidulin | Caffeine vs Treatment | Suppression of adrenergic signaling in cardiomocytes |
| Hispidulin | DMSO vs Treatment | Suppression of renin-angiotensis sytem, renin secretion |
| Kaempferol | Caffeine vs Treatment | Suppression of cardiac myofibril, slow-twitch skeletal muscle fiber contraction, actin-myosin filament sliding |
| Kaempferol | Caffeine vs Treatment | Suppression of cortisol synthesis and secretion, adrenergic signaling in cardiomyocytes, cardiac muscle contraction. Activation of carbohydrate digestion and absorption. |
| Kaempferol | DMSO vs Treatment | Suppression of renin-angiotensis sytem |
| Naringenin | Caffeine vs Treatment | Suppression of muscle contraction |
| Naringenin | Caffeine vs Treatment | Suppression of adrenergic signaling in cardiomocytes |
| Naringenin | DMSO vs Treatment | Suppression of dopaminergic synapse |

As can be seen from Table 6, compounds according to Formula (I) were found to be most frequently associated with the suppression of genes associated with muscle contraction and other muscle-associated pathways. Without wishing to be bound by theory, it is believed that such profiles may be associated e.g. with reduced muscle twitching/spasm etc., which in turn may contribute to e.g. reduced feelings of jitteriness in subjects and/or increased feelings of relaxation. Accordingly, the adenosine receptor antagonism demonstrated in Examples 1 and 2 and the findings of the transcriptomics study of Example 3 are supportive of the compounds of Formula (I) according to the present disclosure providing advantageous stimulatory effects similar to caffeine while avoiding or reducing other undesirable effects such as feelings of jitteriness. Several compounds were further found to be associated with effects on carbohydrate metabolism, e.g. activation of carbohydrate digestion and absorption. Accordingly, the compounds of Formula (I) according to the present disclosure may also be associated with e.g. improved physical and/or mental energy as explained in more detail in the various aspects and embodiments described herein above.

### References

'Dobin A, Davis CA, Schlesinger F, Drenkow J, Zaleski C, Jha S, Batut P, Chaisson M, Gingeras TR. "STAR: ultrafast universal RNA-seq aligner." Bioinformatics. 2013 Jan 1;29(1):15-21. doi: 10.1093/bioinformatics/bts635. Epub 2012 Oct 25. PMID: 23104886; PMCID: PMC3530905.
²Love Ml, Huber W, Anders S (2014). "Moderated estimation of fold change and dispersion for RNA-seq data with DESeq2." Genome Biology, 15, 550. doi:10.1186/s13059-014-0550-8.
³Wu T, Hu E, Xu S, Chen M, Guo P, Dai Z, Feng T, Zhou L, Tang W, Zhan L, Fu x, Liu S, Bo X, Yu G (2021). "ClusterProfiler 4.0: A universal enrichment tool for interpreting omics data." The Innovation, 2(3), 100141. doi:10.1016/j.xinn.2021.100141.
⁴Yu G, Wang L, Han Y, He Q (2012). "ClusterProfiler: An R Package for Comparing Biological Themes Among Gene Clusters." OMICS: A Journal of Integrative Biology, 16(5), 284-287. doi: 1 0.1 089/omi.2011.0118.
⁵Subramanian A, Tamayo P, Mootha VK, Mukherjee S, Ebert BL, Gillette MA, Paulovich A, Pomeroy SL, Golub TR, Lander ES, Mesirov JP. "Gene set enrichment analysis: a knowledge-based approach for interpreting genome-wide expression profiles." Proc Natl Acad Sci USA. 2005 Oct 25;102(43):15545-50. doi: 10.1073/pnas.0506580102. Epub 2005 Sep 30. PMID: 16199517; PMCID: PMC1239896.

The various embodiments described herein are presented only to assist in understanding and teaching the claimed features. These embodiments are provided as a representative sample of embodiments only, and are not exhaustive and/or exclusive. It is to be understood that advantages, embodiments, examples, functions, features, structures, and/or other aspects described herein are not to be considered limitations on the scope of the invention as defined by the claims or limitations on equivalents to the claims, and that other embodiments may be utilised and modifications may be made without departing from the scope of the claimed invention. Various embodiments of the invention may suitably comprise, consist of, or consist essentially of, appropriate combinations of the disclosed elements, components, features, parts, steps, means, etc., other than those specifically described herein. In addition, this disclosure may include other inventions not presently claimed, but which may be claimed in future.

Further embodiments are defined in the following numbered clauses:
1. Use of a compound of Formula (I), a derivative thereof, or a combination thereof, to increase cognitive performance, alertness and/or energy levels of a human or animal: 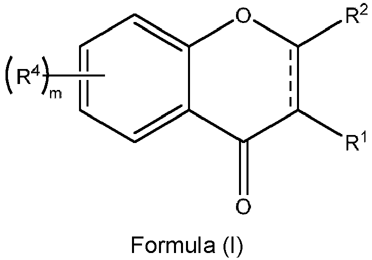 wherein:
   (i) R¹ is H, OH, O-alkyl, O-alkenyl, SH, alkylthio, alkenylthio, NH₂, alkylamino, dialkylamino, alkenylamino, or dialkenylamino; and R² is an optionally substituted phenyl group; or
   (ii) R¹ is an optionally substituted phenyl group; and R² is H, OH, O-alkyl, O-alkenyl, SH, alkylthio, alkenylthio, NH₂, alkylamino, dialkylamino, alkenylamino, or dialkenylamino;

   wherein the optional substituents of the phenyl groups are independently selected from OH, O-alkyl, O-alkenyl, SH, alkylthio, alkenylthio, NH₂, alkylamino, dialkylamino, alkenylamino, and dialkenylamino;
   each R⁴ is independently selected from OH, O-alkyl, O-alkenyl, SH, alkylthio, alkenylthio, NH₂, alkylamino, dialkylamino, alkenylamino, and dialkenylamino; and
   m is an integer from 0 to 4.
2. The use of clause 1, wherein said use further comprises reduced jitteriness in the human or animal.
3. The use of clause 2 wherein the reduced jitteriness in the human or animal is relative to the use of caffeine in said human or animal.
4. The use of any one of clauses 1 to 3, wherein m is 2 or 3.
5. The use of clause 4, wherein m is 2; preferably wherein the R⁴ groups are at the C-5 and C-7 positions of the fused ring.
6. The use of any one of clauses 1 to 5, wherein the phenyl groups are substituted by one or two groups independently selected from OH and O-alkyl; preferably wherein the phenyl groups are substituted by one or two groups independently selected from OH and O-C₁₋₆alkyl.
7. The use of any one of clauses 1 to 6, wherein the phenyl groups are substituted by one group selected from OH and O-alkyl; preferably wherein the phenyl groups are substituted by one group selected from OH and O- C₁₋₆alkyl.
8. The use of any one of clauses 1 to 7, wherein a double bond is present between positions C-2 and C-3 of the fused ring.
9. The use of any one of clauses 1 to 3, wherein the compound of Formula (I) is a compound of Formula (Ia): 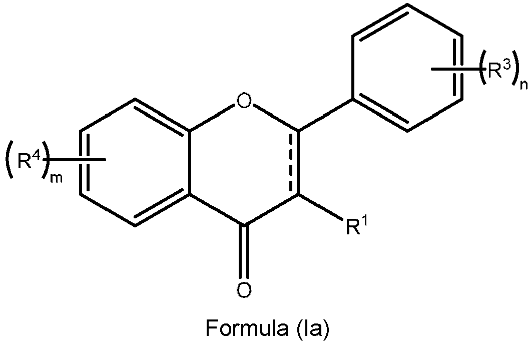 wherein:
   R' is H, OH or O-alkyl, preferably H, OH or O-C₁₋₆alkyl;
   each R³ is independently selected from OH and O-alkyl, preferably OH and O-C₁₋₆alkyl;
   each R⁴ is independently selected from OH and O-alkyl, preferably OH and O-C₁₋₆alkyl;
   m is an integer from 0 to 4; and
   n is an integer from 0 to 5.
10. The use of clause 9, wherein the compound of Formula (Ia) is a compound of Formula (laa): 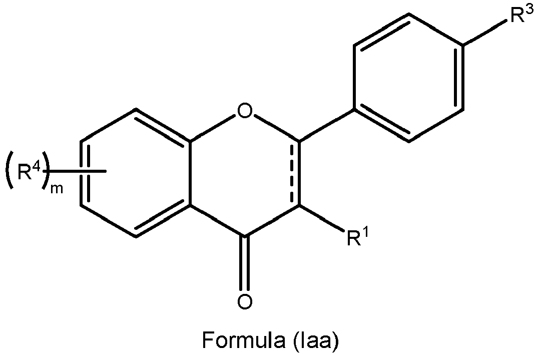
11. The use of clause 9, wherein the compound of Formula (Ia) is a compound of Formula (lab): 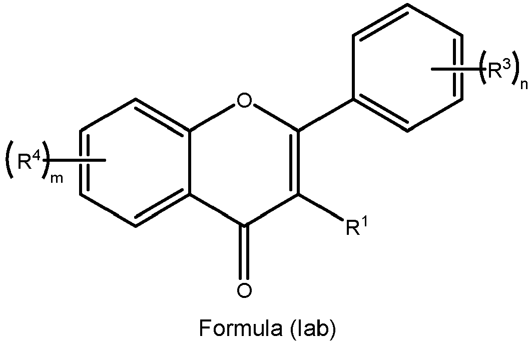
12. The use of clause 9, wherein the compound of Formula (Ia) is a compound of Formula (lac): 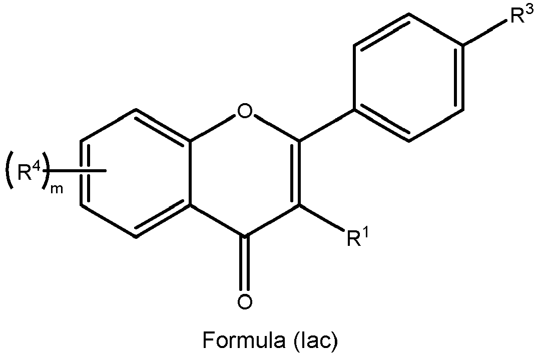
13. The use of clause 9, wherein the compound of Formula (Ia) is a compound of Formula (lad): 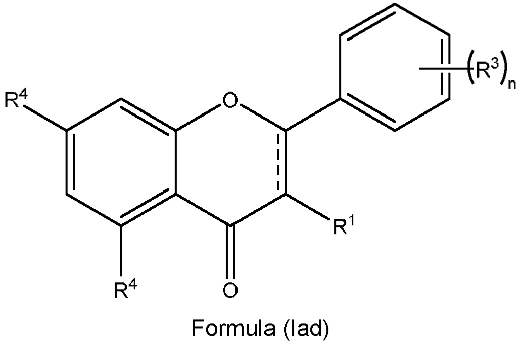
14. The use of clause 9, wherein the compound of Formula (lae) is a compound of Formula (lae): 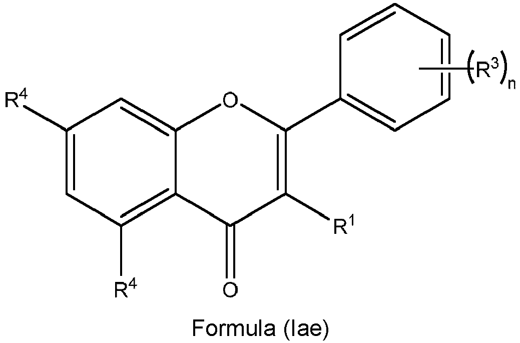
15. The use of clause 9, wherein the compound of Formula (Ia) is a compound of Formula (Iaf): 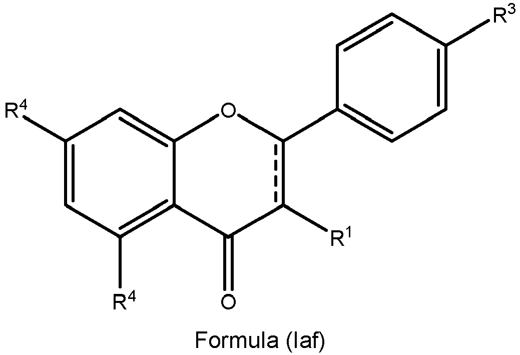
16. The use of clause 9, wherein the compound of Formula (Ia) is a compound of Formula (lag): 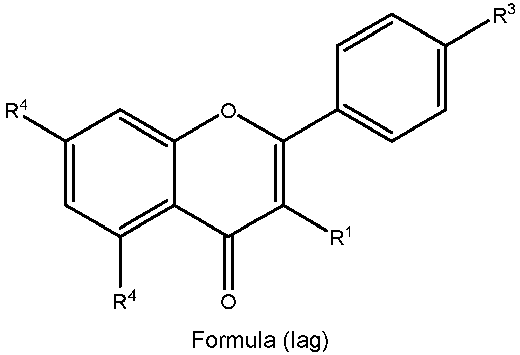
17. The use of clause 9, wherein the compound of Formula (Ia) is a compound of Formula (lah): 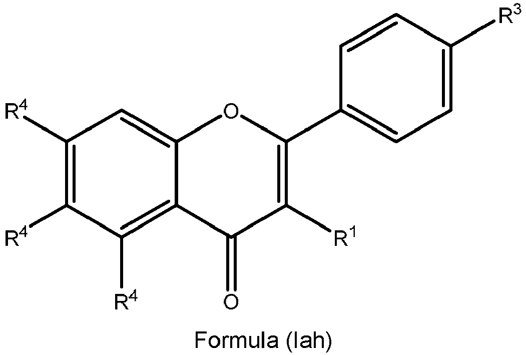
18. The use of any one of clauses 9 to 17, wherein R¹ is H, OH or OMe; preferably wherein R¹ is H or OH.
19. The use of clause 18, wherein R¹ is H.
20. The use of any one of clauses 1 to 3, wherein the compound of Formula (I) is a compound of Formula (Ib): 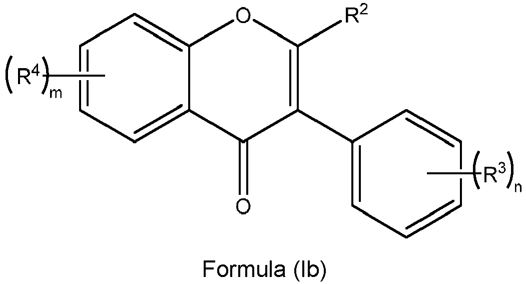 wherein:
   R² is H, OH or O-alkyl, preferably H, OH or O-C₁₋₆alkyl;
   each R³ is independently selected from OH and O-alkyl, preferably OH and O-C₁₋₆alkyl;
   each R⁴ is independently selected from OH and O-alkyl, preferably OH and O-O-C₁₋₆alkyl;
   m is an integer from 0 to 4; and
   n is an integer from 0 to 5.
21. The use of clause 20, wherein the compound of Formula (Ib) is a compound of Formula (Iba): 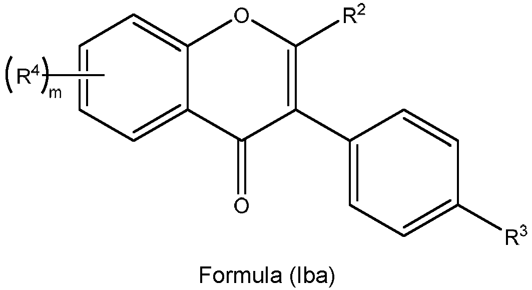
22. The use of clause 20, wherein the compound of Formula (Ib) is a compound of Formula (Ibb): 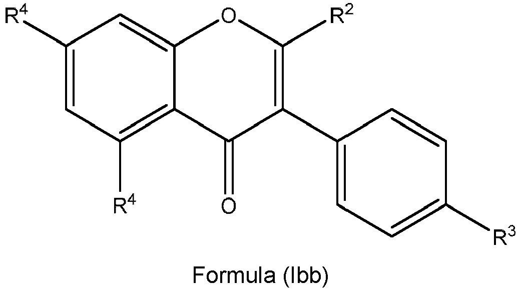
23. The use of any one of clauses 20 to 22, wherein R² is H, OH or OMe; preferably wherein R² is H or OH.
24. The use of clause 23, wherein R² is H.
25. The use of any one of clauses 1 to 24, wherein:
   each R³ is independently H or OH; and/or
   each R⁴ is independently H or OH.
26. The use of any one of clauses 1 to 3, wherein the compound of Formula (I) is selected from flavone, genistein, hispidulin, kaempferol, naringenin, and quercetin.
27. The use of clause 26, wherein the compound of Formula (I) is selected from flavone, genistein, hispidulin, kaempferol, and naringenin.
28. The use of any one of clauses 1 to 27, wherein the compound of Formula (I) is used in combination with one or more B vitamins selected from B1, B2, B6, and B7.
29. The use of any one of clauses 1 to 28, wherein the compound of Formula (I) is further used in combination with one or more compounds selected from taurine, vitamin C, vitamin D, ginseng, zinc or a salt thereof, L-theanine, maca root, beta-alanine, theacrine, coenzyme Q10, theobromine, caffeine, Echinacea, selenium, lemon balm, chamomile, L-tryptophan, piperine or a geometric isomer thereof, saffron, or combinations thereof.
30. An oral product comprising a combination of active ingredients, wherein the combination comprises:
   (a) a compound of Formula (I), a derivative thereof, or a combination thereof: 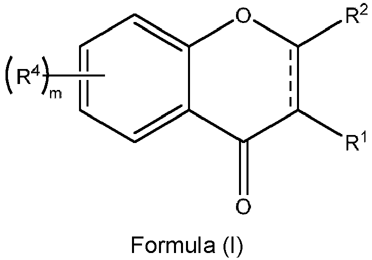 wherein:
      (i) R¹ is H, OH, O-alkyl, O-alkenyl, SH, alkylthio, alkenylthio, NH₂, alkylamino, dialkylamino, alkenylamino, or dialkenylamino; and R² is an optionally substituted phenyl group; or
      (ii) R¹ is an optionally substituted phenyl group; and R² is H, OH, O-alkyl, O-alkenyl, SH, alkylthio, alkenylthio, NH₂, alkylamino, dialkylamino, alkenylamino, or dialkenylamino;

      wherein the optional substituents of the phenyl groups are independently selected from OH, O-alkyl, O-alkenyl, SH, alkylthio, alkenylthio, NH₂, alkylamino, dialkylamino, alkenylamino, and dialkenylamino;
      each R⁴ is independently selected from OH and O-alkyl, O-alkenyl, SH, alkylthio, alkenylthio, NH₂, alkylamino, dialkylamino, alkenylamino, and dialkenylamino; and
      m is an integer from 0 to 4; and
   (b) one or more B vitamins selected from B1, B2, B6 and B7; or a compound selected from maca root, ginseng, coenzyme Q10, caffeine, vitamin C, beta-alanine, theacrine, taurine, zinc or a salt thereof, L-theanine, theobromine Echinacea, selenium, lemon balm, chamomile, L-tryptophan, piperine or a geometric isomer thereof, saffron, and combinations thereof.
31. The oral product of clause 30, wherein the compound of Formula (I), derivative or combination thereof is present in an amount of from about 0.01 % to about 20 % by weight of the oral product.
32. The oral product of clause 30 or clause 31, wherein the oral product does not comprise caffeine.
33. The oral product of any one of clauses 30 to 32, wherein the oral product is a liquid dosage form.
34. The oral product of any one of clauses 30 to 32, wherein the oral product is a solid oral product, preferably a solid oral product in chewable form.
35. The oral product of any one of clauses 30 to 33, wherein the oral product further comprises water in an amount of from about 50% to about 99.9% by weight of the oral product.
36. The oral product of any one of clauses 30 to 35, wherein the compound of Formula (I) is a compound as defined by any one of clauses 4 to 27.

## Claims

1. Use of a compound of Formula (I), a derivative thereof, or a combination thereof, to increase cognitive performance, alertness and/or energy levels of a human or animal: 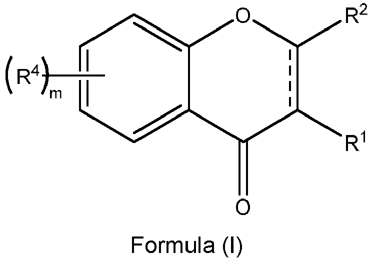 wherein:
(i) R¹ is H, OH, O-alkyl, O-alkenyl, SH, alkylthio, alkenylthio, NH₂, alkylamino, dialkylamino, alkenylamino, or dialkenylamino; and R² is an optionally substituted phenyl group; or
(ii) R¹ is an optionally substituted phenyl group; and R² is H, OH, O-alkyl, O-alkenyl, SH, alkylthio, alkenylthio, NH₂, alkylamino, dialkylamino, alkenylamino, or dialkenylamino;
wherein the optional substituents of the phenyl groups are independently selected from OH, O-alkyl, O-alkenyl, SH, alkylthio, alkenylthio, NH₂, alkylamino, dialkylamino, alkenylamino, and dialkenylamino;
each R⁴ is independently selected from OH, O-alkyl, O-alkenyl, SH, alkylthio, alkenylthio, NH₂, alkylamino, dialkylamino, alkenylamino, and dialkenylamino; and m is an integer from 0 to 4.

2. The use of claim 1, wherein said use further comprises reduced jitteriness in the human or animal, optionally wherein the reduced jitteriness in the human or animal is relative to the use of caffeine in said human or animal.

3. The use of claim 1 or claim 2, wherein m is 2 or 3.

4. The use of claim 3, wherein m is 2; preferably wherein the R⁴ groups are at the C-5 and C-7 positions of the fused ring.

5. The use of any one of claims 1 to 4, wherein the phenyl groups are substituted by one or two groups independently selected from OH and O-alkyl; and/or
wherein a double bond is present between positions C-2 and C-3 of the fused ring.

6. The use of any one of claims 1 to 3, wherein the compound of Formula (I) is a compound of Formula (Ia): 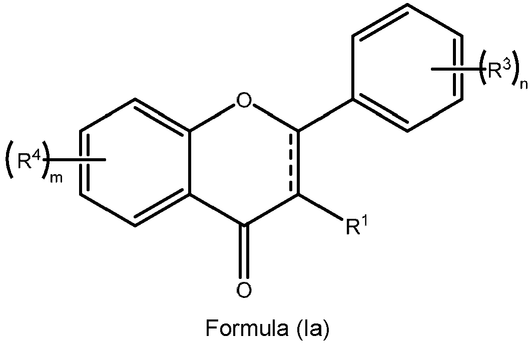 wherein:
R' is H, OH or O-alkyl; preferably H, OH, or OMe; more preferably H or OH;
each R³ is independently selected from OH and O-alkyl;
each R⁴ is independently selected from OH and O-alkyl;
m is an integer from 0 to 4; and
n is an integer from 0 to 5.

7. The use of any one of claims 1 to 3, wherein the compound of Formula (I) is a compound of Formula (Ib): 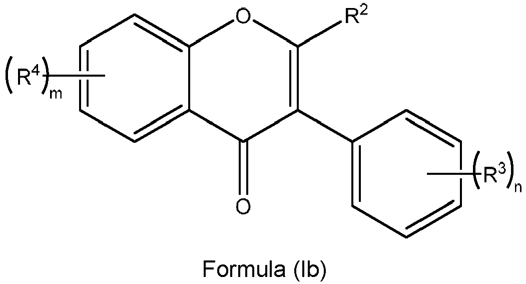 wherein:
R² is H, OH or O-alkyl; preferably H, OH or OMe; more preferably H or OH;
each R³ is independently selected from OH and O-alkyl;
each R⁴ is independently selected from OH and O-alkyl;
m is an integer from 0 to 4; and
n is an integer from 0 to 5.

8. The use of any one of claims 1 to 7, wherein:
each R³ is independently H or OH; and/or
each R⁴ is independently H or OH.

9. The use of any one of claims 1 to 3, wherein the compound of Formula (I) or derivative thereof is selected from flavone, genistein, hispidulin, kaempferol, naringenin, and quercetin; optionally wherein the compound of Formula (I) is selected from flavone, genistein, hispidulin, kaempferol, and naringenin.

10. The use of any one of claims 1 to 9, wherein the compound of Formula (I) is used in combination with one or more B vitamins selected from B1, B2, B6, and B7;
optionally wherein the compound of Formula (I) is further used in combination with one or more compounds selected from taurine, vitamin C, vitamin D, ginseng, zinc or
a salt thereof, L-theanine, maca root, beta-alanine, theacrine, coenzyme Q10, theobromine, caffeine, Echinacea, selenium, lemon balm, chamomile, L-tryptophan, piperine or a geometric isomer thereof, saffron, or combinations thereof.

11. An oral product comprising a combination of active ingredients, wherein the combination comprises:
(a) a compound of Formula (I), a derivative thereof, or a combination thereof: 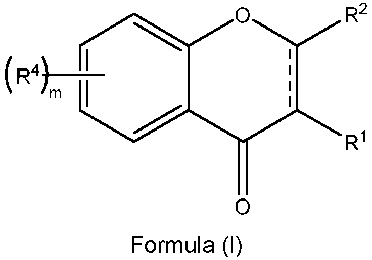 wherein:
(i) R¹ is H, OH, O-alkyl, O-alkenyl, SH, alkylthio, alkenylthio, NH₂, alkylamino, dialkylamino, alkenylamino, or dialkenylamino; and R² is an optionally substituted phenyl group; or
(ii) R¹ is an optionally substituted phenyl group; and R² is H, OH, O-alkyl, O-alkenyl, SH, alkylthio, alkenylthio, NH₂, alkylamino, dialkylamino, alkenylamino, or dialkenylamino;
wherein the optional substituents of the phenyl groups are independently selected from OH, O-alkyl, O-alkenyl, SH, alkylthio, alkenylthio, NH₂, alkylamino, dialkylamino, alkenylamino, and dialkenylamino;
each R⁴ is independently selected from OH, O-alkyl, O-alkenyl, SH, alkylthio, alkenylthio, NH₂, alkylamino, dialkylamino, alkenylamino, and dialkenylamino; and
m is an integer from 0 to 4; and
(b) one or more B vitamins selected from B1, B2, B6 and B7; or a compound selected from maca root, ginseng, coenzyme Q10, caffeine, vitamin C, beta-alanine, theacrine, taurine, zinc or a salt thereof, L-theanine, theobromine, Echinacea, selenium, lemon balm, chamomile, L-tryptophan, piperine or a geometric isomer thereof, saffron, and combinations thereof.

12. The oral product of claim 11, wherein the compound of Formula (I), derivative or combination thereof is present in an amount of from about 0.01 % to about 20 % by weight of the oral product.

13. The oral product of claim 11 or claim 12, wherein the oral product does not comprise caffeine.

14. The oral product of any one of claims 11 to 13, wherein the oral product is a liquid dosage form or a solid oral product.

15. The oral product of any one of claims 11 to 14, wherein the compound of Formula (I) is a compound as defined by any one of claims 3 to 9.
